(19) Europäisches Patentamt
European Patent Office
Office européen des brevets

(11) EP 1 560 809 B1

(12) **EUROPÄISCHE PATENTSCHRIFT**

(45) Veröffentlichungstag und Bekanntmachung des Hinweises auf die Patenterteilung:
**22.08.2007 Patentblatt 2007/34**

(21) Anmeldenummer: **03779844.4**

(22) Anmeldetag: **05.11.2003**

(51) Int Cl.:
***C07C 275/26*** *(2006.01)* ***C07D 209/14*** *(2006.01)*
***C07D 209/18*** *(2006.01)* ***C07C 335/14*** *(2006.01)*
***A61K 31/165*** *(2006.01)* ***A61K 31/17*** *(2006.01)*
***A61K 31/405*** *(2006.01)* ***A61P 25/04*** *(2006.01)*

(86) Internationale Anmeldenummer:
**PCT/EP2003/012304**

(87) Internationale Veröffentlichungsnummer:
**WO 2004/043909 (27.05.2004 Gazette 2004/22)**

(54) **CYCLOHEXYL-HARNSTOFF-DERIVATE**

CYCLOHEXYLUREA DERIVATIVES

DERIVES DE CYCLOHEXYLUREE

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **11.11.2002 DE 10252650**

(43) Veröffentlichungstag der Anmeldung:
**10.08.2005 Patentblatt 2005/32**

(73) Patentinhaber: **Grünenthal GmbH**
**52078 Aachen (DE)**

(72) Erfinder:
- **HINZE, Claudia**
  **52066 Aachen (DE)**
- **SCHICK, Hans**
  **13086 Berlin (DE)**

(56) Entgegenhaltungen:
**EP-A- 0 147 085** **WO-A-02/089783**
**WO-A-02/090317** **WO-A-02/090330**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Cyclohexyl-Harnstoff-Derivate, Verfahren zu deren Herstellung, Arzneimittel enthaltend diese Verbindungen und die Verwendung von Cyclohexyl-Harnstoff-Derivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den $\mu$, $\kappa$ und $\delta$ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535).

Das Nociceptin-Peptid zeigt nach intracerebroventricularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf $\mu$-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Haminkontinenz). Weiter wird die Verwendung von Agonisten und Antagonisten als Anorektika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen gerade im Bereich der Schmerztherapie, aber auch bei anderen der genannten Indikationen, Opioidrezeptoren wie der $\mu$-Rezeptor und andere Subtypen eine große Rolle. Entsprechend ist es günstig, wenn die Verbindung auch Wirkung an diesen Opioidrezeptoren zeigen.

WO 02/090317 offenbart Cyclohexan-1,4-diaminderivate, wobei in der Gruppierung $NR^4R^5$ nur der Rest $R^4$ H bedeuten kann, $R^5$ aber nicht für $(CH_2)_nC(O)(CHR)_mNHR$ stehen kann.

WO 02/090330 offenbart mit einem Pyridylrest substituierte Cyclohexan-1,4-diaminderivate, bei denen ebenfalls in der Gruppierung $NR^4R^5$ nur der Rest $R^4$ H bedeuten kann, $R^5$ aber nicht für $(CH_2)_nC(O)(CHR)_mNHR$ stehen kann.

WO 02/089783 offenbart die Verwendung von Cyclohexan-1,4-diaminderivaten, wobei in der Gruppierung $NR^4R^5$ nur der Rest R H bedeuten kann, $R^5$ aber nicht für $(CH_2)_nC(O)(CHR)_mNHR$ stehen kann.

EP-A-0 147 085 offenbart Cyclohexan-1,2-Derivate, die an den $\mu$- und $\kappa$-Opioid-Rezeptor binden. Diese Verbindungen sind in 4-Position unsubstituiert.

Aufgabe der vorliegenden Erfindung war es, Arzneimittel zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Gegenstand der Erfindung sind daher Cyclohexyl-Harnstoff-Derivate der allgemeinen Formel I,

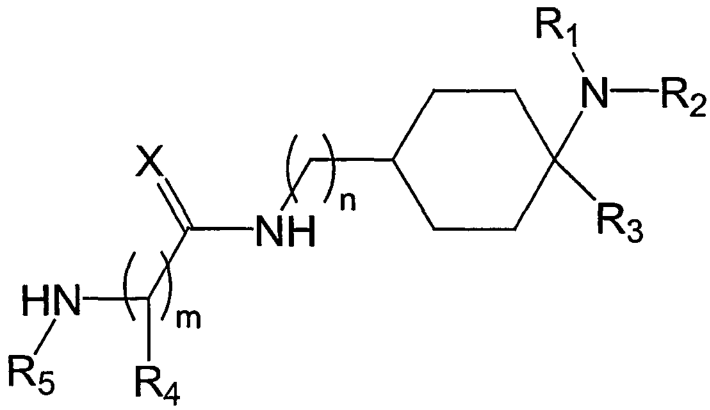

, worin
n=0-3,
m=0-2,
X = O oder S (bei m = 0),
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert; oder $-(CH_2)_o-W-(CH_2)_p-H$
mit W = O, $NR_7$ oder S, und o = 0-3 und p = 0-4 und
mit $R_7$ ausgewählt aus H, $C_{1-8}$-Alkyl, gesättigt oder ungesättigt, verzweigt oder unverzweigt, einfach oder mehrfach substituiert oder unsubstituiert;
$R^5$ , wenn $m \neq 0$, ausgewählt ist aus:
$-(CH_2)_qR^{12}$,
$-C(Y)-Z-R^{12}$ oder
$-C(Y)-O-Z-R^{12}$,
mit Y = O, $CH_2$ oder S,
mit Z = $C_{1-8}$-Alkyl, gesättigt oder ungesättigt, verzweigt oder unverzweigt, einfach oder mehrfach substituiert oder unsubstituiert;
oder $(CH_2)_q$
mit q = 0-8
mit $R^{12}$ ausgewählt aus
H; $C_{3-8}$-Cycloalkyl, Aryl oder Heteroaryl, jeweils unsubstituiert oder einfach oder mehrfach substituiert,
oder
$R^5$ , wenn m = 0, ausgewählt ist aus $C_{3-8}$-Cycloalkyl, Aryl oder Heteroaryl, jeweils unsubstituiert oder einfach oder mehrfach substituiert; $-(CH2)_qR^{12}$,
mit q = 0-8
mit $R^{12}$ ausgewählt aus
H; $C_{3-8}$-Cycloalkyl, Aryl oder Heteroaryl, jeweils unsubstituiert oder einfach oder mehrfach substituiert;
gegebenenfalls in Form ihrer Racemate, ihrer reinen Stereoisomeren, insbesondere Enantiomeren oder Diastereomeren,

oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Alle diese erfindungsgemäßen Verbindungen zeigen gute Bindung an den ORL1-Rezeptor, aber auch an andere Opiatrezeptoren.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4-oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3-oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, $-CH_2-CH_2-$, $-CH_2-CH_2-CH_2-$ und $-CH_2-CH_2-CH_2-CH_2-$ zu verstehen, unter $(CH_2)_{4-5}$ ist $-CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,

die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl-oder Heteroaryl-Rest bedeuten, wobei diese Aryl und Heteroarylreste nicht selbst mit Aryl- oder Heteroaryl-Resten substituiert sein dürfen,
oder die Reste $R^{23}$ und $R^{24}$ bedeuten zusammen $CH_2CH_2OCH_2CH_2$,
$CH_2CH_2NR^{25}CH_2CH_2$ oder $(CH_2)_{3-6}$, und
der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniertals Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-,3-oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz und das Citrat-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid oder Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro $1\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - verträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen Cyclohexyl-Harnstoff-Derivate gilt, dass
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert;
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten, mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert,
oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus H, Methyl oder Ethyl bedeuten.

Für eine bevorzugte Ausführungsform der erfindungsgemäßen Cyclohexyl-Harnstoff-Derivate gilt, dass
$R^3$ ausgewählt ist aus $C_{3-8}$-Cycloalkyl, Aryl oder Heteroaryl, jeweils unsubstituiert oder einfach oder mehrfach substituiert;

über eine gesättigte oder ungesättigte, unverzweigte, substituierte oder unsubstituierte $C_{1-2}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenem Phenyl, Furyl oder Thiophenyl, jeweils unsubstituiert oder einfach oder mehrfach substituiert.

Für eine bevorzugte Ausführungsform der erfindungsgemäßen Cyclohexyl-Harnstoff Derivate gilt, dass
$R^4$ ausgewählt ist aus H oder $C_{1-8}$-Alkyl, gesättigt oder ungesättigt, verzweigt oder unverzweigt, einfach oder mehrfach substituiert oder unsubstituiert, vorzugsweise H, $CH_3$ oder $C_2H_5$.

Für eine bevorzugte Ausführungsform der erfindungsgemäßen Cyclohexyl-Harnstoff-Derivate gilt, dass vorzugsweise
$R^{12}$ ausgewählt ist aus H; Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus H; Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Ein besonders bevorzugter Gegenstand dieser Erfindung sind Cyclohexyl-Harnstoff-Derivate der allgemeinen Formel I,

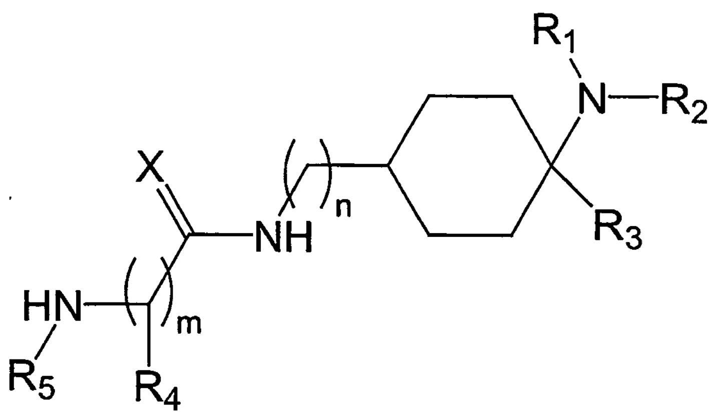

, worin

- $R_1$ und $R_2$ unabhängig voneinander ausgewählt sind aus H, $CH_3$ $C_2H_5$ oder CHO,

insbesondere

- $R_1$ und $R_2$ unabhängig voneinander ausgewählt sind aus H oder $CH_3$.

- $R_3$ ausgewählt ist aus Indolyl, Pyridyl, Thienyl, Pyrrolyl, Phenyl, Benzyl oder Phenethyl, jeweils unsubstituiert oder am Ring ein- oder mehrfach substituiert,

vorzugsweise

■ $R_3$ ausgewählt ist aus Phenyl unsubstituiert oder am Ring einfach substituiert; Benzyl oder Phenethyl, Indolyl, Pyridyl, Thienyl, Pyrrolyl, insbesondere

■ $R_3$ ausgewählt ist aus Phenyl, Benzyl, Phenethyl, o-Fluor-Phenyl, m-Fluor-Phenyl, p-Fluor-Phenyl, o-Chlor-Phenyl, p-Chlorphenyl, m-Chlorphenyl, o-Brom-Phenyl, m-Brom-Phenyl, p-Brom-Phenyl, o-Iod-Phenyl, m-Iod-Phenyl, p-Iod-Phenyl, o-Amino-Phenyl, m-Amino-Phenyl, p-Amino-Phenyl,o-Methyl-Phenyl, m-Methyl-Phenyl, p-Methyl-Phenyl, o-Methoxy-Phenyl, m-Methoxy-Phenyl, p- Methoxyphenyl, o-Ethyl-Phenyl, m-Ethyl-Phenyl, p-Ethyl-Phenyl, o-Ethoxy-Phenyl, m-Ethoxy-Phenyl, p-Ethoxy-Phenyl, o-Hydroxy-Phenyl, m-Hydroxy-Phenyl oder p-Hydroxy-Phenyl; Indolyl, Pyridyl, Thienyl, Pyrrolyl.

■ $R_4$ ausgewählt ist aus H, $CH_3$, $C_2H_5$, n-Propyl, i-Propyl, n-Butyl, i-Butyl oder t-Butyl.

Bezüglich dieses besonders bevorzugten Gegenstandes ist es besonderes bevorzugt wenn für die bevorzugten erfindungsgemässen 4-Aminomethyl-1-Aryl-Cyclohexylamin-Derivaten gilt, dass

■ $R_5$, wenn $m \neq 0$, ausgewählt ist aus:

$-(CH_2)_qR^{12}$,
$-C(Y)-Z-R^{12}$ oder
$-C(Y)-O-Z-R^{12}$,
mit Y = O
mit Z = $C_{1-6}$-Alkyl, gesättigt oder ungesättigt, verzweigt oder unverzweigt, einfach oder mehrfach substituiert oder unsubstituiert;
oder $(CH_2)q$
mit q = 0-6.

Bezüglich dieses besonders bevorzugten Gegenstandes ist es besonderes bevorzugt wenn für die bevorzugten erfindungsgemässen 4-Aminomethyl-1-Aryl-Cyclohexylamin-Derivaten gilt, dass

■ $R_5$, wenn m = 0, ausgewählt ist aus $-(CH_2)_qR_{12}$,
mit q = 0-6.

Bezüglich dieses besonders bevorzugten Gegenstandes ist es besonderes bevorzugt wenn für die bevorzugten erfindungsgemässen 4-Aminomethyl-1-Aryl-Cyclohexylamin-Derivaten gilt, dass vorzugsweise
$R^{12}$ ausgewählt ist aus H; Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus H; Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert. ganz besonders bevorzugt
$R^{12}$ ausgewählt ist aus Phenyl oder Indolyl, jeweils unsubstituiert, ein-oder mehrfach substituiert, wobei unsubstiuiertes Phenyl oder Indolyl oder einfach mit Methyl, Methoxy, Chlor, Fluor oder $CF_3$ in para-Stellung substituiertes Phenyl oder mit mit Methyl, Methoxy, Chlor, Fluor oder $CF_3$ in 5- Position substiuiertes Indolyl erfindungsgemäß insbesondere bevorzugt sind.

Darüber hinaus sind ganz besonders bevorzugt 4-substituierte Cyclohexyl-Harnstoff-Derivate aus der Gruppe

1-(4-Dimethylamino-4-phenylcyclohexyl)-3-(3-phenylpropyl)harnstoff Hydrochlorid (unpolareres und polareres Diastereoisomer)

1-(4-Dimethylamino-4-phenylcyclohexyl)3-[2-(1 H-indol-3-yl)ethyl]-harnstoff-hydrochlorid (unpolareres und polareres Diastereoisomer)

N-[(4-Dimethylamino-4-phenylcyclohexylcarbamoyl)methyl]-3-(1 H-indol-3-yl)butyramid-hydrochlorid (unpolareres

und polareres Diastereoisomer)

5-(1 H-Indol-3-yl)-pentansäure[(4-dimethylamino-4-phenylcyclohexylcarbamoyl)methyl]amid-hydrochlorid (unpolareres und polareres Diastereoisomer)

6-(1 H-Indol-3-yl)hexansäure[(4-dimethylamino-4-phenylcyclohexylcarbamoyl)-methyl]-amid-hydrochlorid (unpolareres und polareres Diastereoisomer)

N-[(4-Dimethylamino-4-phenylcyclohexylcarbamoyl)methyl]-3-(1 H-indol-3-yl)propionamid-hydrochlorid (unpolareres und polareres Diastereoisomer)

N-(4-Dimethylamino-4-phenylcyclohexyl)-2-(2-1 H-indol-3-ylacetylamino)-propionamid-hydrochlorid (unpolareres und polareres Diastereoisomer)

2-(2-1 H-Indol-3-ylacetylamino)-4-methylpentansäure-(4-dimethylamino-4-phenylcyclohexyl)amid-hydrochlorid (unpolareres und polareres Diastereoisomer)

1-(4-Dimethylamino-4-phenylcyclohexyl)-3-[2-(1 H-indol-3-yl)-1-methylethyl]-harnstoff-hydrochlorid (unpolareres und polareres Diastereoisomer)

1-(4-Dimethylamino-4-phenylcyclohexyl)-3-[2-(5-Fluor-1 H-indol-3-yl)ethyl]-harnstoff-hydrochlorid (unpolareres und polareres Diastereoisomer)

1-(4-Dimethylamino-4-phenylcyclohexylmethyl)-3-[2-(1 H-indol-3-yl)ethyl]-harnstoff-hydrochlorid (unpolareres und polareres Diastereoisomer)

1-(4-Dimethylamino-4-phenylcyclohexylmethyl)-3-(3-phenylpropyl)harnstoff-hydrochlorid (unpolareres und polareres Diastereoisomer)

1-(4-Dimethylamino-4-phenylcyclohexylmethyl)-3-[2-(1 H-indol-3-yl)-1-methylethyl]-harnstoff-hydrochlorid (unpolareres und polareres Diastereoisomer)

1-(4-Dimethylamino-4-phenylcyclohexylmethyl)-3-[2-(5-fluor-1 H-indol-3-yl)ethyl]harnstoff-citrat (unpolareres und polareres Diastereoisomer)

1-[3-(4-Dimethylamino-4-phenyl-cyclohexyl)-propyl]-3-[2-(1 H-indol-3-yl)-ethyl]-harnstoff (unpolareres und polareres Diastereiosomer)

1-(4-Dimethylamino-4-phenylcyclohexyl)-3-(4-phenyl-propyl)thioharnstoff-citrat (unpolareres und polareres Diastereiosomer)

1-(4-Dimethylamino-4-phenylcyclohexyl)-3-[2-(1H-indol-3-yl)methylethyl]thioharnstoff-citrat (unpolareres und polareres Diastereiosomer)

1-[3-(4-Dimethylamino-4-phenyl-cyclohexyl)-propyl]-3-[2-(1 H-indol-3-yl)-ethyl]-harnstoff (unpolareres und polareres Diastereiosomer)

1-(4-Dimethylamino-4-phenylcyclohexylmethyl)-3-[2-(1H-indol-3-yl)ethyl]-thioharnstoff-citrat (unpolareres und polareres Diastereoisomer)

1-(4-Dimethylamino-4-phenylcyclohexylmethyl)-3-[2-(1 H-indol-3-yl)-1-methylethyl]thioharnstoff-citrat (unpolareres und polareres Diastereoisomer)

2-[3-(4-Dimethylamino-4-phenylcyclohexylmethyl)thioureido]-3-(1H-indol-3-yl)propionsäure-methylester-citrat (unpolareres und polareres Diastereoisomer)

1-[2-(4-Dimethylamino-4-phenylcyclohexyl)ethyl]-3-[2-(1H-indol-3-yl)-ethyl]thioharnstoff-hydrochlorid (unpolareres Diastereoisomer)

1-[2-(4-Dimethylamino-4-phenylcyclohexyl)ethyl]-3-[2-(1H-indol-3-yl)-ethyl]thioharnstoff-citrat (polareres Diastereoisomer)

Die erfindungsgemäßen Substanzen sind toxikologisch unbedenklich, so daß sie sich als pharmazeutischer Wirkstoff in Arzneimittel eignen.

Ein weiterer Gegenstand der Erfindung sind daher Arzneimittel enthaltend wenigstens ein Cyclohexyl-Harnstoff-Derivat.

Die erfindungsgemäßen Arzneimittel enthalten neben mindestens einem erfindungsgemäßen Cyclohexyl-Harnstoff-Derivat gegebenenfalls geeignete Zusatz-und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Cyclohexyl-Harnstoff-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Cyclohexyl-Harnstoff-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen Cyclohexyl-Harnstoff-Derivats appliziert.

Für alle vorstehenden Formen der erfindungsgemäßen Arzneimittel ist es besonders bevorzugt, wenn das Arzneimittel neben wenigstens einem Cyclohexyl-Harnstoff-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes Cyclohexyl-Harnstoff-Derivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße Cyclohexyl-Harnstoff-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen Cyclohexyl- Harnstoff-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist daher die Verwendung eines erfindungsgemäßen Cyclohexyl-Harnstoff-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes Cyclohexyl-Harnstoff-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen Cyclohexyl-Harnstoff Derivats, oder eines erfindungsgemäßen Arzneimittels.

Ein weiterer Gegenstand der Erfindung sind Verfahren zur Herstellung der erfindungsgemäßen Cyclohexyl-Harnstoff-Derivate.

Ein erstes Verfahren zur Herstellung von Cyclohexyl-Harnstoff-Derivaten der Formel I umfasst die Verfahrensschritte

a1) für Verbindungen, in denen n= 0 ist, Überführung eines 4-Aminocyclohexanons durch Oximbildung und anschließende Reduktion oder durch reduktive Aminierung in das entsprechende 4-Aminocylohexylamin (siehe auch nachfolgendes allgemeines Syntheseschema (I)),

a2) für Verbindungen, in denen n = 1 ist, Umsetzung eines 4-Aminocyclohexanons mit dem Umsetzungsprodukt aus Alkoxymethyl-triphenylphosphoniumhalogenid und einer starken Base zum 4-Aminocyclohexancarbaldehyd, und anschließende reduktive Aminierung bzw. Oximbildung mit nachfolgender Reduktion zum 4-Aminocyclohexylmethylamin (siehe auch nachfolgendes allgemeines Syntheseschema (III));

a3) für Verbindungen, in denen n = 2 ist Umsetzung eines 4-Aminocyclohexanons mit dem Umsetzungsprodukt aus Cyanomethylphosphonsäure-dialkylester und einer starken Base zum $\alpha,\beta$-ungesättigten Nitril, der anschließend zum 4-Aminocyclohexylethylamin reduziert wird (siehe auch nachfolgendes allgemeines Syntheseschema (VII));

a4) für Verbindungen, in denen n = 3 ist, Umsetzung eines wie unter a2) beschrieben erhaltenen 4-Aminocyclohexancarbaldehyds zur weiteren Kettenverlängerung mit dem Umsetzungsprodukt aus Cyanmethanphosphonsäuredialkylester und einer starken Base zum $\alpha$, $\beta$-ungesättigten Nitril, und anschließende Reduktion des entstandenen $\alpha,\beta$-ungesättigten Nitrils zum Amin (siehe auch nachfolgendes allgemeines Syntheseschema (IX));

b) Umsetzung des in Schritt a1), a2) a3) oder a4) gebildeten Amins mit einem aktivierten Kohlesäurenderivat in Gegenwart einer Base zum Carbamidsäureester;

c) Umsetzung des in Schritt b) gebildeten Carbamidsäureesters mit Aminen der Formel $R_5$-$NH_2$ zu Cyclohexyl-Harnstoff-Derivaten der Formel I mit m=0.

Ein Verfahren zur Herstellung von Cyclohexyl-Harnstoff-Derivaten der Formel I, in denen $m \neq 0$ ist und $R^5$ ausgewählt ist aus -C(Y)-Z-$R^{12}$ oder -C(Y)-O-Z-$R^{12}$ mit Y=O oder S ist dadurch gekennzeichnet, dass ein gemäß dem Verfahren in Anspruch 15, Schritt a1, a2), a3) oder a4) hergestelltes Amin mit einem am Carbonylterminus aktivierten Boc-Aminosäure-Derivat acyliert wird, anschließend die Boc-Schutzgruppe sauer abgespalten wird und nachfolgend die entschützte Aminogruppe mit einem aktivierten Carbonsäurederivat acyliert wird (siehe auch nachfolgendes allgemeines Syntheseschema (V)).

Ein weiteres Verfahren zur Herstellung von Cyclohexyl-Harnstoff-Derivaten der Formel I ist dadurch gekennzeichnet, dass ein Amin der Formel $R_5$-$NH_2$ mit einem aktivierten Kohlensäurederivat zu einem Carbamidsäureester umgesetzt wird und anschließend die Umsetzung mit einem gemäß Schritt a1, a2), a3) oder a4) von Anspruch 15 erhaltenen Amin zu einem Cyclohexyl-Harnstoff-Derivat der Formel I mit m=0 umgesetzt wird (siehe auch nachfolgende allgemeine Syntheseschemata (II), (IV) und (VIII)).

Ein Verfahren zur Herstellung von Cyclohexyl-Harnstoff-Derivaten der Formel I mit $m\neq0$ ist dadurch gekennzeichnet, dass gemäß Schritt a1, a2), a3) oder a4) von Anspruch 15 hergestellte 4-Aminocyclohexylamine gemäß dem nachfolgenden Syntheseschema (VI) mit am Carboxyl-Terminus aktivierten Aminosäure-Derivaten acyliert werden.

Ein alternatives Verfahren zur Herstellung von Cyclohexyl-Hamstoff-Derivaten der Formel 1, in denen X=O oder S ist und m=0 ist, ist dadurch gekennzeichnet, dass ein gemäß Schritt a1), a2), a3) oder a4) von Anspruch 15 hergestelltes Amin mit einem Isocyanat der Formel OCN-$R_5$ oder mit einem Isothiocyanat der Formel SCN-$R_5$ zum Cyclohexyl-Harnstoff-Derivat der Formel I umgesetzt wird.

Weitere Einzelheiten werden in der folgenden Beschreibung und den Beispielen ausgeführt, wobei $R_1$, $R_2$, $R_3$, $R_4$, $R_5$ die für erfindungsgemäße Verbindungen gemäß Formel angegebene Bedeutung haben, und $R_{01}$ und $R_{02}$ unabhängig voneinander ausgewählt sind aus einer Schutzgruppe oder den für erfindungsgemäße Verbindungen gemäß Formel I für $R_1$ und $R_2$ angegebenen Gruppen:

Allgemeines Syntheseschema Cyclohexyl-Harnstoffe (1):

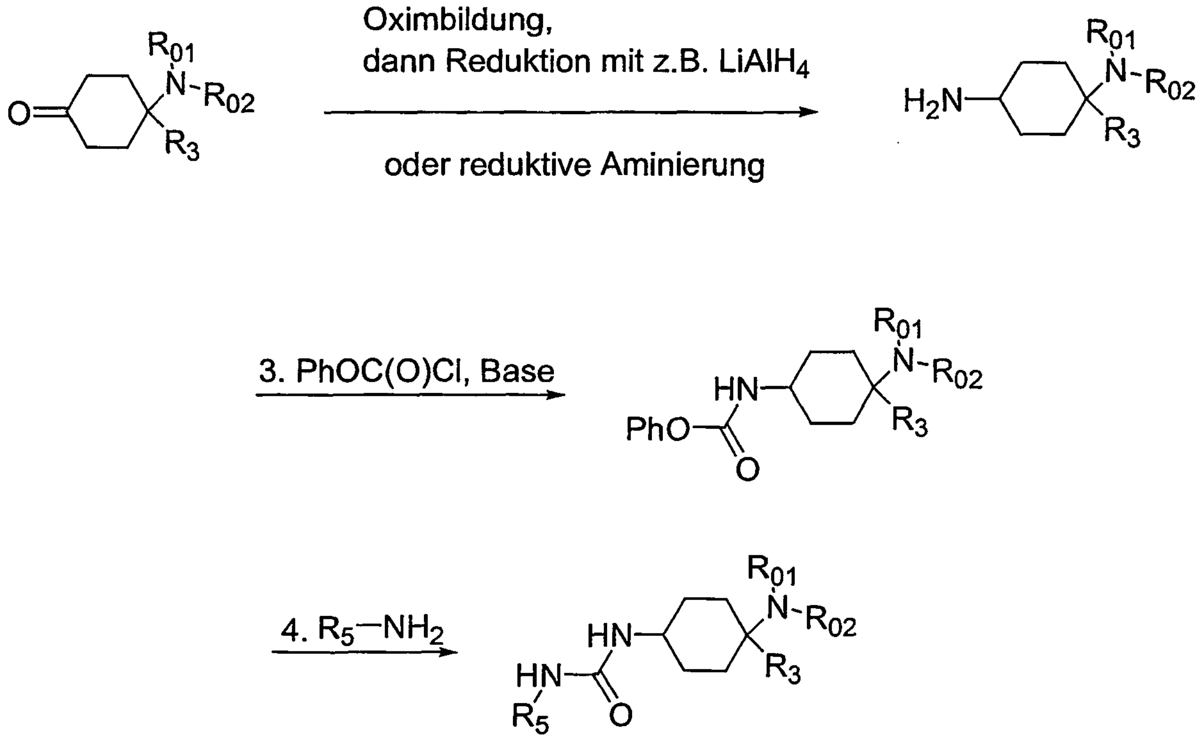


Die Herstellung geeigneter 4-Aminocyclohexanone ist aus der Literatur bekannt (Lednicer et al., J. Med. Chem., 23, 1980, 424-430; WO 0290317).

Ein 4-Aminocyclohexanon kann nach dem Fachmann bekannten Bedingungen, z.B. mit Hydroxylamin-Hydrochlorid in trockenem Pyridin und abs. Ethanol ins Oxim überführt werden. Dieses kann nach gängigen Reduktionsmethoden, z.B. durch Reduktion mit Devarda-Legierung (Cu, Al, Zn); bevorzugt mit Nickel(II)halogenid und Natriumboranat; oder besonders bevorzugt mit komplexen Hydriden (z.B. Lithiumalanat) in das Amin umgewandelt werden.
Alternativ kann das Keton nach dem Fachmann bekannten Methoden reduktiv aminiert werden, z.B. mit Ammoniumacetat und Natriumcyanoborhydrid.
Die gebildeten 4-Amino-cyclohexylamin-Intermediate werden mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit primären Aminen zu den korrespondierenden Harnstoffen umgesetzt.

Alternatives Syntheseschema Cyclohexyl-Harnstoffe (II):

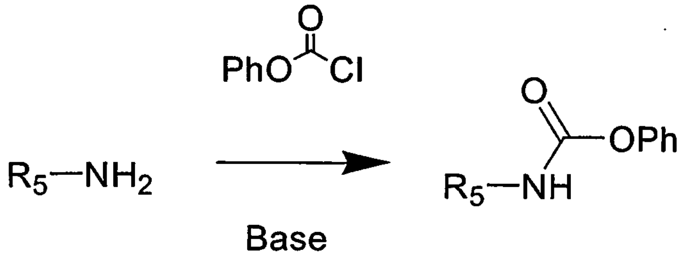


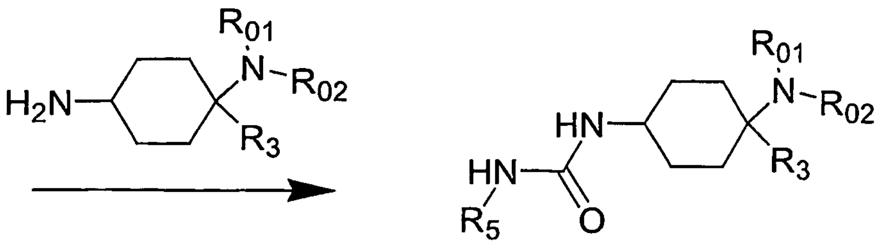

Primäre Amine werden mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit den aus Verfahren I bekannten 4-Amino-cyclohexylamin-Intermediaten zu den korrespondierenden Harnstoffen umgesetzt.

Allgemeines Syntheseschema Cyclohexymethyl-Harnstoffe (III):

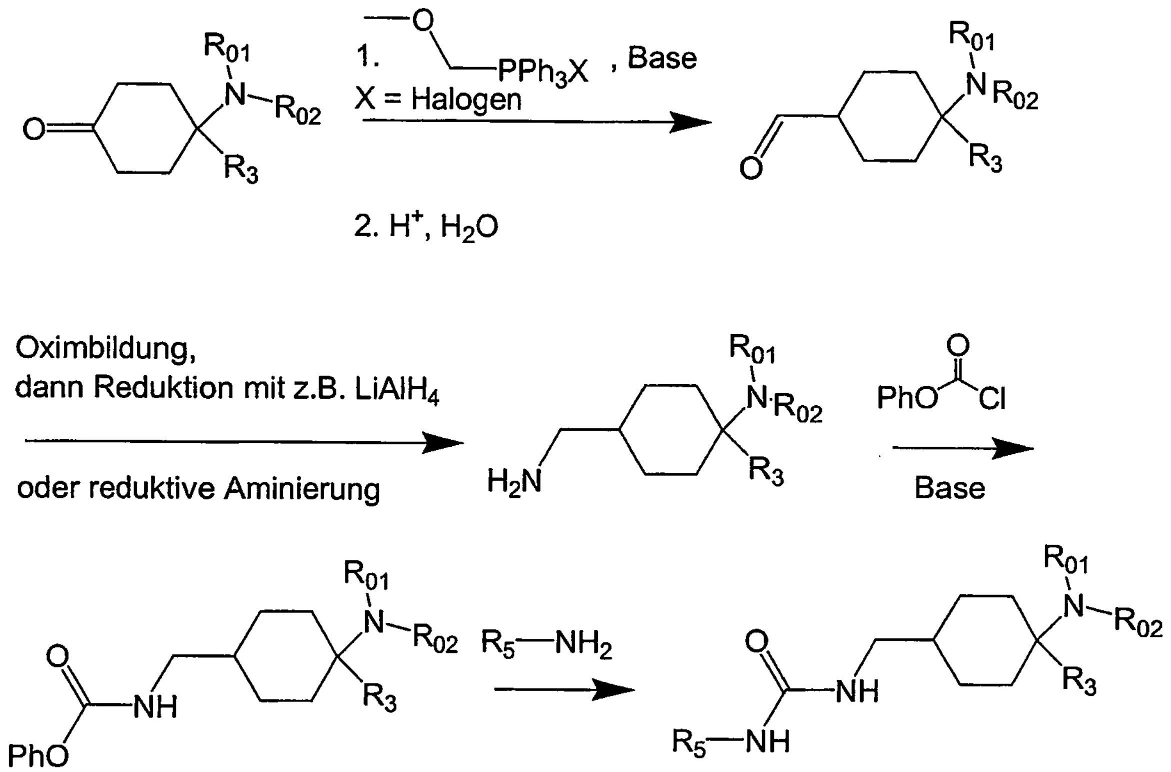


Methoxymethyltriphenylphosphoniumhalogenid wird zunächst mit einer starken Base, beispielsweise Natriumhydrid oder Butyllithium , dann mit einem 4-Aminocyclohexanon umgesetzt und der intermediär gebildete Methylvinylether unter sauren wässrigen Bedingungen, beispielsweise mit Salzsäure oder Schwefelsäure, in den korrespondierenden 4-Aminocyclohexancarbaldehyd überführt. Dieser wird unter dem Fachmann bekannten Bedingungen ins Oxim überführt. Dieses kann nach gängigen Reduktionsmethoden, z.B. durch Reduktion mit Devarda-Legierung (Cu, Al, Zn); oder bevorzugt mit Nickel(II)halogenid und Natriumboranat; oder besonders bevorzugt mit komplexen Hydriden (z.B. Lithiumalanat) in das korrespondierende 4-Aminomethyl-cyclohexylamin umgewandelt werden.
Alternativ kann das Keton nach dem Fachmann bekannten Methoden reduktiv aminiert werden, z.B. mit Ammoniumacetat und Natriumcyanoborhydrid.
Die gebildeten Cyclohexylmethylamin-Intermediate werden mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit primären Aminen zu den korrespondierenden Harnstoffen umgesetzt.

Alternatives Syntheseschema Cyclohexylmethyl-Harnstoffe (IV):

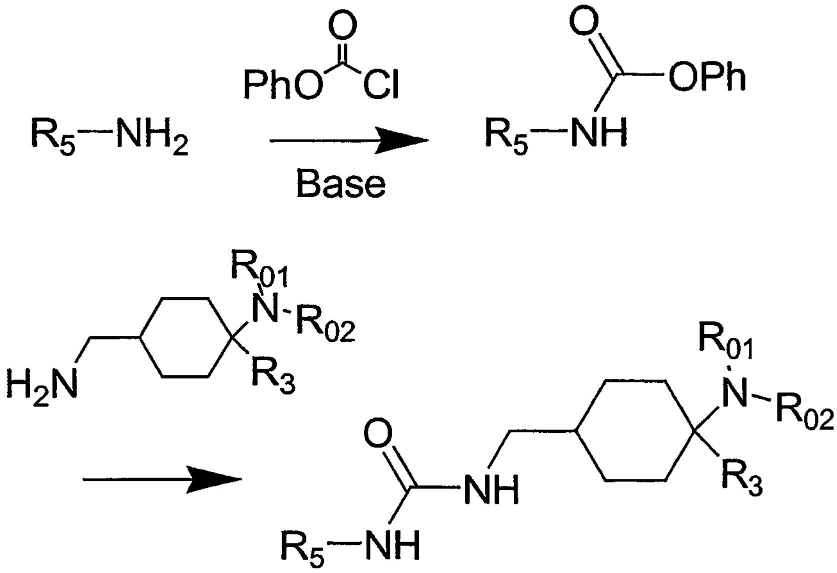


Primäre Amine werden mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit den aus Verfahren III bekannten 4-Aminomethyl-cyclohexylamin-Intermediaten zu den korrespondierenden Harnstoffen umgesetzt.

Allgemeines Syntheseschema Acylaminosäure Cyclohexylamid-Derivate (V):

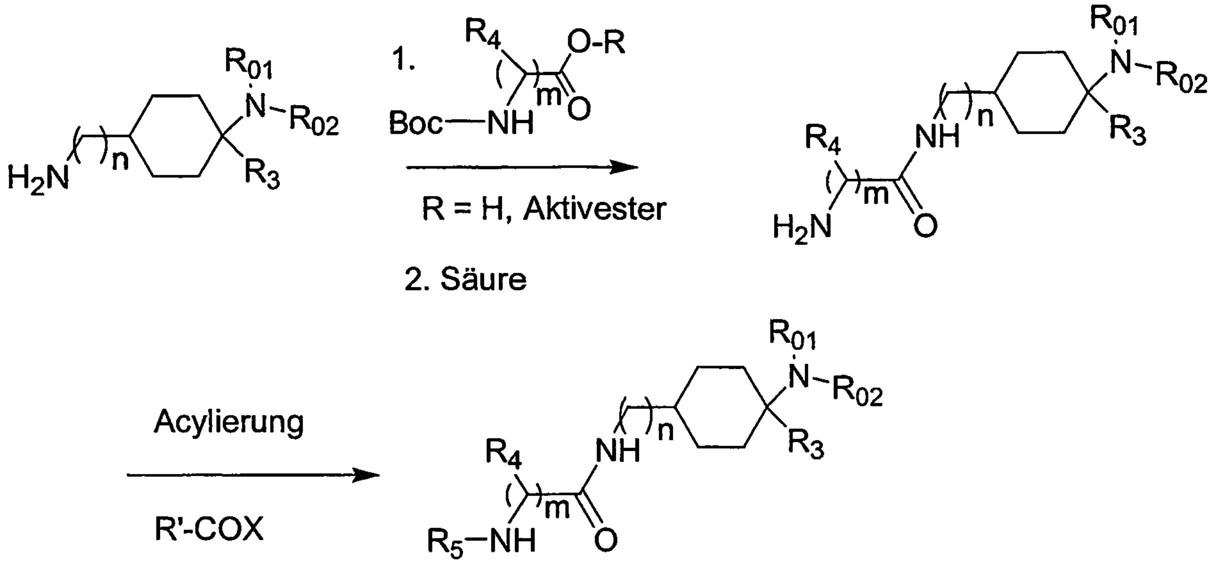


Die 4-Amino-cyclohexyl-(alkyl)-amin-Intermediate (in den Syntheseschemata I, III bzw. VII beschrieben) werden mit am Carboxyl-Terminus aktivierten Boc-Aminosäure-Derivaten (Boc = *tert.*-Butyloxycarbonyl) acyliert, die nach gängigen, literaturbekannten Methoden der Peptidkupplung (Miklos Bodanszky, Agnes Bodanszky, *The Practice of Peptide Synthesis;* Springer Verlag Heidelberg 1984) oder mit anderen geeigneten Kupplungsreagentien in situ hergestellt werden. Alternativ können die Aktivester isoliert und dann in einem nachfolgenden Schritt weiter umgesetzt werden.

Anschließend wird die Boc-Schutzgruppe sauer abgespalten.
Danach wird die entschützte Aminogruppe mit aktivierten Carbonsäure-Derivaten, z.B. Carbonsäure-halogeniden oder -aktivestern acyliert. Letztere werden entweder mit geeigneten Kupplungsreagentien wie z.B. 4-(4,6-Dimethoxy-1,3,5-triazin-2-yl)-4-methylmorpholinium-chlorid oder literaturbekannten Peptidkupplungsreagentien wie z.B. Diisopropylcarbodiimid / N-Hydroxy-benzotriazol in situ hergestellt oder als separat hergestellte Reaktanden eingesetzt.

Alternatives Syntheseschema Acylaminosäure Cyclohexylamid-Derivate (VI):

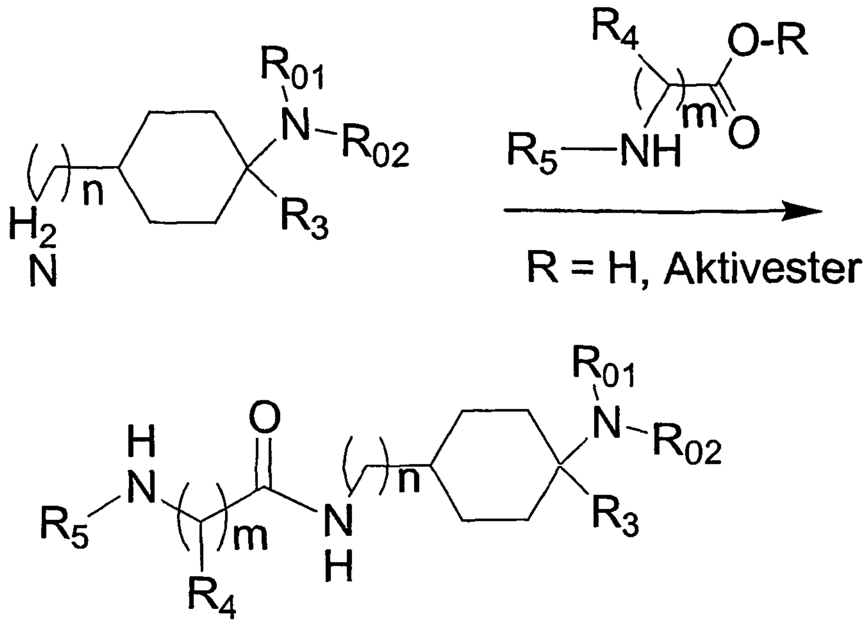


Die 4-Amino-cyclohexyl-(alkyl)-amin-Intermediate (in Syntheseschemata I, III bzw. VII beschrieben) werden mit am Carboxyl-Terminus aktivierten Aminosäure-Derivaten acyliert, die kommerziell erhältlich sind, nach gängigen, literaturbekannten Methoden der Peptidkupplung (Miklos Bodanszky, Agnes Bodanszky, *The Practice of Peptide Synthesis*; Springer Verlag Heidelberg 1984) oder mit anderen geeigneten Kupplungsreagentien in situ hergestellt werden. Alternativ können die Aktivester isoliert und dann in einem nachfolgenden Schritt weiter umgesetzt werden.

Allgemeines Syntheseschema für die Herstellung von Cyclohexylethylharnstoffen (VII):

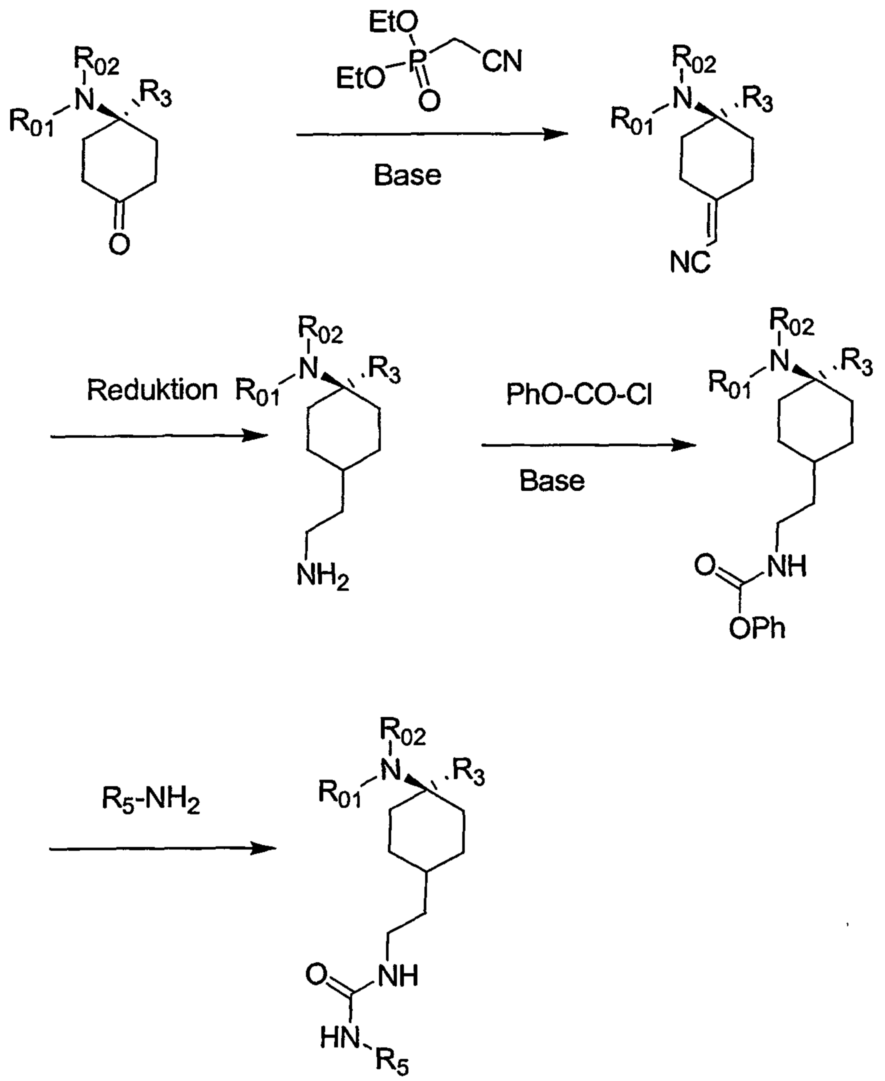


Ein 4-Aminocyclohexanon (Herstellung nach Literatur wie in Synteseschema I beschrieben) wird z.B. nach Horner mit Cyanomethanphosphonsäure-diethylester zum a,β-ungesättigten Nitril umgesetzt. Die Reduktion des Nitrils kann entweder zweistufig durch Überführung in das gesättigte Nitril, z.B. durch katalytische Hydrierung mit Wasserstoff an Palladium/Kohle und anschließende Reduktion zum Amin z.B. mit Natriumborhydrid in Gegenwart von Cobalt- oder Nickelchlorid durchgeführt werden. Bevorzugt kann die Reduktion zum gesättigten Amin einstufig durch Verwendung von z.B. mit Natriumborhydrid in Gegenwart von Cobalt- oder Nickelchlorid durchgeführt werden.
Die primäre Aminogruppe der 4-Aminoethyl-cyclohexylamine wird mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit primären Aminen zu den korrespondierenden Harnstoffen umgesetzt.

Alternatives Syntheseschema für die Herstellung von Cyclohexylethylharnstoffen (VIII):

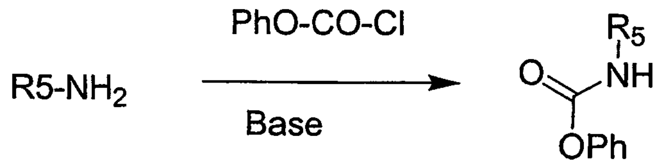


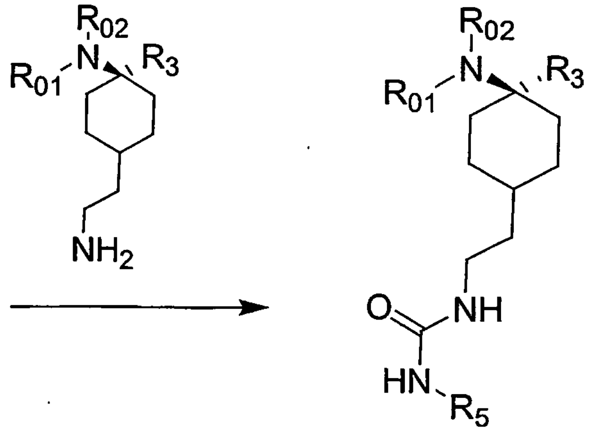

Ein primäres Amin wird mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit einem 4-Aminoethylcyclohexylamin (in Syntheseschema VII beschrieben) zu den korrespondierenden Harnstoffen umgesetzt.

Allgemeines Syntheseschema für die Herstellung von Cyclohexylpropylharnstoffen (IX):

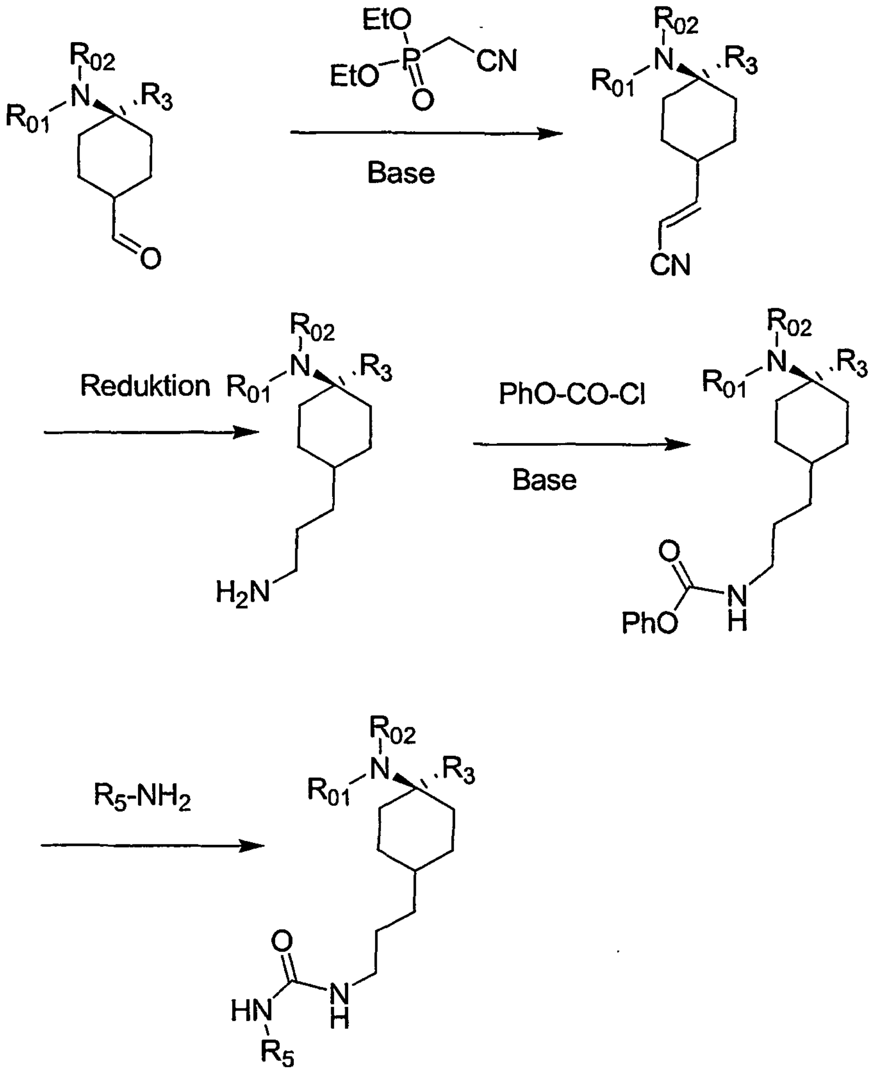


Ein 4-Aminocyclohexan-carbaldehyd (wie in Syntheseschema III beschrieben) wird z.B. nach Horner mit Cyanomethanphosphonsäure-diethylester zum $\alpha,\beta$-ungesättigten Nitril umgesetzt. Die Reduktion des Nitrils kann entweder zweistufig durch Überführung in das gesättigte Nitril, z.B. durch katalytische Hydrierung mit Wasserstoff an Palladium/Kohle und anschließende Reduktion zum Amin z.B. mit Natriumborhydrid in Gegenwart von Cobalt- oder Nickelchlorid durchgeführt werden. Bevorzugt kann die Reduktion zum gesättigten Amin einstufig durch Verwendung von z.B. Natriumborhydrid in Gegenwart von Cobalt- oder Nickelchlorid durchgeführt werden.

Die primäre Aminogruppe der 4-Aminopropyl-cyclohexylamine wird mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit primären Aminen zu den korrespondierenden Harnstoffen umgesetzt.

Alternatives Syntheseschema für die Herstellung von Cyclohexylpropylharnstoffen (X):

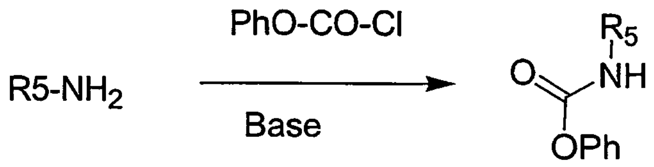


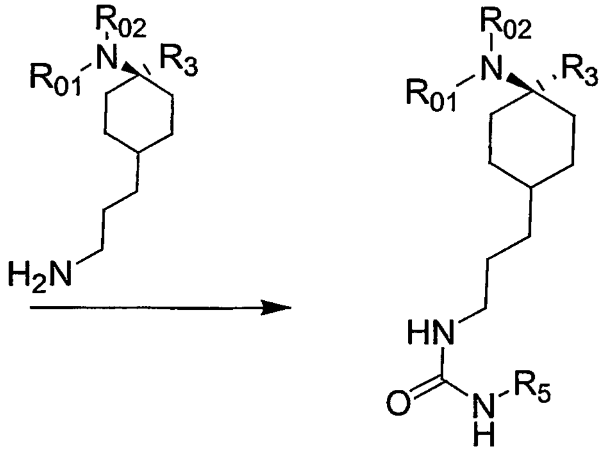

Ein primäres Amin wird mit aktivierten Kohlensäure-Derivaten wie z.B. mit Chlorameisensäure-phenylester, Chlorameisensäure-p-nitrophenylester o.ä. und einer geeigneten Base, z.B. Pyridin oder DMAP umgesetzt. Die erhaltenen Zwischenprodukte, z.B. Carbamidsäureester, werden mit einem 4-Aminopropylcyclohexylamin (in Syntheseschaema IX beschrieben) zu den korrespondierenden Harnstoffen umgesetzt.

Alternatives Syntheseschema für die Herstellung von Cyclohexyl-(alkyl)-harnstoffen unter Einsatz von Isocyanaten (XI):

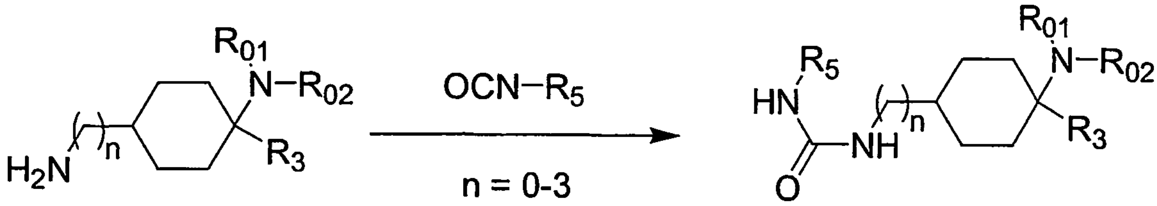


Die vorstehend beschriebenen Cyclohexyl-(alkyl)-amine können auch in einem Reaktionsschritt mit Isocyanaten carbamoyliert werden. Die Isocyanate sind entweder kommerziell erhältlich oder können nach literaturbeschriebenen Methoden, z.B. aus Phosgen oder Phosgenäquivalenten und primären Aminen, oder durch Umlagerung aus den korrespondierenden, um ein Kohlenstoff-Atom längeren Carbonsäure-Derivaten (z.B. aus Carbonsäure-aziden nach dem Curtius-Abbau) hergestellt werden.

Allgemeines Syntheseschema für die Herstellung von Cyclohexyl-(alkyl)-thioharnstoffen unter Einsatz von Isothiocyanaten (XII):

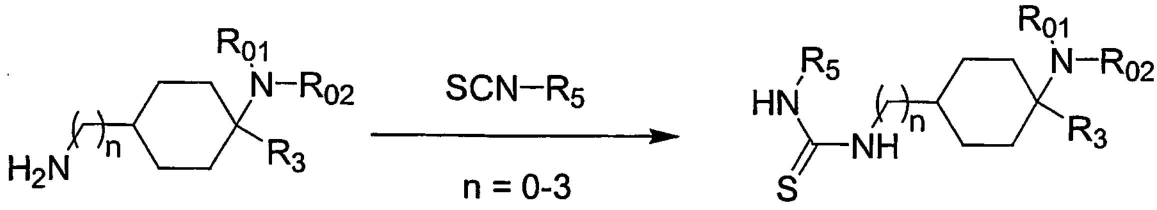


Die vorstehend beschriebenen Cyclohexyl-(alkyl)-amine können auch in einem Reaktionsschritt mit Isothiocyanaten umgesetzt werden. Die Isothiocyanate sind entweder kommerziell erhältlich oder können nach literaturbeschriebenen Methoden, z.B. aus Thiophosgen oder Thiophosgenäquivalenten und primären Aminen hergestellt werden.

Im folgenden wird die Erfindung weiter durch Beispiele erläutert, ohne sie darauf zu beschränken.

**Beispiele**

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." bedeutet Schmelzpunkt bzw. Schmelzbereich, "RT" bedeutet Raumtemperatur, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen-Nolumen angegeben.

**(3-PHENYLPROPYL)CARBAMINSÄURE-PHENYLESTER**

Zu einer Lösung von 3-Phenyl-propylamin (2,7 g, 20,0 mmol) in $CH_2Cl_2$ wurden Chlorameisensäure-phenylester (3,29 g, 21,0 mmol) und Pyridin (1,74 g, 22,0 mmol) gegeben und 24 h bei Raumtemperatur gerührt. Zur Aufarbeitung wurde der Ansatz nacheinander mit $H_2O$ mit 1M HCl und mit 1 M NaOH extrahiert. Die organische Phase wurde mit $Na_2SO_4$ getrocknet und eingedampft. Das Produkt wurde durch Umkristallisation aus Ethylacetat/Hexan (1 : 1) in einer Ausbeute von 4,11 g (81 %) als farbloser Feststoff erhalten (Smp 55-56°C).

**1-(4-DIMETHYLAMINO-4-PHENYLCYCLOHEXYL)-3-(3-PHENYLPROPYL)HARNSTOFF**

Zu einer Lösung von (3-Phenylpropyl)carbaminsäure-phenylester (255 mg, 1,0 mmol) in Dioxan wurde 4-Dimethylamino-4-phenyl-cyclohexylamin (unpolareres Diastereomer) (218 mg, 1,0 mmol) gegeben und 12 h unter Rückfluss gekocht. Zur Aufarbeitung wurde Dioxan abdestilliert und der Rückstand mit Wasser versetzt. Der Ansatz wurde mit 5M NaOH auf pH 11 eingestellt und mit Ethylacetat extrahiert. Die organische Phase wurde mit $Na_2SO_4$ getrocknet und eingedampft. Das unpolarere Produkt wurde in einer Ausbeute von 200 mg (53 %) als farbloses Öl erhalten.

Zu einer Lösung von (3-Phenylpropyl)carbaminsäure-phenylester (255 mg, 1,0 mmol) in Dioxan wurde 4-Dimethylamino-4-phenyl-cyclohexylamin (polareres Diastereomer) (218 mg, 1,0 mmol) gegeben und 12 h unter Rückfluss gekocht. Zur Aufarbeitung wurde Dioxan abdestilliert und der Rückstand mit Wasser versetzt. Der Ansatz wurde mit 5M NaOH auf pH 11 eingestellt und mit Ethylacetat extrahiert. Die organische Phase wurde mit $Na_2SO_4$ getrocknet und eingedampft. Das polarere Produkt wurde in einer Ausbeute von 379 mg (100 %) als farbloses Öl erhalten.

**Beispiele 1 und 2:**

**1-(4-DIMETHYLAMINO-4-PHENYLCYCLOHEXYL)-3-(3-PHENYLPROPYL)HARNSTOFF HYDROCHLORID**

Zur Herstellung des Hydrochlorids Beispiel 1 wurde der unpolarere Harnstoff (200 mg, 0,53 mmol) in Ethylmethylketon gelöst und mit Trimethylchlorsilan (101 μl, 0,8 mmol) versetzt. Der dabei entstandene Feststoff wurde abgesaugt und getrocknet. Das Produkt wurde so in einer Ausbeute von 219 mg (100 %) als farbloser Feststoff gewonnen (Smp. 33-35 °C).

Zur Herstellung des Hydrochlorids Beispiel 2 wurde der polarere Harnstoff (379 mg, 1,0 mmol) in Ethylmethylketon (5 ml) gelöst und mit Trimethylchlorsilan (190 μl, 1,5 mmol) versetzt. Der dabei entstandene Feststoff wurde abgesaugt und getrocknet. Das Produkt wurde so in einer Ausbeute von 416 mg (100 %) als farbloser Feststoff gewonnen. Diese Verbindung war hygroskopisch.

**[2-(1 *H*-INDOL-3-YL)ETHYLICARBAMINSÄURE-PHENYLESTER**

Zu einer Lösung von Tryptamin (3,2 g, 20,0 mmol) in $CH_2Cl_2$ wurden Chlorameisensäure-phenylester (3,29 g, 21,0 mmol) und Pyridin (1,74 g, 22,0 mmol) gegeben und 24 h bei Raumtemperatur gerührt. Zur Aufarbeitung wurde der Ansatz nacheinander mit Wasser, mit 1 M HCl und mit 1 M NaOH extrahiert. Die organische Phase wurde mit $Na_2SO_4$ getrocknet und eingedampft. Das Produkt wurde in einer Ausbeute von 5,58 g (100 %) als farbloser Feststoff erhalten (Smp 44-46 °C).

**1-(4-DIMETHYLAMINO-4-PHENYLCYCLOHEXYL)-3-[2-(1*H*-INDOL-3-YL)ETHYL]HARNSTOFF**

Zu einer Lösung von [2-(1*H*-Indol-3-yl)ethyl]carbaminsäure-phenylester (280 mg, 1,0 mmol) in Dioxan wurde 4-Dimethylamino-4-phenyl-cyclohexylamin (unpolareres Diastereomer) (218 mg, 1,0 mmol) gegeben und 12 h unter Rückfluss gekocht. Zur Aufarbeitung wurde Dioxan abdestilliert und der Rückstand mit Wasser versetzt. Der Ansatz wurde mit 5M NaOH auf pH 11 eingestellt und mit Ethylacetat extrahiert. Die organische Phase wurde mit $Na_2SO_4$ getrocknet und eingedampft. Das Produkt wurde in einer Ausbeute von 404 mg (100 %) als farbloses Öl erhalten.

Zu einer Lösung von [2-(1*H*-Indol-3-yl)ethyl]carbaminsäure-phenylester (280 mg, 1,0 mmol) in Dioxan wurde 4-Dimethylamino-4-phenyl-cyclohexylamin (polareres Diastereomer) (218 mg, 1,0 mmol) gegeben und 12 h unter Rückfluss gekocht. Zur Aufarbeitung wurde Dioxan abdestilliert und der Rückstand mit Wasser versetzt. Der Ansatz wurde mit 5M NaOH auf pH 11 eingestellt und mit Ethylacetat extrahiert. Die organische Phase wurde mit $Na_2SO_4$ getrocknet und eingedampft. Das Produkt wurde in einer Ausbeute von 404 mg (100 %) als farbloses Öl erhalten.

**BEISPIELE 3 UND 4:**

**1-(4-DIMETHYLAMINO-4-PHENYLCYCLOHEXYL)-3-[2-(1*H*-INDOL-3-YL)ETHYL]HARNSTOFF-HYDROCHLORID**

Zur Herstellung des Hydrochlorids Beispiel 3 wurde der unpolarere Harnstoff (404 mg, 1,0 mmol) in Ethylmethylketon gelöst und mit Trimethylchlorsilan (190 μl, 1,5 mmol) versetzt. Der dabei entstandene Feststoff wurde abgesaugt und getrocknet. Das Hydrochlorid Beispiel 3 wurde so in einer Ausbeute von 440 mg (100 %) als farbloser Feststoff (Smp 99-101 °C) gewonnen.

Zur Herstellung des Hydrochlorids Beispiel 4 wurde der polarere Harnstoff (404 mg, 1,0 mmol) in Ethylmethylketon gelöst und mit Trimethylchlorsilan (190 μl, 1,5 mmol) versetzt. Der dabei entstandene Feststoff wurde abgesaugt und getrocknet. Das Hydrochlorid Beispiel 4 wurde so in einer Ausbeute von 440 mg (100 %) als farbloser Feststoff (Smp 185-187 °C) gewonnen.

**[(4-DIMETHYLAMINO-4-PHENYLCYCLOHEXYLCARBAMOYL)METHYL]CARBAMINSÄURE-*TERT*-BUTYLESTER**

Zu einer Lösung von 4-Dimethylamino-4-phenyl-cyclohexylamin (unpolareres Diastereomer) (218 mg, 1,0 mmol) in Acetonitril wurde N-tert.Butyloxycarbonyl-glycin-N-hydroxysuccinimidester (272 mg, 1,0 mmol) gegeben und 24 h bei Raumtemperatur gerührt. Zur Aufarbeitung wurde der Acetonitril am Rotationsverdampfer abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das Produkt wurde in einer Ausbeute von 370 mg (99 %) als farbloser Feststoff erhalten.

Zu einer Lösung von 4-Dimethylamino-4-phenyl-cyclohexylamin (polareres Diastereomer) (840 mg, 3,85 mmol) in Acetonitril wurde wurde N-tert.Butyloxycarbonyl-glycin-N-hydroxysuccinimidester (1,05 g, 3,85 mmol) gegeben und 24 h bei Raumtemperatur gerührt. Zur Aufarbeitung wurde der Acetonitril abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das Produkt wurde in einer Ausbeute von 1,39 g (97 %) als farbloser Feststoff erhalten.

**2-Amino-*N*-(4-dimethylamino-4-phenylcyclohexyl)acetamid-hydrochlorid**

Zur Herstellung des unpolareren Hydrochlorids unter gleichzeitiger Abspaltung der Boc-Gruppe wurde das unpolarere Amid (370 mg, 1,0 mmol) in EtOH gelöst und mit 3,3M ethanolischer HCl (763 μl, 2,5 mmol) versetzt. Der dabei entstandene Feststoff wurde abfiltriert und getrocknet. Das Hydrochlorid des unpolareren Amins wurde in einer Ausbeute von 360 mg (100 %) als farbloser Feststoff (Smp 162-164 °C) gewonnen.

Zur Herstellung des polareren Hydrochlorids unter gleichzeitiger Abspaltung der Boc-Gruppe wurde das polarere Amid (1,39 g, 3,7 mmol) in EtOH gelöst und mit 3,3M ethanolischer HCl (2,82 ml, 9,3 mmol) versetzt. Der dabei entstandene Feststoff wurde abfiltriert und getrocknet. Das Hydrochlorid des polareren Amins wurde in einer Ausbeute von 1,35 g (100 %) als farbloser Feststoff gewonnen.

***N*-[(4-DIMETHYLAMINO-4-PHENYLCYCLOHEXYLCARBAMOYL)METHYL]-3-(1H-INDOL-3-YL)BUTYRAMID**

Zu einer Lösung von Indol-3-yl-buttersäure (203 mg, 1,0 mmol) in MeOH wurde das unpolarere Diastereomer von 2-Amino-N-(4-dimethylamino-4-phenylcyclohexyl)acetamid (276 mg, 1,0 mmol) und 4-(4,6-Dimethoxy-1,3,5-triazin-2-yl)-4-methylmorpholinium-chlorid (415 mg, 1,5 mmol) gegeben und 24 h bei Raumtemperatur gerührt. Zur Aufarbeitung wurde MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das unpolarere Amid wurde in einer Ausbeute von 450 mg (98 %) als farbloses Öl erhalten.

Zu einer Lösung von Indol-3-yl-buttersäure (117 mg, 0,57 mmol) in MeOH wurde das polarere Diastereomer von 2-Amino-*N*-(4-dimethylamino-4-phenylcyclohexyl)-acetamid (159 mg, 0,57 mmol) und 4-(4,6-Dimethoxy-1,3,5-triazin-2-yl)-4-methylmorpholinium-chlorid (239 mg, 0,86 mmol) gegeben und 24 h bei Raumtemperatur gerührt. Zur Aufarbeitung wurde MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das polarere Amid wurde in einer Ausbeute von 265 mg (100 %) als farbloses Öl erhalten.

**Beispiele 5 und 6:**

***N*-[(4-DIMETHYLAMINO-4-PHENYLCYCLOHEXYLCARBAMOYL)METHYL]-3-(1 H-INDOL-3-YL)BUTYRAMID-HYDROCHLORID**

Zur Herstellung des Hydrochlorids Beispiel 5 wurde das unpolarere Diastereomer von *N*-[(4-Dimethylamino-4-phenylcyclohexylcarbamoyl)methyl]-3-(1*H*-indol-3-yl)butyramid (450 mg, 0,98 mmol) in Ethylmethylketon gelöst und mit Trimethylchlorsilan (190 μl, 1,5 mmol) versetzt. Der dabei entstandene Feststoff wurde abfiltriert und getrocknet. Das Hydrochlorid Beispiel 5 wurde in einer Ausbeute von 300 mg (60 %) als farbloser Feststoff (Smp 134-136 °C) gewonnen.

Zur Herstellung des Hydrochlorids Beispiel 6 wurde das polarere Diastereomer von *N*-[(4-Dimethylamino-4-phenylcyclohexylcarbamoyl)methyl]-3-(1*H*-indol-3-yl)butyramid (265 mg, 0,57 mmol) in Ethylmethylketon gelöst und mit Trimethylchlorsilan (108 μl, 0,86 mmol) versetzt. Der dabei entstandene Feststoff wurde abfiltriert und getrocknet. Das Hydrochlorid Beispiel 6 wurde in einer Ausbeute von 225 mg (79 %) als farbloser Feststoff (Smp 148-150 °C) gewonnen.

**5-(1 *H*-Indol- 3-yl) pentansäure[(4-dimethylamino- 4-phenylcyclohexylcarbamoyl)-methyl] amid**

Zu einer Lösung von Indol-3-yl-pentansäure (217 mg, 1,0 mmol) in MeOH wurde das unpolarere Diastereomer von 2-Amino-*N*-(4-dimethylamino-4-phenylcyclohexyl)-acetamid (276 mg, 1,0 mmol) und 4-(4,6-Dimethoxy-1,3,5-triazin-2-yl)-4-methylmorpholinium-chlorid (415 mg, 1,5 mmol) gegeben und 24 h bei Raumtemperatur gerührt. Zur Aufarbeitung wurde das MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das unpolarere Amid wurde in einer Ausbeute von 474 mg (100 %) als farbloses Öl erhalten.

Zu einer Lösung von Indol-3-yl-pentansäure (125 mg, 0,57 mmol) in MeOH wurde das polarere Diastereomer von 2-Amino-*N*-(4-dimethylamino-4-phenylcyclohexyl)-acetamid (159 mg, 0,57 mmol) und 4-(4,6-Dimethoxy-1,3,5-triazin-2-yl)-4-methylmorpholinium-chlorid (239 mg, 0,86 mmol) gegeben und 24 h bei Raumtemperatur gerührt. Zur Aufarbeitung wurde MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das polarere Amid wurde in einer Ausbeute von 273 mg (100 %) als farbloses Öl erhalten.

**Beispiele 7 und 8:**

**5-(1*H*-INDOL-3-YL)-PENTANSÄURE[(4-DIMETHYLAMINO-4-PHENYLCYCLOHEXYLCARB-AMOYL)METHYL] AMID-HYDROCHLORID**

Zur Herstellung von Beispiel 7 wurde das unpolarere Diastereomer von 5-(1*H*-Indol-3-yl)pentansäure[(4-dimethylamino-4-phenylcyclohexylcarbamoyl)-methyl]amid (474 mg, 1,0 mmol) in Ethylmethylketon gelöst und mit Trimethylchlorsilan (190 µl, 1,5 mmol) versetzt. Der dabei entstandene Feststoff wurde abfiltriert und getrocknet. Das Hydrochlorid Beispiel 7 wurde in einer Ausbeute von 440 mg (86 %) als rosa Feststoff (Smp 45-47 °C) gewonnen.

Zur Herstellung von Beispiel 8 wurde das polarere Diastereomer von 5-(1 H-Indol-3-yl)pentansäure[(4-dimethylamino-4-phenylcyclohexylcarbamoyl)-methyl]amid (273 mg, 0,57 mmol) in Ethylmethylketon gelöst und mit Trimethylchlorsilan (108 µl, 0,86 mmol) versetzt. Der dabei entstandene Feststoff wurde abfiltriert und getrocknet. Das Hydrochlorid Beispiel 8 wurde in einer Ausbeute von 200 mg (68 %) als rosa Feststoff (Smp 54-56 °C) gewonnen.

**6-(1 *H*-INDOL-3-YL)HEXANSÄURE[(4-DIMETHYLAMINO-4-PHENYLCYCLOHEXYLCARBA-MOYL)METHYL] AMID**

Zu einer Lösung von Indol-3-yl-hexansäure (231 mg, 1,0 mmol) in MeOH wurde das unpolarere Diastereomer von 2-Amino-*N*-(4-dimethylamino-4-phenylcyclohexyl)-acetamid (276 mg, 1,0 mmol) und 4-(4,6-Dimethoxy-1,3,5-triazin-2-yl)-4-methyl-morpholinium-chlorid (415 mg, 1,5 mmol) gegeben und 24 h bei Raumtemperatur gerührt. Zur Aufarbeitung wurde MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das unpolarere Amid wurde in einer Ausbeute von 525 mg (100 %) als farbloses Öl erhalten.

Zu einer Lösung von Indol-3-yl-hexansäure (133 mg, 0,57 mmol) in MeOH wurde das polarere Diastereomer von 2-Amino-*N*-(4-dimethylamino-4-phenylcyclohexyl)-acetamid (159 mg, 0,57 mmol) und 4-(4,6-Dimethoxy-1,3,5-triazin-2-yl)-4-methylmorpholinium-chlorid (239 mg, 0,86 mmol) gegeben und 24 h bei Raumtemperatur gerührt. Zur Aufarbeitung wurde MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das polarere Amid wurde in einer Ausbeute von 281 mg (100 %) als farbloses Öl erhalten.

Beispiele 9 und 10:

**6-(1*H*-INDOL-3-YL)HEXANSÄURE[(4-DIMETHYLAMINO-4-PHENYLCYCLOHEXYLCARBAMOYL)-METHYL]-AMID-HYDROCHLORID**

Zur Herstellung des Hydrochlorids Beispiel 9 wurde das unpolarere Diastereomer von 6-(1 H-Indol-3-yl)hexansäure[(4-dimethylamino-4-phenylcyclohexylcarbamoyl)-methyl]amid (525 mg, 1,0 mmol) in Ethylmethylketon gelöst und mit Trimethylchlorsilan (190 µl, 1,5 mmol) versetzt. Der dabei entstandene Feststoff wurde abfiltriert und getrocknet. Das Hydrochlorid Beispiel 9 wurde in einer Ausbeute von 402 mg (82 %) als rosa Feststoff (Smp 32-34 °C) gewonnen.

Zur Herstellung des Hydrochlorids Beispiel 10 wurde das polarere Diastereomer von 6-(1 *H*-Indol-3-yl)hexansäure[(4-dimethylamino-4-phenylcyclohexylcarbamoyl)-methyl]amid (281 mg, 0,57 mmol) in Ethylmethylketon gelöst und mit Trimethylchlorsilan (108 µl, 0,86 mmol) versetzt. Der dabei entstandene Feststoff wurde abfiltriert und getrocknet. Das Hydrochlorid Beispiel 10 wurde in einer Ausbeute von 300 mg (99 %) als rosa Feststoff (Smp 31-33 °C) gewonnen.

***N*-[(4-DIMETHYLAMINO-4-PHENYLCYCLOHEXYLCARBAMOYL)METHYL]-3-(1 *H*-INDOL-3-YL)PROPIONAMID**

Zu einer Lösung von Indol-3-yl-propionsäure (189 mg, 1,0 mmol) in MeOH wurde das unpolarere Diastereomer von 2-Amino-*N*-(4-dimethylamino-4-phenylcyclohexyl)-acetamid (276 mg, 1,0 mmol) und 4-(4,6-Dimethoxy-1 ,3,5-triazin-2-yl)-4-methylmorpholinium-chlorid (415 mg, 1,5 mmol) gegeben und 24 h bei Raumtemperatur gerührt. Zur Aufarbeitung wurde MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das unpolarere Amid wurde in einer Ausbeute von 446 mg (100 %) als farbloses Öl erhalten.

Zu einer Lösung von Indol-3-yl-propionsäure (109 mg, 0,57 mmol) in MeOH wurde das polarere Diastereomer von 2-Amino-*N*-(4-dimethylamino-4-phenylcyclohexyl)-acetamid (159 mg, 0,57 mmol) und 4-(4,6-Dimethoxy-1,3,5-triazin-2-yl)-4-methylmorpholinium-chlorid (239 mg, 0,86 mmol) gegeben und 24 h bei Raumtemperatur gerührt. Zur Aufarbeitung wurde MeOH abdestilliert. Der Ansatz wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das polarere Amid wurde in einer Ausbeute von 257 mg (100 %) als farbloses Öl erhalten.

**Beispiele 11 und 12:**

**N-[(4-Dimethylamino-4-phenylcyclohexylcarbamoyl)methyl]-3-(1 *H*-indol-3-yl)propionamid-hydrochlorid**

Zur Herstellung des Hydrochlorids Beispiel 11 wurde das unpolarere Diastereomer von *N*-[(4-Dimethylamino-4-phenylcyclohexylcarbamoyl)methyl]-3-(1*H*-indol-3-yl)propionamid (446 mg, 1,0 mmol) in Ethylmethylketon gelöst und mit Trimethylchlorsilan (190 µl, 1,5 mmol) versetzt. Der dabei entstandene Feststoff wurde abfiltriert und getrocknet. Beispiel 11 wurde in einer Ausbeute von 370 mg (77 %) als farbloser Feststoff (Smp von 143-145 °C) gewonnen.

Zur Herstellung des Hydrochlorids Beispiel 12 wurde das polarere Diastereomer von *N*-[(4-Dimethylamino-4-phenylcyclohexylcarbamoyl)methyl]-3-(1*H*-indol-3-yl)propionamid (257 mg, 0,57 mmol) in Ethylmethylketon gelöst und mit Trimethylchlorsilan (108 µl, 0,86 mmol) versetzt. Der dabei entstandene Feststoff wurde abfiltriert und getrocknet. Beispiel 12 wurde in einer Ausbeute von 220 mg (79 %) als farbloser Feststoff (Smp 135-137 °C) gewonnen.

***N*-(4-DIMETHYLAMINO-4-PHENYLCYCLOHEXYL)-2-(2-1 *H*-INDOL-3-YLACETYLAMINO)-PROPIONAMID**

Zu einer Lösung von *N*-(Indolyl-3-yl-acetyl)-L-alanin (123 mg, 0,5 mmol) in MeOH wurde das unpolarere Diastereomer von (4-Dimethylamino-4-phenylcyclohexyl)amin (109 mg, 0,5 mmol) und 4-(4,6-Dimethoxy-1,3,5-triazin-2-yl)-4-methylmorpholinium-chlorid (208 mg, 0,75 mmol) gegeben und 24 h bei Raumtemperatur gerührt. Zur Aufarbeitung wurde MeOH abdestilliert. Der Rückstand wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das unpolarere Amid wurde in einer Ausbeute von 110 mg (49 %) als farbloses Öl erhalten.

Zu einer Lösung von *N*-(Indolyl-3-yl-acetyl)-L-alanin (161 mg, 0,66 mmol) in MeOH wurde das polarere Diastereomer von (4-Dimethylamino-4-phenylcyclohexyl)amin (143 mg, 0,66 mmol) und 4-(4,6-Dimethoxy-1,3,5-triazin-2-yl)-4-methylmorpholinium-chlorid (272 mg, 0,98 mmol) gegeben und 24 h bei Raumtemperatur gerührt. Zur Aufarbeitung wurde MeOH abdestilliert. Der Rückstand wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das polarere Amid wurde in einer Ausbeute von 297 mg (100 %) als farbloses Öl erhalten.

**Beispiele 13 und 14:**

**N-(4-DIMETHYLAMINO-4-PHENYLCYCLOHEXYL)-2-(2-1 *H*-INDOL-3-YLACETYLAMINO)-PROPIONAMID-HYDROCHLORID**

Zur Herstellung des Hydrochlorids **Beispiel 13** wurde das unpolarere Diastereomer von *N*-(4-Dimethylamino-4-phenylcyclohexyl)-2-(2-1*H*-indol-3-ylacetylamino)-propionamid (110 mg, 0,25 mmol) in Ethylmethylketon gelöst und mit Trimethylchlorsilan (48 µl, 0,38 mmol) versetzt. Der dabei entstandene Feststoff wurde abfiltriert und getrocknet. Beispiel 13 wurde in einer Ausbeute von 119 mg (100 %) als farbloser Feststoff (Smp 98-100 °C) gewonnen.

Zur Herstellung des Hydrochlorids **Beispiel 14** wurde das polarere Diastereomer von *N*-(4-Dimethylamino-4-phenylcyclohexyl)-2-(2-1*H*-indol-3-ylacetylamino)propionamid (297 mg, 0,66 mmol) in Ethylmethylketon gelöst und mit Trimethylchlorsilan (125 µl, 0,99 mmol) versetzt. Der dabei entstandene Feststoff wurde abfiltriert und getrocknet. Beispiel 14 wurde in einer Ausbeute von 200 mg (63 %) als farbloser Feststoff (Smp 30-32 °C) gewonnen.

**2-(2-1*H*-INDOL-3-YLACETYLAMINO)-4-METHYLPENTANSÄURE-(4-DIMETHYLAMINO-4-PHENYLCYCLOHEXYL)AMID**

Zu einer Lösung von *N*-(3-Indolylacetyl)-L-leucin (144 mg, 0,5 mmol) in MeOH wurde das unpolarere Diastereomer von (4-Dimethylamino-4-phenylcyclohexyl)amin (109 mg, 0,5 mmol) und 4-(4,6-Dimethoxy-1,3,5-triazin-2-yl)-4-methylmorpholinium-chlorid (208 mg, 0,75 mmol) gegeben und 24 h bei Raumtemperatur gerührt. Zur Aufarbeitung wurde MeOH abdestilliert. Der Rückstand wurde mit Wasser aufgenommen, mit 5M NaOH auf pH 11 eingestellt und mit Ethylacetat extrahiert. Die organische Phase wurde mit $Na_2SO_4$ getrocknet und eingedampft. Das Produkt wurde in einer Ausbeute von 115 mg (47 %) als farbloses Öl erhalten.

Zu einer Lösung von *N*-(3-Indolylacetyl)-L-leucin (189 mg, 0,66 mmol) in MeOH wurde das polarere Diastereomer von (4-Dimethylamino-4-phenylcyclohexyl)amin (143 mg, 0,66 mmol) und 4-(4,6-Dimethoxy-1,3,5-triazin-2-yl)-4-methylmorpholinium-chlorid (272 mg, 0,98 mmol) gegeben und 24 h bei Raumtemperatur gerührt. Zur Aufarbeitung wurde MeOH abdestilliert. Der Rückstand wurde mit Wasser aufgenommen, mit 5M NaOH auf pH 11 eingestellt und mit Ethylacetat extrahiert. Die organische Phase wurde mit $Na_2SO_4$ getrocknet und anschließend eingedampft. Das Produkt wurde in einer Ausbeute von 320 mg (100 %) als farbloses Öl erhalten.

**Beispiele 15 und 16:**

**2-(2-1 *H*-INDOL-3-YLACETYLAMINO)-4-METHYLPENTANSÄURE-(4-DIMETHYLAMINO-4-PHENYLCYCLOHEXYL)AMID-HYDROCHLORID**

Zur Herstellung des Hydrochlorids **Beispiel 15** wurde das unpolarere Derivat von 2-(2-1 H-Indol-3-ylacetylamino)-4-methylpentansäure-(4-dimethylamino-4-phenylcyclohexyl)amid (115 mg, 0,24 mmol) in Ethylmethylketon gelöst und mit Trimethylchlorsilan (48 µl, 0,38 mmol) versetzt. Der dabei entstandene Feststoff wurde abfiltriert und getrocknet. **Beispiel 15** wurde in einer Ausbeute von 123 mg (100 %) als farbloser Feststoff (Smp 118-120 °C) gewonnen.

Zur Herstellung des Hydrochlorids **Beispiel 16** wurde das polarere Derivat von 2-(2-1*H*-Indol-3-ylacetylamino)-4-methylpentansäure-(4-dimethylamino-4-phenylcyclohexyl)amid (320 mg, 0,66 mmol) in Ethylmethylketon gelöst und mit Trimethylchlorsilan (125 µl, 0,99 mmol) versetzt. Der dabei entstandene Feststoff wurde abfiltriert und getrocknet. **Beispiel 16** wurde in einer Ausbeute von 170 mg (50 %) als farbloser Feststoff (Smp 174-176 °C) gewonnen.

**1-(4-Dimethylamino-4-phenylcyclohexyl)-3-[2-(1*H*-indol-3-yl)ethyl]-thioharnstoff**

2-(1 H-Indol-3-yl)-ethylamin (Tryptamin; 320 mg, 2 mmol) wurde in trockenem Chloroform (10 ml) gelöst und mit Triethylamin (555 µl, 4 mmol) versetzt. Zu diesem Gemisch wurde Thiophosgen (153 µl, 2 mmol) hinzugefügt. Nach einer Reaktionszeit von 16 h wurde N,N-Dimethyl-1-phenyl-cyclohexan-1,4-diamin zugesetzt und weitere 16 h bei RT gerührt. Zur Aufarbeitung wurde der Ansatz mit gesättigter $NaHCO_3$-Lösung (3 × 20 ml) extrahiert. Die organische Phase wurde mit $Na_2SO_4$ getrocknet und eingedampft. Das Produkt war ein Gemisch aus zwei Diastereoisomeren und konnte durch Säulenchromatographie [Kieselgel 60 (50 g); Methanol (500 ml)] gereinigt werden. Das polarere Diastereoisomer von 1-(4-Dimethylamino-4-phenyl-cyclohexyl)-3-[2-(1 H-indol-3-yl)-ethyl]-thioharnstoff wurde in einer Ausbeute von 185 mg (22 %) als farbloser Schaum erhalten. Das unpolarere Diastereoisomer von 1-(4-Dimethylamino-4-phenyl-cyclohexyl)-3-[2-(1 H-indol-3-yl)-ethyl]-thioharnstoff wurde in einer Ausbeute von 433 mg (52 %) als farbloser Schaum erhalten.

**[2-(1 *H*-INDOL-3-YL)-1-METHYLETHYL]CARBAMINSÄURE-PHENYLESTER**

Zu einer Lösung von 2-(1H-Indol-3-yl)-1-methyl-ethylamine (1,0 g, 5,74 mmol) in abs. DCM (20 ml) wurde Chlorameisensäure-phenylester (760 µl, 6,03 mmol) und Pyridin (510 µl, 6,31 mmol) gegeben. Anschließend wurde 24 h bei RT gerührt. Zur Aufarbeitung wurde der Ansatz mit Wasser (2 × 20 ml), mit 1 M HCl (2 × 20 ml) und mit 1 M NaOH (2 × 20 ml) extrahiert. Die organische Phase wurde mit $Na_2SO_4$ getrocknet und eingedampft. [2-(1*H*-Indol-3-yl)-1-methylethyl]carbaminsäure-phenylester wurde in einer Ausbeute von 1,35 g (80 %) als farbloser Feststoff erhalten.

**1-(4-Dimethylamino-4-phenylcyclohexyl)-3-[2-(1*H*-indol-3-yl)-1-methylethyl]harnstoff**

Zu einer Lösung von [2-(1H-Indol-3-yl)-1-methyl-ethyl]-carbaminsäure-phenylester (147 mg, 0,5 mmol) in Dioxan (10 ml) wurde das unpolarere Diastereoisomer von *N,N*-Dimethyl-1-phenyl-cyclohexan-1,4-diamin (109 mg, 0,5 mmol) gegeben. Anschließend wurde 6 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das unpolarere Diastereoisomer von 1-(4-Dimethylamino-4-phenylcyclohexyl)-3-[2-(1*H*-indol-3-yl)-1-methylethyl]harnstoff wurde nach Chromatographie an Kieselgel mit Methanol in einer Ausbeute von 125 mg (60 %) als farbloses Öl erhalten.

Zu einer Lösung von [2-(1 H-Indol-3-yl)-1-methyl-ethyl]-carbaminsäure-phenylester (147 mg, 0,5 mmol) in Dioxan (10 ml) wurde das polarere Diastereoisomer von *N,N*-Dimethyl-1-phenyl-cyclohexan-1,4-diamin (109 mg, 0,5 mmol) gegeben. Anschließend wurde 6 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das polarere Diastereoisomer von 1-(4-Dimethylamino-4-phenylcyclohexyl)-3-[2-(1*H*-indol-3-yl)-1-methylethyl]harnstoff wurde nach Chromatographie an Kieselgel mit Methanol in einer Ausbeute von 100 mg (48 %) als farbloses Öl erhalten.

**Beispiele 17 und 18:**

**1-(4-DIMETHYLAMINO-4-PHENYLCYCLOHEXYL)-3-[2-(1*H*-INDOL-3-YL)-1-METHYLETHYL]HARNSTOFF-HYDROCHLORID**

Zur Herstellung von 1-(4-Dimethylamino-4-phenylcyclohexyl)-3-[2-(1 *H*-indol-3-yl)-1-methylethyl]harnstoff-hydrochlorid (Beispiel 17) wurde das unpolarere Diastereoisomer von 1-(4-Dimethylamino-4-phenylcyclohexyl)-3-[2-(1*H*-indol-3-yl)-1-methylethyl]harnstoff (125 mg, 0,3 mmol) in Ethylmethylketon (3 ml) gelöst und mit Trimethylchlorsilan (57 µl, 0,45 mmol) versetzt. Der dabei entstandene Feststoff wurde abfiltriert und getrocknet. Das Hydrochlorid des unpolareren Diastereoisomers von 1-(4-Dimethylamino-4-phenylcyclohexyl)-3-[2-(1*H*-indol-3-yl)-1-methylethyl]harnstoff wurde so in einer Ausbeute von 135 mg (100 %) als farbloser Feststoff mit einem Schmelzpunkt von 154-156 °C gewonnen.

Zur Herstellung des Hydrochlorids (Beispiel 18) wurde das polarere Diastereoisomer von 1-(4-Dimethylamino-4-phenylcyclohexyl)-3-[2-(1*H*-indol-3-yl)-1-methylethyl]harnstoff (100 mg, 0,24 mmol) in Ethylmethylketon (3 ml) gelöst und mit Trimethylchlorsilan (46 µl, 0,36 mmol) versetzt. Der dabei entstandene Feststoff wurde abfiltriert und getrocknet. Das Hydrochlorid des polareren Diastereoisomers von 1-(4-Dimethylamino-4-phenylcyclohexyl)-3-[2-(1 *H*-indol-3-yl)-1-methylethyl]harnstoff wurde so in einer Ausbeute von 108 mg (100 %) als farbloser Feststoff mit einem Schmelzpunkt von 175-177 °C gewonnen.

**[2-(5-Fluor-1*H*-indol-3-yl)ethyl]carbaminsäure-phenylester**

Zu einer Lösung von 2-(5-Fluoro-1 H-indol-3-yl)-ethylamine (0,5 g, 2,33 mmol) in abs. DCM (10 ml) wurde Chlorameisensäure-phenylester (310 µl, 2,45 mmol) und Pyridin (415 µl, 5,13 mmol) gegeben. Anschließend wurde 24 h bei RT gerührt. Zur Aufarbeitung wurde der Ansatz mit Wasser (2 × 20 ml), mit 1 M HCl (2 × 20 ml) und mit 1 M NaOH (2 × 20 ml) extrahiert. Die organische Phase wurde mit $Na_2SO_4$ getrocknet und eingedampft. [2-(5-Fluoro-1 H-indol-3-yl)-ethyl]-carbaminsäure-phenylester wurde in einer Ausbeute von 0,69 g (100 %) als farbloser Feststoff erhalten.

**1-(4-Dimethylamino-4-phenylcyclohexyl)-3-[2-(5-Fluor-1 H-indol-3-yl)ethyl]harnstoff**

Zu einer Lösung von [2-(5-Fluoro-1 H-indol-3-yl)-ethyl]-carbaminsäure-phenylester (298 mg, 1,0 mmol) in Dioxan (20 ml) wurde das unpolarere Diastereoisomer von 4-Dimethylamino-4-phenyl-cyclohexylamin (218 mg, 1,0 mmol) gegeben. Anschließend wurde 8 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das unpolarere Diastereoisomer von 1-(4-Dimethylamino-4-phenylcyclohexyl)-3-[2-(5-Fluor-1*H*-indol-3-yl)ethyl]hamstoff wurde nach Chromatographie an Kieselgel mit Methanol in einer Ausbeute von 140 mg (33 %) als farbloses Öl erhalten.

Zu einer Lösung von [2-(5-Fluoro-1 H-indol-3-yl)-ethyl]-carbaminsäure-phenylester (370 mg, 1,24 mmol) in Dioxan (20 ml) wurde das polarere Diastereoisomer von 4-Dimethylamino-4-phenyl-cyclohexylamin (271 mg, 1,24 mmol) gegeben. Anschließend wurde 8 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das polarere Diastereoisomer von 1-(4-Dimethylamino-4-phenylcyclohexyl)-3-[2-(5-Fluor-1*H*-indol-3-yl)ethyl]harnstoff wurde nach Chromatographie an Kieselgel mit Methanol in einer Ausbeute von 267 mg (51 %) als farbloses Öl erhalten.

Beispiele 19 und 20:

**1-(4-Dimethylamino-4-phenylcyclohexyl)-3-[2-(5-Fluor-1*H*-indol-3-yl)ethyl]harnstoff-hydrochlorid**

Zur Herstellung des Hydrochlorids (Verbindung 19) wurde das unpolarere Diastereoisomer von 1-(4-Dimethylamino-4-phenylcyclohexyl)-3-[2-(5-Fluor-1 *H*-indol-3-yl)ethyl]harnstoff (140 mg, 0,33 mmol) in Ethylmethylketon (3 ml) gelöst und mit Trimethylchlorsilan (60 µl, 0,45 mmol) versetzt. Der dabei entstandene Feststoff wurde abfiltriert und getrocknet. Das Hydrochlorid des unpolareren Diastereoisomers von 1-(4-Dimethylamino-4-phenylcyclohexyl)-3-[2-(5-Fluor-1*H*-indol-3-yl)ethyl]harnstoff wurde so in einer Ausbeute von 152 mg (100 %) als farbloser Feststoff mit einem Schmelzpunkt von 197-199 °C gewonnen.

Zur Herstellung des Hydrochlorids (Verbindung 20) wurde das polarere Diastereoisomer von 1-(4-Dimethylamino-4-phenylcyclohexyl)-3-[2-(5-Fluor-1*H*-indol-3-yl)ethyl]harnstoff (267 mg, 0,63 mmol) in Ethylmethylketon (5 ml) gelöst und mit Trimethylchlorsilan (120 µl, 0,95 mmol) versetzt. Der dabei entstandene Feststoff wurde abfiltriert und getrocknet. Das Hydrochlorid des polareren Diastereoisomers von 1-(4-Dimethylamino-4-phenylcyclohexyl)-3-[2-(5-Fluor-1 *H*-indol-3-yl)ethyl]harnstoff wurde so in einer Ausbeute von 290 mg (100 %) als farbloser Feststoff mit einem Schmelzpunkt von 219-221 °C gewonnen.

**1-(4-Dimethylamino-4-phenylcyclohexylmethyl)-3-[2-(1*H*-indol-3-yl)ethyl]-harnstoff**

Zu einer Lösung von [2-(1 H-Indol-3-yl)-ethyl]-carbaminsäure-phenylester (560,6 mg, 2,0 mmol) in Dioxan (15

ml) wurde das Gemisch von (4-Aminomethyl-1-phenyl-cyclohexyl)-dimethylamin (464,8 mg, 2,0 mmol) gegeben. Anschließend wurde 12 h unter Rückfluss gekocht. Zur Aufarbeitung wurde Dioxan abdestilliert und der Rückstand mit Wasser (10 ml) versetzt. Der Ansatz wurde mit 5M NaOH auf pH 11 eingestellt und mit EE (3 × 20 ml) extrahiert. Dabei trat eine kleine Menge Feststoff auf. Der Feststoff, der analytisch dem polareren Diastereoisomer von 1-(4-Dimethylamino-4-phenylcyclohexylmethyl)-3-[2-(1H-indol-3-yl)ethyl]hamstoff (100 mg, Fp. 204-208 °C, 12 %) entsprach, wurde abfiltriert, mit Diethylether (2 × 2 ml) gewaschen und getrocknet. Die vereinigten EE-Extrakte wurden mit 1 M NaOH (5 ml) gewaschen und mit $Na_2SO_4$ getrocknet. Das Lösungsmittel wurde im Vakuum abdestilliert. Der Rückstand war das Diastereoisomeren-Gemisch von 1-(4-Dimethylamino-4-phenylcyclohexylmethyl)-3-[2-(1*H*-indol-3-yl)ethyl]harnstoff, das durch Flash-Chromatographie an Kieselgel (50 g) getrennt und gereinigt wurde. Als Eluent wurde Ethanol/EE (1 : 1, 1100 ml) eingesetzt. Das unpolarere Diastereoisomer von 1-(4-Dimethylamino-4-phenylcyclohexylmethyl)-3-[2-(1 *H*-indol-3-yl)ethyl]harnstoff (264 mg, Fp. 159-163 °C, 32 %) und ein weiterer Teil des polareren Diastereoisomers von 1-(4-Dimethylamino-4-phenylcyclohexylmethyl)-3-[2-(1*H*-indol-3-yl)ethyl]harnstoff (55 mg, Fp. 202-207 °C, 7 %) wurden so isoliert.

**Beispiele 21 und 22:**

**1-(4-Dimethylamino-4-phenylcyclohexylmethyl)-3-[2-(1 H-indol-3-yl)ethyl]-harnstoff-hydrochlorid**

Das unpolarere Diastereoisomer von 1-(4-Dimethylamino-4-phenylcyclohexylmethyl)-3-[2-(1 *H*-indol-3-yl) ethyl]harnstoff (256 mg, 0,6 mmol) wurde in Ethylmethylketon (15 ml) gelöst. Unter Rühren wurde bei RT 5M isopropanolische Salzsäure (184 µl, 0,92 mmol) tropfenweise zugegeben. Nach einstündigem Rühren bei RT war ein Niederschlag ausgefallen. Der Feststoff wurde abfiltriert, mit Diethylether (3 × 1,5 ml) gewaschen und im Vakuum getrocknet. Das Hydrochlorid (Beispiel 21) des unpolareren Harnstoffes wurde so in einer Ausbeute von 272 mg (99 %) als farbloser Feststoff gewonnen.

Das polarere Diastereoisomer von 1-(4-Dimethylamino-4-phenylcyclohexylmethyl)-3-[2-(1 *H*-indol-3-yl)ethyl] harnstoff (128 mg, 0,3 mmol) wurde in abs. Ethanol (15 ml) und Ethylmethylketon (5 ml) gelöst. Unter Rühren wurde bei RT 5M isopropanolische Salzsäure (92 µl, 0,46 mmol) tropfenweise zugegeben. Nach einstündigem Rühren bei RT war kein Niederschlag ausgefallen. Die Reaktionslösung wurde im Vakuum auf ca. 2 ml reduziert und mit Diethylether (10 ml) versetzt. Der klebrige Niederschlag wurde mechanisch von der Kolbenwand gelöst. Die Suspension wurde bei RT 1 h kräftig gerührt. Es bildete sich ein feiner, heller Niederschlag. Der Feststoff wurde abfiltriert, mit Diethylether (3 × 1,5 ml) gewaschen und im Vakuum getrocknet. Das Hydrochlorid (Beispiel 22) des polareren Harnstoffes wurde so in einer Ausbeute von 137,2 mg (99 %) als cremefarbener Feststoff gewonnen.

**1-(4-Dimethylamino-4-phenylcyclohexylmethyl)-3-(3-phenylpropyl)harnstoff**

Zu einer Lösung von (3-Phenyl-propyl)-carbaminsäure-phenylester (510,6 mg, 2,0 mmol) in Dioxan (15 ml) wurde das Diastereoisomerengemisch von (4-Aminomethyl-1-phenyl-cyclohexyl)-dimethyl-amin (465,8 mg, 2,0 mmol) gegeben. Anschließend wurde 12 h unter Rückfluss gekocht. Zur Aufarbeitung wurde Dioxan am Rotationsverdampfer abdestilliert und der Rückstand mit Wasser (10 ml) versetzt. Der Ansatz wurde mit 5M NaOH auf pH 11 eingestellt und mit EE (3 × 20 ml) extrahiert. Die vereinigten EE-Extrakte wurden mit 1 M NaOH (1 × 5 ml) gewaschen und mit $Na_2SO_4$ getrocknet. Das Lösungsmittel wurde im Vakuum abdestilliert. Der Rückstand war das Diastereoisomerengemisch von 1-(4-Dimethylamino-4-phenyl-cyclohexylmethyl)-3-(3-phenyl-propyl)-harnstoff, das durch Flash-Chromatographie an Kieselgel (60 g) getrennt und gereinigt wurde. Als Eluent wurde Ethanol/EE (1:1, 1100 ml) eingesetzt. Das unpolarere Diastereoisomer von 1-(4-Dimethylamino-4-phenyl-cyclohexylmethyl)-3-(3-phenyl-propyl)-harnstoff (310 mg, gelbes Öl, 39 %) und das polarere Diastereoisomer von 1-(4-Dimethylamino-4-phenyl-cyclohexylmethyl)-3-(3-phenyl-propyl)-harnstoff (55 mg, Fp. 159-165 °C, 32 %) wurden so isoliert.

**Beispiele 23 und 24:**

**1-(4-Dimethylamino-4-phenylcyclohexylmethyl)-3-(3-phenylpropyl)harnstoff-hydrochlorid**

Das unpolarere Diastereoisomer von 1-(4-Dimethylamino-4-phenyl-cyclohexylmethyl)-3-(3-phenyl-propyl)-harnstoff (310 mg, 0,79 mmol) wurde in Aceton (5 ml) und Ethylmethylketon (15 ml) gelöst. Unter Rühren wurde bei RT Chlortrimethylsilan (150 µl, 1,2 mmol) tropfenweise zugegeben. Nach einstündigem Rühren war kein Niederschlag ausgefallen. Die Reaktionslösung wurde im Vakuum auf ca. 1 ml reduziert und mit Diethylether (10 ml) versetzt. Danach wurde bei RT 1 h kräftig gerührt. Es bildete sich ein farbloser Niederschlag. Der Feststoff wurde abfiltriert, mit Diethylether (3 × 3 ml) gewaschen und im Vakuum getrocknet. Das Hydrochlorid des unpolareren Diastereoisomers von 1-(4-Dimethylamino-4-phenyl-cyclohexylmethyl)-3-(3-phenyl-propyl)-harnstoff (339 mg, Fp. 85-95 °C, 100 %; Beispiel 23)

wurde als farbloser Feststoff gewonnen.

Das polarere Diastereoisomer von 1-(4-Dimethylamino-4-phenyl-cyclohexylmethyl)-3-(3-phenyl-propyl)-harnstoff (249 mg, 0,63 mmol) wurde in Aceton (7 ml) und Ethylmethylketon (15 ml) gelöst. Unter Rühren wurde bei RT Chlortrimethylsilan (121 μl, 0,95 mmol) tropfenweise zugegeben. Nach einstündigem Rühren war kein Niederschlag ausgefallen. Die Reaktionslösung wurde im Vakuum auf ca. 0,5 ml reduziert und mit Diethylether (10 ml) versetzt. Danach wurde bei RT 1 h kräftig gerührt. Es bildete sich ein fast farbloser Niederschlag. Der Feststoff wurde abfiltriert, mit Diethylether (3 × 2,5 ml) gewaschen und im Vakuum getrocknet. Das Hydrochlorid des polareren Diastereoisomers von 1-(4-Dimethylamino-4-phenyl-cyclohexylmethyl)-3-(3-phenyl-propyl)-harnstoff (270 mg, Fp. 100-110 °C, 99 %;

Beispiel 24) wurde als graufarbloser Feststoff gewonnen.

**1-(4-Dimethylamino-4-phenylcyclohexylmethyl)-3-[2-(1 *H*-indol-3-yl)-1- methylethyl]harnstoff**

Zu einer Lösung von [2-(1 H-Indol-3-yl)-1-methyl-ethyl]-carbaminsäure-phenylester (206 mg, 07 mmol) in Dioxan (7 ml) wurde das unpolarere Diastereoisomer von (4-Aminomethyl-1-phenyl-cyclohexyl)-dimethyl-amin (162,7 mg, 0,7 mmol) gegeben. Anschließend wurde 14 h unter Rückfluss gekocht. Zur Aufarbeitung wurde das Reaktionsgemisch auf Eiswasser (15 ml) gegeben, mit 5M NaOH auf pH 11 eingestellt und mit Ether (3 × 20 ml) extrahiert. Die organische Phase wurde mit $Na_2SO_4$ getrocknet und anschließend eingedampft. Der Rückstand wurde durch Flash-Chromatographie an Kieselgel (30 g) gereinigt. Als Eluent wurde Ethanol/EE (1 : 2, 400 ml) eingesetzt. Das unpolarere Diastereoisomer von 1-(4-Dimethylamino-4-phenyl-cyclohexylmethyl)-3-[2-(1 H-indol-3-yl)-1-methyl-ethyl]-harnstoff wurde so in einer Ausbeute von 185 mg (61 %) als farbloser Feststoff mit einem Schmelzpunkt von 89-92 °C erhalten.

Zu einer Lösung von [2-(1H-Indol-3-yl)-1-methyl-ethyl]-carbaminsäure-phenylester (206 mg, 0,7 mmol) in Dioxan (7 ml) wurde das polarere Diastereoisomer von (4-Aminomethyl-1-phenyl-cyclohexyl)-dimethyl-amin (162,7 mg, 0,7 mmol) gegeben. Anschließend wurde 14 h unter Rückfluss gekocht. Bei RT fiel ein Niederschlag aus. Der Niederschlag wurde abfiltriert, einmal mit kaltem Dioxan (2 ml) und mit Diethylether (3x3 ml) gewaschen. Das polarere Diastereoisomer von 1-(4-Dimethylamino-4-phenyl-cyclohexylmethyl)-3-[2-(1H-indol-3-yl)-1-methyl-ethyl]-harnstoff wurde so in einer Ausbeute von 231 mg (76 %) als farbloser Feststoff mit einem Schmelzpunkt von 140-146 °C erhalten.

**Beispiele 25 und 26:**

**1-(4-Dimethylamino-4-phenylcyclohexylmethyl)-3-[2-(1 *H*-indol-3-yl)-1-methylethyl]harnstoff-hydrochlorid**

Das unpolarere Diastereoisomer von 1-(4-Dimethylamino-4-phenyl-cyclohexylmethyl)-3-[2-(1 H-indol-3-yl)-1-methyl-ethyl]-harnstoff (157 mg, 0,36 mmol) wurde in Ethylmethylketon (6 ml) gelöst. Unter Rühren wurde bei RT Chlortrimethylsilan (69 μl, 0,6 mmol) tropfenweise zugegeben. Bei einstündigem Rühren bei RT fiel nur wenig Niederschlag aus. Die Reaktionslösung wurde mit Diethylether (25 ml) versetzt. Danach wurde bei RT 1 h kräftig gerührt. Es bildete sich ein farbloser Niederschlag. Der Feststoff wurde abfiltriert, mit Diethylether (3x2 ml) gewaschen und im Vakuum getrocknet. Das Hydrochlorid des unpolareren Diastereoisomers von 1-(4-Dimethylamino-4-phenyl-cyclohexylmethyl)-3-[2-(1H-indol-3-yl)-1-methyl-ethyl]-harnstoff (163 mg, Fp. 150-155 °C, 97 %; Beispiel 25) wurde so als farbloser Feststoff gewonnen.

Das polarere Diastereoisomer von 1-(4-Dimethylamino-4-phenyl-cyclohexylmethyl)-3-[2-(1H-indol-3-yl)-1-methyl-ethyl]-harnstoff (219 mg, 0,5 mmol) wurde in Ethylmethylketon (40 ml) gelöst. Unter Rühren wurde bei RT Chlortrimethylsilan (95 μl, 0,75 mmol) tropfenweise zugegeben. Nach einstündigem Rühren war kein Niederschlag ausgefallen. Die Reaktionslösung wurde im Vakuum auf 5 ml reduziert und mit Diethylether (15 ml) versetzt. Danach wurde bei RT 1 h kräftig gerührt. Es bildete sich ein fast farbloser Niederschlag. Der Feststoff wurde abfiltriert, mit Diethylether (3 × 3 ml) gewaschen und im Vakuum getrocknet. Das Hydrochlorid des polareren Diastereoisomers von 1-(4-Dimethylamino-4-phenyl-cyclohexylmethyl)-3-[2-(1H-indol-3-yl)-1-methyl-ethyl]-harnstoff (219 mg, Fp. 170-174 °C, 93 %; Beispiel 26) wurde so als farbloser Feststoff gewonnen.

**1-(4-Dimethylamino-4-phenylcyclohexylmethyl)-3-[2-(5 fluor-1*H*-indol-3-yl)ethyl]harnstoff**

Zu einer Lösung von 2-(5-Fluoro-1H-indol-3-yl)-ethylamine (297,5 mg, 1,67 mmol) in Dioxan (14 ml) wurde das Diastereoisomerengemisch von (4-Dimethylamino-4-phenyl-cyclohexylmethyl)-carbaminsäure-phenylester (588,6 mg, 1,67 mmol) gegeben. Die Reaktionsmischung wurde 24 h unter Rückfluss gekocht. Bei RT bildete sich ein farbloser Niederschlag. Der Niederschlag wurde abfiltriert, mit Dioxan (1 × 1 ml) und mit Diethylether (4 × 2 ml) gewaschen und dann getrocknet. Der erhaltene farblose Feststoff war das polarere Diastereoisomer von 1-(4-Dimethylamino-4-phenyl-cyclohexylmethyl)-3-[2-(5-fluor-1H-indol-3-yl)-ethyl]-harnstoff (220 mg, Fp. 97-101 °C, 30 %). Das Filtrat wurde eingeengt. Der Rückstand enthielt neben Phenol überwiegend das unpolarere Diastereoisomer von 1-(4-Dimethylamino-4-phenyl-cyclohexylmethyl)-3-[2-(5-fluor-1 H-indol-3-yl)-ethyl]-harnstoff. Durch Flash-Chromatographie an Kieselgel (60 g) wurde dieses Diastereoisomer gereinigt. Als Eluent wurde Methanol/EE (1:1; 800 ml) eingesetzt. Das unpolarere Diastereoisomer von 1-(4-Dimethylamino-4-phenyl-cyclohexylmethyl)-3-[2-(5-fluor-1 H-indol-3-yl)-ethyl]-harnstoff (187 mg, Fp. 70-73 °C) wurde so in einer Ausbeute von 26 % isoliert.

**Beispiele 27 und 28:**

**1-(4-Dimethylamino-4-phenylcyclohexylmethyl)-3-[2-(5-fluor-1** H-indol-3-**yl)ethyl]harnstoff-citrat**

Das unpolarere Diastereoisomer von 1-(4-Dimethylamino-4-phenyl-cyclohexylmethyl)-3-[2-(5-fluor-1 H-indol-3-yl)-ethyl]-harnstoff (187 mg, 0,428 mmol) wurde in abs. Ethanol (3,5 ml) gelöst. Unter Rühren wurde bei ca. 40 °C die Zitronensäure (83 mg, 0,43 mmol) in einer Portion zugegeben. Nach zweistündigem Rühren bei RT war kein Niederschlag ausgefallen. Das Reaktionsgemisch wurde mit Diethylether (20 ml) versetzt und nochmals 1 h bei RT gerührt. Nach kurzer Kühlung wurde der farblose Niederschlag abfiltriert, mit kaltem Diethylether (3 × 3 ml) gewaschen und im Vakuum getrocknet. Das unpolarere Diastereoisomer von 1-(4-Dimethylamino-4-phenyl-cyclohexylmethyl)-3-[2-(5-fluor-1 H-indol-3-yl)-ethyl]-harnstoff-citrat (214 mg, 80 %, Beispiel 27) war ein farbloser Feststoff.

Das polarere Diastereoisomer von 1-(4-Dimethylamino-4-phenyl-cyclohexylmethyl)-3-[2-(5-fluor-1 H-indol-3-yl)-ethyl]-harnstoff (208 mg, 0,476 mmol) wurde in abs. Ethanol (3,5 ml) gelöst. Unter Rühren wurde bei ca. 40 °C die Zitronensäure (92,5 mg, 0.481 mmol) in einer Portion zugegeben. Nach zweistündigem Rühren bei RT war kein Niederschlag ausgefallen. Die Reaktionsmischung wurde auf ca. 1 ml Lösung reduziert und portionsweise mit Diethylether (20 ml) versetzt. Der entstandene Niederschlag wurde nach 1 h abfiltriert, mit Ether (3 × 3 ml) gewaschen und im Vakuum getrocknet. Das polarere Diastereoisomer von 1-(4-Dimethylamino-4-phenyl-cyclohexylmethyl)-3-[2-(5-fluor-1 H-indol-3-yl)-ethyl]-harnstoff-citrat (230 mg, 77 %, Beispiel 28) war ein farbloser Feststoff.

Beispiele 29 und 30:

**1-(4-Dimethylamino-4-phenylcyclohexyl)-3-[2-(1 *H*-indol-3-yl)ethyl]thioharnstoff-citrat**

Zur Herstellung des Citrates wurde das unpolarere Diastereoisomer von 1-(4-Dimethylamino-4-phenyl-cyclohexyl)-3-[2-(1 H-indol-3-yl)-ethyl]-thioharnstoff (433 mg, 1,03 mmol) in heißem Ethanol (15 ml) gelöst und mit einer ebenfalls heißen Lösung von Citronensäure (435 mg, 2,27 mmol) in Ethanol (2 ml) versetzt. Nach dem Abkühlen auf ca. 5 °C wurde der Ansatz 4 h stehen gelassen. Der entstandene Feststoff wurde abfiltriert. Das unpolarere Diastereoisomer von 1-(4-Dimethylamino-4-phenyl-cyclohexyl)-3-[2-(1 H-indol-3-yl)-ethyl]-thioharnstoff konnte so in einer Ausbeute von 630 mg (100 %) als farbloser Feststoff (Beispiel 29, Smp. 165-170 °C) erhalten werden.

Zur Herstellung des Citrates wurde das polarere Diastereoisomer von 1-(4-Dimethylamino-4-phenyl-cyclohexyl)-3-[2-(1 H-indol-3-yl)-ethyl]-thioharnstoff (185 mg, 0,44 mmol) in heißem Ethanol (7 ml) gelöst und mit einer ebenfalls heißen Lösung von Citronensäure (186 mg, 0,97 mmol) in Ethanol (1 ml) versetzt. Nach dem Abkühlen auf ca. 5 °C wurde der Ansatz 4 h stehen gelassen. Der entstandene Feststoff wurde abfiltriert. Das polarere Diastereoisomer von 1-(4-Dimethylamino-4-phenyl-cyclohexyl)-3-[2-(1 H-indol-3-yl)-ethyl]-thioharnstoff konnte so in einer Ausbeute von 269 mg (100 %) als farbloser Feststoff (Beispiel 30, Smp. 98-103 °C) erhalten werden.

**1-(4-Dimethylamino-4-phenylcyclohexyl)-3-(4-phenylpropyl)thioharnstoff**

Phenylpropylamin (285 μl, 2 mmol) wurde in trockenem Chloroform (15 ml) gelöst und mit Triethylamin (555 μl, 4 mmol) versetzt. Zu diesem Gemisch wurde Thiophosgen (153 μl, 2 mmol) hinzugefügt. Nach einer Reaktionszeit von 16 h wurde das Diastereoisomerengemisch von *N,N*-Dimethyl-1-phenyl-cyclohexan-1,4-diamin zugesetzt und weitere 16 h bei RT gerührt. Zur Aufarbeitung wurde der Ansatz mit gesättigter $NaHCO_3$-Lösung (3 × 20 ml) extrahiert. Die organische Phase wurde mit $Na_2SO_4$ getrocknet und eingedampft. Das Produkt war ein Gemisch aus zwei Diastereoisomeren und konnte durch Säulenchromatographie [Kieselgel 60 (50 g); Methanol (500 ml)] gereinigt werden. Das polarere Diastereoisomer von 1-(4-Dimethylamino-4-phenyl-cyclohexyl)-3-(3-phenyl-propyl)-thioharnstoff wurde in einer Ausbeute von 205 mg (25 %) als farbloser Schaum erhalten. Das unpolarere Diastereoisomer von 1-(4-Dimethylamino-4-phenyl-cyclohexyl)-3-(3-phenyl-propyl)-thioharnstoff wurde in einer Ausbeute von 410 mg (50 %) als farbloser Schaum erhalten.

**Beispiele 31 und 32:**

**1-(4-Dimethylamino-4-phenylcyclohexyl)-3-(4-phenyl-propyl)thioharnstoff-citrat**

Zur Herstellung des Citrates wurde das unpolarere Diastereoisomer von 1-(4-Dimethylamino-4-phenyl-cyclohexyl)-3-(3-phenyl-propyl)-thioharnstoff (410 mg, 1 mmol) in heißem Ethanol (15 ml) gelöst und mit einer ebenfalls heißen Lösung von Citronensäure (422 mg, 2,2 mmol) in Ethanol (2 ml) versetzt. Nach dem Abkühlen auf ca. 5 °C wurde der Ansatz 4 h stehen gelassen. Der entstandene Feststoff wurde abfiltriert. Das unpolarere Diastereoisomer von 1-(4-Dimethylamino-4-phenyl-cyclohexyl)-3-(3-phenyl-propyl)-thiohamstoff-citrat wurde so in einer Ausbeute von 588 mg (100 %) als farbloser Feststoff **(Beispiel 31,** Schmelzpunkt: 63-67 °C) erhalten.

Zur Herstellung des Citrates wurde das polarere Diastereoisomer von 1-(4-Dimethylamino-4-phenyl-cyclohexyl)-3-(3-phenyl-propyl)-thioharnstoff (205 mg, 0,5 mmol) in heißem Ethanol (7 ml) gelöst und mit einer ebenfalls heißen Lösung von Citronensäure (211 mg, 1,1 mmol) in Ethanol (1 ml) versetzt. Nach dem Abkühlen auf ca. 5 °C wurde der Ansatz 4 h stehen gelassen. Der entstandene Feststoff wurde abfiltriert. Das polarere Diastereoisomer von 1-(4-Dimethylamino-4-phenyl-cyclohexyl)-3-(3-phenyl-propyl)-thioharnstoff wurde so in einer Ausbeute von 294 mg (100 %) als farbloser Feststoff (Beispiel 32, Schmelzpunkt: 75-80 °C) erhalten.

**1-(4-Dimethylamino-4-phenylcyclohexyl)-3-[2-(1*H*-indol-3-yl)methylethyl]thioharnstoff**

2-(1 H-Indol-3-yl)-1-methyl-ethylamin (349 mg, 2 mmol) wurde in trockenem Chloroform (10 ml) gelöst und mit Triethylamin (555 µl, 4 mmol) versetzt. Zu diesem Gemisch wurde Thiophosgen (153 µl, 2 mmol) hinzugefügt. Nach einer Reaktionszeit von 16 h wurde das Diastereoisomerengemisch von *N,N*-Dimethyl-1-phenyl-cyclohexan-1,4-diamin zugesetzt und weitere 16 h bei RT gerührt. Zur Aufarbeitung wurde der Ansatz mit gesättigter $NaHCO_3$-Lösung (3 × 20 ml) extrahiert. Die organische Phase wurde mit $Na_2SO_4$ getrocknet und eingedampft. Das Produkt war ein Gemisch aus zwei Diastereoisomeren und konnte durch Säulenchromatographie [Kieselgel 60 (50 g); Methanol (500 ml)] gereinigt werden. Das polarere Diastereoisomer von 1-(4-Dimethylamino-4-phenyl-cyclohexyl)-3-[2-(1 H-indol-3-yl)-1-methyl-ethyl]-thioharnstoff wurde in einer Ausbeute von 256 mg (29 %) als farbloser Feststoff erhalten. Das unpolarere Diastereoisomer von 1-(4-Dimethylamino-4-phenyl-cyclohexyl)-3-[2-(1 H-indol-3-yl)-1-methyl-ethyl]-thioharnstoff wurde in einer Ausbeute von 324 mg (37 %) als farbloser Feststoff erhalten.

**Beispiel 33 und 34:**

**1-(4-DIMETHYLAMINO-4-PHENYLCYCLOHEXYL)-3-[2-(1** H-INDOL-3-YL)METHYL**ETHYL]THIOHARNSTOFF-CITRAT**

Zur Herstellung des Citrates wurde der unpolarere Diastereoisomer von 1-(4-Dimethylamino-4-phenyl-cyclohexyl)-3-[2-(1H-indol-3-yl)-1-methyl-ethyl]-thioharnstoff (324 mg, 0,75 mmol) in heißem Ethanol (10 ml) gelöst und mit einer ebenfalls heißen Lösung von Citronensäure (316 mg, 1,65 mmol) in Ethanol (1,5 ml) versetzt. Nach dem Abkühlen auf ca. 5°C wurde der Ansatz 4 h stehen gelassen. Der entstandene Feststoff wurde abfiltriert. Das unpolarere Diastereoisomer von 1-(4-Dimethylamino-4-phenyl-cyclohexyl)-3-[2-(1 H-indol-3-yl)-1-methyl-ethyl]-thioharnstoff-citrat konnte so in einer Ausbeute von 467 mg (100 %) als farbloser Feststoff (Beispiel 33, Schmelzpunkt: 145-148 °C) erhalten werden.

Zur Herstellung des Citrates wurde das polarere Diastereoisomer von 1-(4-Dimethylamino-4-phenyl-cyclohexyl)-3-[2-(1 H-indol-3-yl)-1-methyl-ethyl]-thioharnstoff (256 mg, 0,59 mmol) in heißem Ethanol (8 ml) gelöst und mit einer ebenfalls heißen Lösung von Citronensäure (249 mg, 1,3 mmol) in Ethanol (1 ml) versetzt. Nach dem Abkühlen auf ca. 5 °C wurde der Ansatz 4 h stehen gelassen. Der entstandene Feststoff wurde abfiltriert. Das polarere Diastereoisomer von 1-(4-Dimethylamino-4-phenyl-cyclohexyl)-3-[2-(1 H-indol-3-yl)-1-methyl-ethyl]-thioharnstoff-citrat konnte so in einer Ausbeute von 369 mg (100 %) als farbloser Feststoff (Beispiel 34, Schmelzpunkt: 108-113 °C) erhalten werden.

**3-(4-DIMETHYLAMINO-4-PHENYL-CYCLOHEXYL)-ACRYLONITRIL**

Das Diastereoisomeren-Gemisch von 4-Dimethylamino-4-phenyl-cyclohexyl-carbaldehyd (518 mg, 2,24 mmol) und Cyanmethanphosphonsäure-diethylester (476,7 g, 2,69 mmol) wurden in DCM (10 ml) gelöst. Diese Lösung wurde unter Kühlung mit Eiswasser bei einer Temperatur von 5 bis 10 °C in eine Lösung von 40-proz. wässriger NaOH (5 ml) getropft. Zur Aufarbeitung wurde der Ansatz nach 2 h mit Eis (20 g) und DCM (10 ml) versetzt. Die Phasen wurden getrennt. Die wässrige Phase wurde mit DCM extrahiert (2 × 5 ml). Die vereinigten Extrakte wurden mit gesättigter NaCl-Lösung (4 × 5 ml) gewaschen, getrocknet und eingedampft. Der Rückstand wurde durch Chromatographie [Kieselgel (70 g) Eluent: EE/MeOH 500 ml (10 : 1) und 700 ml (4 : 1)] gereinigt. Die vier Diastereoisomeren von 3-(4-Dimethylamino-4-phenyl-cyclohexyl)-acrylonitril wurden so in einer Gesamtausbeute von 71 % (406 mg) erhalten.

**[4-(3-AMINOPROPYL)-1 -PHENYLCYCLOHEXYL]DIMETHYLAMIN**

3-(4-Dimethylamino-4-phenyl-cyclohexyl)-acrylonitril (400 mg, 1,57 mmol) wurde in Methanol (25 ml) gelöst. Zu der Lösung wurde Nickei(II)chlorid-hexahydrat (747,6 mg, 3,14 mmol) gegeben. Anschließend wurde unter Kühlung mit Eiswasser $NaBH_4$ (594 mg, 15,7 mmol) in Portionen zugegeben. Unter Entwicklung von Wasserstoff bildete sich ein schwarzer Niederschlag. Nach beendeter Zugabe wurde 1 h bei 20 °C weitergerührt. Zur Aufarbeitung wurde das Reaktionsgemisch mit 1 M Salzsäure (15 ml) versetzt. Der schwarze Niederschlag wurde über Celite abfiltriert und gründlich mit 1 M Salzsäure (3 × 7 ml) gewaschen. Die salzsaure wässrig-methanolische Lösung wurde auf das halbe Volumen reduziert, um das Methanol zu entfernen. Die zurückbleibende wässrige Phase wurde zur Entfernung von Neutralstoffen mit Ether (3 × 15 ml) extrahiert. Danach wurde durch Zugabe von 2M NaOH alkalisch gemacht (pH 11). Dabei fiel ein voluminöser Niederschlag aus, der sich auch durch Zugabe von EE nicht auflöste. Der Niederschlag wurde abfiltriert und mit Wasser (1 × 3 ml) und EE (3 × 5 ml) gewaschen. Die zwei Phasen der klaren Mutterlauge wurden getrennt. Die wässrige Phase wurde mit EE (3 × 15 ml) extrahiert. Die vereinigten Extrakte wurden mit NaCl-Lösung (2x10 ml) gewaschen, über Natriumsulfat getrocknet und das Lösungsmittel abdestilliert. Der erhaltene Rückstand war das Gemisch der Diastereoisomeren von [4-(3-Aminopropyl)-1-phenylcyclohexyl]dimethylamin (203 mg, hellgelbes Öl, 50 %)

**[2-(*1H*-Indol-3-yl)ethyl]carbaminsäure-phenylester**

Zu einer Lösung von Tryptamin (3,2 g, 20,0 mmol) in abs. DCM (50 ml) wurden Chlorameisensäure-phenylester (3,29 g, 21,0 mmol) und Pyridin (1,74 g, 22,0 mmol) gegeben. Anschließend wurde 24 h bei RT gerührt. Zur Aufarbeitung wurde der Ansatz mit Wasser (2 × 20 ml), mit 1 M HCl (2 × 20 ml) und 1 M NaOH (2 × 20 ml) extrahiert. Die organische Phase wurde mit $Na_2SO_4$ getrocknet und anschließend eingedampft. [2-(1*H*-Indol-3-yl)ethyl]carbaminsäure-phenylester wurde in einer Ausbeute von 5,58 g (100 %) als farbloser Feststoff mit einem Smp. von 44-46 °C erhalten.

**Beispiele 35 und 36:**

**1-[3-(4-Dimethylamino-4-phenyl-cyclohexyl)-propyl]-3-[2-(1H-indol-3-yl)-ethyl]-harnstoff**

Zu einer Lösung von [2-(1*H*-Indol-3-yl)ethyl]carbaminsäure-phenylester (409 mg, 1,46 mmol) in Dioxan (10 ml) wurde das Gemisch von [4-(3-Aminopropyl)-1-phenyl-cyclohexyl]-dimethylamin (380 mg, 1,46 mmol) gegeben. Anschließend wurde 12 h unter Rückfluss gekocht. Zur Aufarbeitung wurde Dioxan abdestilliert und der Rückstand mit Wasser (10 ml) versetzt. Der Ansatz wurde mit 5M NaOH auf pH 11 eingestellt und mit EE (3 × 20 ml) extrahiert. Die kombinierten EE-Extrakte wurden mit 1 M NaOH (5 ml) gewaschen und mit $Na_2SO_4$ getrocknet. Das Lösungsmittel wurde im Vakuum abdestilliert. Der Rückstand war das Diastereoisomeren-Gemisch von 1-[3-(4-Dimethylamino-4-phenyl-cyclohexyl)-propyl]-3-[2-(1 H-indol-3-yl)-ethyl]-hamstoff, das durch Flash-Chromatographie an Kieselgel (70 g) getrennt und gereinigt wurde. Als Eluent wurde Methanol/EE (3 : 1, 1500 ml; 1 : 1, 500 ml; und 1 : 2, 1000 ml) eingesetzt. Das unpolarere Diastereoisomer von 1-[3-(4-Dimethylamino-4-phenyl-cyclohexyl)-propyl]-3-[2-(1H-indol-3-yl)-ethyl]-harnstoff (Beispiel 35, 95 mg, Fp. 126 °C, 15 %) und das polarere Diastereoisomer von 1-[3-(4-Dimethylamino-4-phenyl-cyclohexyl)-propyl]-3-[2-(1 H-indol-3-yl)-ethyl]-harnstoff (Beispiel 36, 116 mg, Fp. 57 °C, 18 %) wurden so isoliert.

**1-(4-DIMETHYLAMINO-4-PHENYLCYCLOHEXYLMETHYL)-3-[2-(1 *H*-INDOL-3-YL)ETHYL]THIOHARNSTOFF**

Tryptamin (320,4 mg, 2 mmol) wurde in trockenem Chloroform (10 ml) gelöst und mit Triethylamin (533 µl, 4 mmol) versetzt. Die Mischung wurde auf -5 °C gekühlt. Dann wurde Thiophosgen (153,3 µl, 2 mmol) tropfenweise hinzugefügt. Dabei fiel ein Niederschlag aus. Nach einer Reaktionszeit von 18 h bei RT wurde (4-Aminomethyl-1-phenylcyclohexyl)-dimethyl-amin (464,74 mg, 2 mmol) gelöst in Chloroform (5 ml) zugesetzt. Nach 20 h Rühren bei RT war die Reaktionsmischung klar. Zur Aufarbeitung wurde der Ansatz mit gesättigter $NaHCO_3$-Lösung (3 × 5 ml) und Wasser (5 ml) gewaschen. Die organische Phase wurde mit $Na_2SO_4$ getrocknet und eingedampft. Das Produkt war ein Gemisch aus zwei Diastereoisomeren. Diese wurden durch Säulenchromatographie [Kieselgel 60 (70 g); Eluent: MeOH/EE 1 : 1 (1500 ml)] gereinigt und getrennt. Das unpolarere Diastereoisomer von 1-(4-Dimethylamino-4-phenylcyclohexylmethyl)-3-[2-(1*H*-indol-3-yl)ethyl]thioharnstoff (266 mg, Fp. 84-87 °C, 31 %) und auch das polarere Diastereoisomer von 1-(4-Dimethylamino-4-phenylcyclohexylmethyl)-3-[2-(1*H*-indol-3-yl)ethyl]thiohamstoff (274 mg, Fp. 92-95 °C, 32 %) waren hellbeige Feststoffe.

**Beispiele 37 und 38:**

**1-(4-Dimethylamino-4-phenylcyclohexylmethyl)-3-[2-(1 *H*-indol-3-yl)ethyl]-thioharnstoff-citrat**

Zur Herstellung des Citrates wurde das unpolarere Diastereoisomer von 1-(4-Dimethylamino-4-phenylcyclohexylmethyl)-3-[2-(1 *H*-indol-3-yl)ethyl]thioharnstoff (253 mg, 0,582 mmol) in Ethanol (4 ml) bei RT gelöst und mit einer Lösung von Citronensäure (123 mg, 0,588 mmol) in Ethanol (1 ml) versetzt. Nach 2 h wurde der Ansatz mit 20 ml Ether versetzt und 18 h gerührt. Der entstandene Feststoff wurde abfiltriert, mit Ether (3 × 2 ml) gewaschen und getrocknet. Das unpolare Diastereoisomer von 1-(4-Dimethylamino-4-phenylcyclohexylmethyl)-3-[2-(1 H-indol-3-yl)ethyl]-thioharnstoff-citrat konnte so in einer Ausbeute von 297 mg (Beispiel 37, 81 %) als hellgelber Feststoff erhalten werden.

Zur Herstellung des Citrates wurde das polarere Diastereoisomer von 1-(4-Dimethylamino-4-phenylcyclohexylmethyl)-3-[2-(1 *H*-indol-3-yl)ethyl]thioharnstoff (262 mg, 0,603 mmol) in Ethanol (5 ml) gelöst und mit einer Lösung von Citronensäure (117 mg, 0,608 mmol) in Ethanol (1 ml) versetzt. Es fiel sofort ein voluminöser Niederschlag aus, dessen Struktur sich durch die Zugabe von Ether (20 ml) verbesserte. Nach 20 h Rühren bei RT wurde der entstandene Feststoff abfiltriert. Das polarere Diastereoisomer von 1-(4-Dimethylamino-4-phenylcyclohexylmethyl)-3-[2-(1*H*-indol-3-yl)ethyl]thioharnstoff-citrat (Beispiel 38, 317 mg, 84 %) war ein beiger und hygroskopischer Feststoff.

**1-(4-DIMETHYLAMINO-4-PHENYLCYCLOHEXYLMETHYL)-3-[2-(1*H*-INDOL-3-YL)-1 - METHYLETHYL]THIOHARNSTOFF**

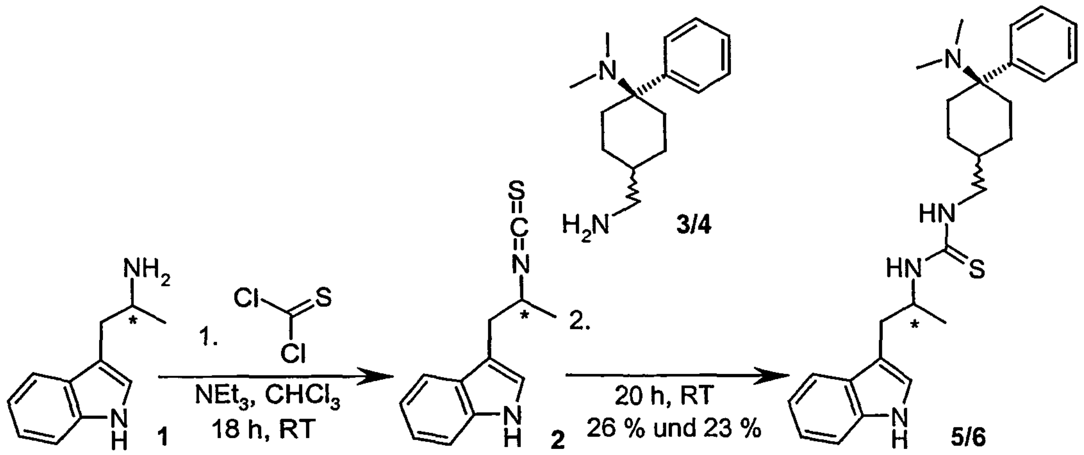


α-Methyltryptamin (261,4 mg, 1,5 mmol) wurde in trockenem Chloroform (15 ml) gelöst und mit Triethylamin (422 µl, 3 mmol) versetzt. Die Mischung wurde auf -5 °C gekühlt. Dann wurde Thiophosgen (115 µl, 1,5 mmol) gelöst in Chloroform (10 ml) zugetropft. Dabei fiel ein Niederschlag aus. Nach einer Reaktionszeit von 18 h bei RT wurde (4-Aminomethyl-1-phenyl-cyclohexyl)-dimethyl-amin (348,55 mg, 1,5 mmol) gelöst in Chloroform (10 ml) zugesetzt. Nach 20 h Rühren bei RT war die Reaktionsmischung klar. Zur Aufarbeitung wurde der Ansatz mit gesättigter $NaHCO_3$-Lösung (3 × 5 ml) und Wasser (5 ml) gewaschen. Die organische Phase wurde mit $Na_2SO_4$ getrocknet und eingedampft. Das Produkt war ein Gemisch aus zwei Diastereoisomeren. Diese wurden durch Säulenchromatographie [Kieselgel 60 (70 g); Eluent: MeOH/EE 1 : 5 (1500 ml)] gereinigt und getrennt. Das unpolarere Diastereoisomer von 1-(4-Dimethylamino-4-phenylcyclohexylmethyl)-3-[2-(1*H*-indol-3-yl)-1-methylethyl]thioharnstoff (177 mg, Fp. 99-104 °C, 26 %) und auch das polarere Diastereoisomer von 1-(4-Dimethylamino-4-phenylcyclohexylmethyl)-3-[2-(1H-indol-3-yl)-1-methylethyl]thioharnstoff (152 mg, Fp. 93-95 °C, 23 %) waren farblose Feststoffe.

**Beispiele 39 und 40:**

**1-(4-Dimethylamino-4-phenylcyclohexylmethyl)-3-[2-(1H-Indol-3-yl)-1 - methylethyl]thioharnstoff -citrat**

Zur Herstellung des Citrates wurde das unpolarere Diastereoisomer von 1-(4-Dimethylamino-4-phenylcyclohexylmethyl)-3-[2-(1*H*-indol-3-yl)-1-methylethyl]-thioharnstoff (167 mg, 0,37 mmol) in Ethanol (4 ml) bei RT gelöst und

mit einer Lösung von Citronensäure (78,7 mg, 0,41 mmol) in Ethanol (1 ml) versetzt. Nach 1 h war ein klebriger Niederschlag an der Kolbenwand sichtbar, der mechanisch abgelöst wurde. Dann wurde der Ansatz mit Ether (35 ml) versetzt und 20 h gerührt. Der entstandene Feststoff wurde abfiltriert, mit Ether (3 × 3 ml) gewaschen und getrocknet. Das unpolare Diastereoisomer von 1-(4-Dimethylamino-4-phenyl-cyclohexylmethyl)-3-[2-(1*H*-indol-3-yl)-1-methylethyl]thioharnstoff-citrat wurde so in einer Ausbeute von 206 mg (87 %) als farbloser Feststoff (Beispiel 39) erhalten.

Zur Herstellung des Citrates wurde das polarere Diastereoisomer von 1-(4-Dimethylamino-4-phenylcyclohexylmethyl)-3-[2-(1*H*-indol-3-yl)-1-methylethyl]-thioharnstoff (140 mg, 0,31 mmol) in Ethanol (4 ml) gelöst und mit einer Lösung von Citronensäure (65,9 mg, 0,34 mmol) in Ethanol (1 ml) versetzt. Es fiel nur wenig Niederschlag aus. Das Gemisch wurde mit deshalb Ether (50 ml) versetzt. Nach 20 h Rühren bei RT wurde der entstandene Feststoff abfiltriert. Das polarere Diastereoisomer von 1-(4-Dimethylamino-4-phenylcyclohexylmethyl)-3-[2-(1*H*-indol-3-yl)-1-methylethyl] thioharnstoff-citrat wurde so in einer Ausbeute von 185,5 mg (93 %) als farbloser Feststoff (Beispiel 40) erhalten.

**2-[3-(4-Dimethylamino-4-phenylcyclohexylmethyl)thioureido]-3-(1 *H*-indol-3-yl)propionsäure-methylester**

Tryptophan-methylester-Hydrochlorid (382 mg, 1,5 mmol) wurde in trockenem Chloroform (15 ml) gelöst und mit Triethylamin (633 µl, 4,5 mmol) versetzt. Die Mischung wurde auf-5 °C gekühlt. Dann wurde Thiophosgen (115 µl, 1,5 mmol) gelöst in Chloroform (10 ml) zugetropft. Dabei fiel ein Niederschlag aus. Nach 18 h Rühren bei RT war die Reaktionsmischung klar. Bei RT wurde (4-Aminomethyl-1-phenyl-cyclohexyl)-dimethyl-amin (348,55 mg, 1,5 mmol), gelöst in Chloroform (10 ml), zugesetzt. Nach einer Reaktionszeit von 48 h wurde der Ansatz mit gesättigter $NaHCO_3$-Lösung (3 × 5 ml) und Wasser (5 ml) gewaschen. Die organische Phase wurde mit $Na_2SO_4$ getrocknet und eingedampft. Das Produkt war ein Gemisch aus zwei Diastereoisomeren. Diese wurden durch Säulenchromatographie [Kieselgel 60 (100 g); Eluent: MeOH/EE 1 : 4 (1500 ml) und MeOH/EE 1 : 1 (500 ml)] gereinigt und getrennt. Das unpolarere Diastereoisomer von 2-[3-(4-Dimethylamino-4-phenylcyclohexylmethyl)thioureido]-3-(1 *H*-indol-3-yl)propionsäure-methylester (193 mg, Fp. 178-183 °C, 26 %) und auch das polarere Diastereoisomer von 2-[3-(4-Dimethylamino-4-phenylcyclohexylmethyl)thioureido]-3-(1 *H*-indol-3-yl)propionsäure-methylester (157 mg, Fp. 228-234 °C, 21 %) waren hellgelbe Feststoffe.

**Beispiele 41 und 42:**

**2-[3-(4-Dimethylamino-4-phenylcyclohexylmethyl)thioureido]-3-(1 *H*-indol-3-yl)propionsäure-methylester-citrat**

Zur Herstellung des Citrates wurde das unpolarere Diastereoisomer von 2-[3-(4-Dimethylamino-4-phenylcyclohexylmethyl)thioureido]-3-(1 *H*-indol-3-yl)propionsäure-methylester (135 mg, 0,274 mmol) in heißem Ethanol (11 ml) gelöst und mit einer Lösung von Citronensäure (57,9 mg, 0,3 mmol) in Ethanol (1 ml) versetzt. Nach 4 h war kein Niederschlag sichtbar. Ethanol wurde bis auf 2 ml abdestilliert und langsam mit Ether (30 ml) versetzt. Die Suspension wurde 20 h bei RT gerührt und 2 h im Kühlschrank gekühlt. Der entstandene Feststoff wurde abfiltriert, mit kaltem Ether (2 × 1 ml) gewaschen und getrocknet. Das unpolare Diastereoisomer von 2-[3-(4-Dimethylamino-4-phenylcyclohexylmethyl)thioureido]-3-(1 *H*-indol-3-yl)propionsäure-methylester-citrat konnte so in einer Ausbeute von 165 mg (88 %) als gelber Feststoff **(Beispiel 41,** Fp. 158-163°C) erhalten werden.

Zur Herstellung des Citrates wurde das polarere Diastereoisomer von 2-[3-(4-Dimethylamino-4-phenylcyclohexylmethyl)thioureido]-3-(1 *H*-indol-3-yl)propionsäure-methylester (156 mg, 0,317 mmol) in heißem Ethanol (50 ml) suspendiert und mit einer Lösung von Citronensäure (66,9 mg, 0,35 mmol) in Ethanol (1 ml) versetzt. Bei Zugabe der Citronensäure löste sich der polarere Harnstoff sofort auf. Bei RT fiel kein Niederschlag aus. Ethanol wurde bis auf 3 ml abdestilliert und langsam mit Ether (30 ml) versetzt. Die Suspension wurde 20 h bei RT gerührt und 2 h im Kühlschrank gekühlt. Der entstandene Feststoff wurde abfiltriert, mit kaltem Ether (2 × 1 ml) gewaschen und getrocknet. Das polarere Diastereoisomer von 2-[3-(4-Dimethylamino-4-phenylcyclohexylmethyl)thioureido]-3-(1 *H*-indol-3-yl)propionsäure-methylester-citrat wurde so in einer Ausbeute von 207 mg (95 %) als hellgelber Feststoff (Beispiel 42, Fp. 167-172 °C) erhalten.

**(4-Dimethylamino-4-phenylcyclohexyliden)acetonitril**

4-Dimethylamino-4-phenyl-cyclohexanon (10,85 g, 50 mmol) und Cyanmethanphosphonsäure-diethylester (10,65 g, 12 mmol) wurden in DCM (100 ml) gelöst. Diese Lösung wurde unter Kühlung mit Eiswasser bei einer Temperatur von 5 bis 10 °C unter kräftigem Rühren in eine Lösung von 40-proz. wässriger NaOH (50 ml) getropft und 2 h bei RT gerührt. Zur Aufarbeitung wurde der Ansatz mit Eis (100 g) versetzt. Die organische Phase wurde abgetrennt. Die wässrige Phase wurde mit DCM extrahiert (3 × 40 ml). Die vereinigten Extrakte wurden mit gesättigter NaCl-Lösung (50 ml) gewaschen, über $Na_2SO_4$ getrocknet und anschließend eingedampft. (4-Dimethylamino-4-phenylcyclohexyliden)acetonitril wurde als gelbes Öl in einer Ausbeute von 95 % (11,4 g) erhalten.

**[4-(2-Aminoethyl)-1-phenylcyclohexyl]dimethylamin**

(4-Dimethylamino-4-phenyicyclohexyliden)acetonitril (1,16 g, 4,8 mmol) wurde in Methanol (30 ml) gelöst. Zu dieser Lösung wurde Nickel(II)chlorid-hexahydrat (2,28 g, 9,6 mmol) gegeben. Anschließend wurde unter Rühren und unter Kühlung mit Eiswasser $NaBH_4$ (1,82 g, 48 mmol) in Portionen zugegeben. Nach beendeter Zugabe wurde 1 h bei RT weitergerührt. Das entstandene schwarze Nickelborid wurde nach Zugabe von 2M Salzsäure (15 ml) abgesaugt und mit 2M Salzsäure (3 × 10 ml) gewaschen. Die salzsaure wässrig-methanolische Lösung wurde auf das halbe Volumen reduziert, um das Methanol zu entfernen. Die zurückgebliebene wässrige Phase wurde zur Entfernung von Neutralstoffen mit Ether (3x10 ml) extrahiert. Danach wurde die wässrige Phase durch Zugabe von konz. wässriger Anmmoniaklösung alkalisch gemacht und mit Ether (3 × 20 ml) erneut extrahiert. Die vereinigten Extrakte wurden mit gesättigter NaCl-Lösung (30 ml) gewaschen, über $Na_2SO_4$ getrocknet und anschließend eingedampft. Das so erhaltene Rohprodukt konnte durch Säulenchromatographie [Kieselgel 60 (100 g); MeOH (1000 ml)] gereinigt werden. Dabei wurden die entstandenen Diastereoisomeren aber nicht getrennt. [4-(2-Aminoethyl)-1-phenylcyclohexyl]dimethylamin wurde in einer Ausbeute von 643 mg (54 %) als farbloses Öl erhalten.

**1-[2-(4-Dimethylamino-4-phenylcyclohexyl)ethyl]-3-[2-(1 H-indol-3-yl)-ethyl]thioharnstoff**

Tryptamin (320 mg, 2 mmol) wurde in trockenem Chloroform (10 ml) gelöst und mit Triethylamin (555 µl, 4 mmol) versetzt. Zu diesem Gemisch wurde Thiophosgen (153 µl, 2 mmol) hinzugefügt. Nach einer Reaktionszeit von 16 h wurde [4-(2-Aminoethyl)-1-phenylcyclohexyl]dimethylamin (491 mg, 2 mmol) zugesetzt und weitere 16 h bei RT gerührt. Zur Aufarbeitung wurde der Ansatz mit gesättigter $NaHCO_3$-Lösung (3 × 20 ml) extrahiert. Die organische Phase wurde mit $Na_2SO_4$ getrocknet und eingedampft. Das Produkt war ein Gemisch der erwarteten Diastereoisomeren und konnte durch Säulenchromatographie [Kieselgel 60 (100 g); MeOH (1000 ml)] gereinigt werden. Das polarere Diastereoisomer von 1-[2-(4-Dimethylamino-4-phenylcyclohexyl)ethyl]-3-[2-(1 *H*-indol-3-yl)ethyl]thioharnstoff wurde in einer Ausbeute von 330 mg (36 %) als farbloses Öl erhalten. Das unpolarere Diastereoisomer von 1-[2-(4-Dimethylamino-4-phenylcyclohexyl)ethyl]-3-[2-(1*H*-indol-3-yl)ethyl]thioharnstoff wurde in einer Ausbeute von 230 mg (25 %) als farbloses Öl erhalten.

**Beispiel 43:**

**1-[2-(4-DIMETHYLAMINO-4-PHENYLCYCLOHEXYL)ETHYL]-3-[2-(1*H*-INDOL-3-YL)-ETHYL]THIOHARNSTOFF-HYDROCHLORID**

Zur Herstellung des Hydrochlorids wurde das unpolarere Diastereoisomer von 1-[2-(4-Dimethylamino-4-phenylcyclohexyl)ethyl]-3-[2-(1 *H*-indol-3-yl)ethyl]thioharnstoff (230 mg, 0,5 mmol) in Ethylmethylketon (5 ml) gelöst und mit Trimethylchlorsilan (2,5 ml, 26,8 mmol) versetzt. Der dabei ausgefallene farblose Feststoff wurde nach 2 h abgesaugt, mit Ethylmethylketon (3 × 5 ml) gewaschen und anschließend getrocknet. Das unpolarere Diastereoisomer von 1-[2-(4-Dimethylamino-4-phenylcyclohexyl)-ethyl]-3-[2-(1*H*-indol-3-yl)ethyl]thioharnstoff-hydrochlorid wurde so in einer Ausbeute von 240 mg (99 %) als farbloser Feststoff **(Beispiel 43,** Smp. 130-138 °C) erhalten.

**Beispiel 44:**

**1-[2-(4-DIMETHYLAMINO-4-PHENYLCYCLOHEXYL)ETHYL]-3-[2-(1*H*-INDOL-3-YL)-ETHYL]THIOHARNSTOFF-CITRAT**

Zur Herstellung des Citrates wurde das polarere Diastereoisomer von 1-[2-(4-Dimethylamino-4-phenylcyclohexyl)ethyl]-3-[2-(1 *H*-indol-3-yl)ethyl]thioharnstoff (330 mg, 0,71 mmol) in heißem Ethanol (4 ml) gelöst und mit einer ebenfalls heißen Lösung von Citronensäure (137 mg, 0,71 mmol) in Ethanol (1 ml) versetzt. Nach dem Abkühlen auf ca. 5 °C wurde der Ansatz 4 h stehen gelassen. Da dabei kein Feststoff ausfiel, wurde das Ethanol abdestilliert Das polarere Diastereoisomer von 1-[2-(4-Dimethylamino-4-phenylcyclohexyl)ethyl]-3-[2-( 1*H*-indol-3-yl)-ethyl]thioharnstoff-citrat wurde so in einer Ausbeute von 467 mg (100 %) als farbloser Schaum **(Beispiel 44,** Schmelzpunkt: 90-95 °C) erhalten.

**Messung der ORL1-Bindung**

Die Cyclohexan-Derivate der allgemeinen Formel I wurden in einem Rezeptorbindungsassay mit $^3$H-Nociceptin/

Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 μg Membranprotein je 200 μl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird in Tabelle 1 als $K_i$-Wert oder % Inhibition bei c=1 μM angegeben.

**Messung der Bindung an Opiatrezeptoren**

Analog zu Beispiel 21 wurde nach den dem Fachmann bekannten Verfahren zur Messung der Bindung an die μ-, δ- und κ-Opiatrezeptoren (μ-, δ- und κ-OR) die Bindung der Cyclohexan-Derivate der allgemeinen Formel I in einem Rezeptorbindungsassay untersucht. Die Affinität wird in Tabelle 1 als $K_i$-Wert oder % Inhibition bei c=1 μM angegeben.

**Analgesieprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

Die untersuchten erfindungsgemäßen Verbindungen zeigten eine analgetische Wirkung gemäß Tabelle 1.

Tabelle 1:

| **Beispiel Nr.** | **ORL1**<br>Ki [μM] oder % Hemmung [1 μM] | μ<br>Ki [μM] Oder % Hemmung [1 μM] | **Tail Flick**<br>(Maus, i.v.) $ED_{50}$ [mg/kg] oder %Hemmung (Dosis [mg/kg]) |
|---|---|---|---|
| 1 | 0,0028 | 0,0006 | 65 % (1) |
| 2 | 0,1 | 94 % | |
| 3 | 0,0011 | 0,0013 | |
| 4 | 0,051 | 99 % | |
| 5 | 0,0043 | 0,0018 | |
| 6 | 53 % | 0,065 | |
| 7 | 0,0027 | 0,0011 | |
| 8 | 64 % | 0,053 | |
| 9 | 0,0003 | 0,0009 | |
| 10 | 0,01 | 0,05 | |
| 11 | 0,0046 | 0,001 | |
| 12 | 46 % | 0,18 | |

(fortgesetzt)

| Beispiel Nr. | ORL1 Ki [µM] oder % Hemmung [1 µM] | µ Ki [µM] Oder % Hemmung [1 µM] | Tail Flick (Maus, i.v.) $ED_{50}$ [mg/kg] oder %Hemmung (Dosis [mg/kg]) |
|---|---|---|---|
| 13 | 0,031 | 0,0124 | 60 % (10) |
| 14 | 32 % | 50 % | |
| 15 | 0,013 | 0,0032 | 69% (10) |
| 16 | 43 % | 71 % | |
| 17 | 0,0015 | 0,0008 | |
| 18 | 0,036 | 0,024 | |
| 19 | 0,0013 | 0,0011 | |
| 20 | 0,034 | 0,0077 | |
| 21 | 0,037 | 0,011 | |
| 22 | 0,0079 | 0,001 | |
| 23 | 0,051 | 0,0093 | |
| 24 | 0,012 | 0,024 | |
| 25 | 0,018 | 0,0061 | |
| 26 | 0,0089 | 0,0033 | |
| 27 | 0,043 | 0,011 | |
| 28 | 0,0069 | 0,0009 | |
| 29 | 0,051 | 0,025 | |
| 30 | 0,008 | 0,0031 | |
| 31 | 0,0024 | 0,012 | |
| 32 | 0,088 | 0,159 | |
| 33 | 0,0039 | 0,0053 | |
| 34 | 0,036 | 0,19 | |

**Parenterale Lösung eines erfindungsgemäßen Cyclohexyl-Harnstoff-Derivats**

38 g eines der erfindungsgemäßen Cyclohexyl-Harnstoff-Derivate, hier Beispiel 9, wird in 1 1 Wasser für Injektionszwecke bei Raumtemperatur gelöst und anschließend durch Zugabe von wasserfreier Glukose für Injektionszwecke auf isotone Bedingungen eingestellt.

**Patentansprüche**

**1.** Cyclohexyl-Harnstoff-Derivate der allgemeinen Formel I,

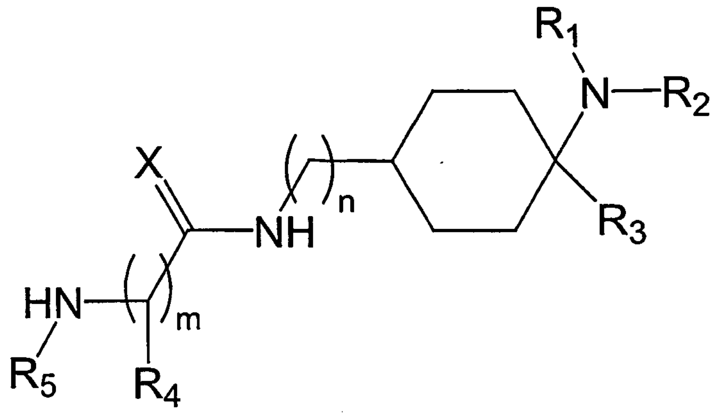

, worin
n=0-3,
m=0-2,
X = O oder S (bei m = 0),
$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert,
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl,
jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^4$ ausgewählt ist aus H, $C_{1-8}$-Alkyl, gesättigt oder ungesättigt, verzweigt oder unverzweigt, einfach oder mehrfach substituiert oder unsubstituiert; oder $-(CH_2)_o-W-(CH_2)_p-H$
mit W = O, $NR_7$ oder S, und o = 0-3 und p = 0-4 und
mit $R_7$ ausgewählt aus H, $C_{1-8}$-Alkyl, gesättigt oder ungesättigt, verzweigt oder unverzweigt, einfach oder mehrfach substituiert oder unsubstituiert;
$R^5$ , wenn $m \neq 0$, ausgewählt ist aus:

$-(CH_2)_qR^{12}$,
$-C(Y)-Z-R^{12}$ oder
$-C(Y)-O-Z-R^{12}$,
mit Y = O, $CH_2$ oder S,
mit Z = $C_{1-8}$-Alkyl, gesättigt oder ungesättigt, verzweigt oder unverzweigt, einfach oder mehrfach substituiert oder unsubstituiert;
oder $(CH_2)q$
mit q = 0-8
mit $R^{12}$ ausgewählt aus
H; $C_{3-8}$-Cycloalkyl, Aryl oder Heteroaryl, jeweils unsubstituiert oder einfach oder mehrfach substituiert,
oder
$R^5$ , wenn m = 0, ausgewählt ist aus $C_{3-8}$-Cycloalkyl, Aryl oder Heteroaryl, jeweils unsubstituiert oder einfach oder mehrfach substituiert; $-(CH_2)_qR^{12}$,
mit q = 0-8
mit $R^{12}$ ausgewählt aus
H; $C_{3-8}$-Cycloalkyl, Aryl oder Heteroaryl, jeweils unsubstituiert oder einfach oder mehrfach substituiert;

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**2.** Cyclohexyl-Harnstoff-Derivate gemäß Anspruch 1, **dadurch gekennzeichnet, dass**
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert;
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert,
oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus H, Methyl oder Ethyl bedeuten.

**3.** Cyclohexyl-Harnstoff-Derivate gemäß einem der Ansprüche 1 oder 2, **dadurch gekennzeichnet, dass**
$R^3$ ausgewählt ist aus $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenem Aryl, $C_{3-8}$-Cycloalkyl oder Heteroaryl, jeweils unsubstituiert oder einfach oder mehrfach substituiert;
vorzugsweise
$R^3$ ausgewählt ist aus $C_{5-6}$-Cycloalkyl, Phenyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus Phenyl, Furyl, Thiophenyl, Cyclohexanyl, Naphthyl, Benzofuranyl, Indolyl, Indanyl, Benzodioxanyl, Benzodioxolanyl, Pyrrolyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenem Phenyl, Furyl oder Thiophenyl, jeweils unsubstituiert oder einfach oder mehrfach substituiert.

**4.** Cyclohexyl-Harnstoff-Derivate gemäß einem der Ansprüche 1 bis 3, **dadurch gekennzeichnet, dass**
$R^4$ ausgewählt ist aus H oder $C_{1-8}$-Alkyl, gesättigt oder ungesättigt, verzweigt oder unverzweigt, einfach oder mehrfach substituiert oder unsubstituiert, vorzugsweise H, $CH_3$ oder $C_2H_5$.

**5.** Cyclohexyl-Harnstoff-Derivate gemäß Anspruch 1 bis 4, **dadurch gekennzeichnet, dass**

- $R_1$ und $R_2$ unabhängig voneinander ausgewählt sind aus H, $CH_3$ $C_2H_5$ oder CHO,

, insbesondere

- $R_1$ und $R_2$ unabhängig voneinander ausgewählt sind aus H oder $CH_3$.

**6.** Cyclohexyl-Harnstoff-Derivate gemäß einem der Ansprüche 1 bis 4, **dadurch gekennzeichnet, dass**

- $R_3$ ausgewählt ist aus Indolyl, Pyridyl, Thienyl, Pyrrolyl, Phenyl, Benzyl oder Phenethyl, jeweils unsubstituiert oder am Ring ein- oder mehrfach substituiert,

vorzugsweise

- $R_3$ ausgewählt ist aus Phenyl unsubstituiert oder am Ring einfach substituiert; Benzyl oder Phenethyl, Indolyl, Pyridyl, Thienyl, Pyrrolyl,

insbesondere

■ $R_3$ ausgewählt ist aus Phenyl, Benzyl, Phenethyl, o-Fluor-Phenyl, m-Fluor-Phenyl, p-Fluor-Phenyl, o-Chlor-Phenyl, p-Chlorphenyl, m-Chlorphenyl, o-Brom-Phenyl, m-Brom-Phenyl, p-Brom-Phenyl, o-Iod-Phenyl, m-Iod-Phenyl, p-Iod-Phenyl, o-Amino-Phenyl, m-Amino-Phenyl, p-Amino-Phenyl,o-Methyl-Phenyl, m-Methyl-Phenyl, p-Methyl-Phenyl, o-Methoxy-Phenyl, m-Methoxy-Phenyl, p- Methoxyphenyl, o-Ethyl-Phenyl, m-Ethyl-Phenyl, p-Ethyl-Phenyl, o-Ethoxy-Phenyl, m-Ethoxy-Phenyl, p-Ethoxy-Phenyl, o-Hydroxy-Phenyl, m-Hydroxy-Phenyl oder p-Hydroxy-Phenyl; Indolyl, Pyridyl, Thienyl, Pyrrolyl.

**7.** Cyclohexyl-Harnstoff-Derivate gemäß einem der Ansprüche 1 bis 4, **dadurch gekennzeichnet, dass**

■ $R_4$ ausgewählt ist aus H, $CH_3$, $C_2H_5$, n-Propyl, i-Propyl, n-Butyl, i-Butyl oder t-Butyl.

**8.** Cyclohexyl-Harnstoff-Derivate gemäß einem der Ansprüche 1 bis 4, **dadurch gekennzeichnet, dass**

■ $R_5$, wenn $m \neq 0$, ausgewählt ist aus:

- $(CH_2)_qR^{12}$,
- C (Y)-Z- $R^{12}$ oder
- C(Y)-O-(Z)$R^{12}$,
mitY=O
mit Z = $C_{1-8}$-Alkyl, gesättigt oder ungesättigt, verzweigt oder unverzweigt, einfach oder mehrfach substituiert oder unsubstituiert;
oder $(CH_2)_q$,
mit q = 0-8

insbesondere
-$(CH_2)_qR^{12}$,
-C(Y)-Z-$R^{12}$ oder
-C(Y)-O-(Z)-$R^{12}$,
mit Y=O
mit Z = $C_{1-6}$-Alkyl, gesättigt oder ungesättigt, verzweigt oder unverzweigt, einfach oder mehrfach substituiert oder unsubstituiert;
oder $(CH_2)$q
mit q = 0-6.

**9.** Cyclohexyl-Harnstoff-Derivate gemäß einem der Ansprüche 1 bis 4, **dadurch gekennzeichnet, dass**

■ $R_5$, wenn m = 0, ausgewählt ist aus -$(CH_2)_qR_{12}$,
mit q = 0-6.

**10.** Cyclohexyl-Harnstoff-Derivate gemäß einem der Ansprüche 1 bis 4, **dadurch gekennzeichnet, dass** $R^{12}$ ausgewählt ist aus H; Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus H; Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**11.** Cyclohexyl-Harnstoff-Derivate ausgewählt aus der Gruppe

1-(4-Dimethylamino-4-phenylcyclohexyl)-3-(3-phenylpropyl)harnstoff Hydrochlorid (unpolareres und polareres Diastereoisomer)
1-(4-Dimethylamino-4-phenylcyclohexyl)-3-[2-(1 H-indol-3-yl)ethyl]-harnstoff-hydrochlorid (unpolareres und

polareres Diastereoisomer)
N-[(4-Dimethylamino-4-phenylcyclohexylcarbamoyl)methyl]-3-(1H-indol-3-yl)butyramid-hydrochlorid (unpolareres und polareres Diastereoisomer)
5-(1H-Indol-3-yl)-pentansäure[(4-dimethylamino-4-phenylcyclohexylcarbamoyl)methyl]amid-hydrochlorid (unpolareres und polareres Diastereoisomer)
6-(1H-Indol-3-yl)hexansäure[(4-dimethylamino-4-phenylcyclohexylcarbamoyl)-methyl]-amid-hydrochlorid (unpolareres und polareres Diastereoisomer)
N-[(4-Dimethylamino-4-phenylcyclohexylcarbamoyl)methyl]-3-(1 H-indol-3-yl)propionamid-hydrochlorid (unpolareres und polareres Diastereoisomer)
N-(4-Dimethylamino-4-phenylcyclohexyl)-2-(2-1 H-indol-3-ylacetylamino)-propionamid-hydrochlorid (unpolareres und polareres Diastereoisomer)
2-(2-1 H-Indol-3-ylacetylamino)-4-methylpentansäure-(4-dimethylamino-4-phenylcyclohexyl)amid-hydrochlorid (unpolareres und polareres Diastereoisomer)
1-(4-Dimethylamino-4-phenylcyclohexyl)-3-[2-(1 1H-indol-3-yl)-1-methylethyl]-harnstoff-hydrochlorid (unpolareres und polareres Diastereoisomer)
1-(4-Dimethylamino-4-phenylcyclohexyl)-3-[2-(5-Fluor-1 H-indol-3-yl)ethyl]-harnstoff-hydrochlorid (unpolareres und polareres Diastereoisomer)
1-(4-Dimethylamino-4-phenylcyclohexylmethyl)-3-[2-(1 H-indol-3-yl)ethyl]-harnstoff-hydrochlorid (unpolareres und polareres Diastereoisomer)
1-(4-Dimethylamino-4-phenylcyclohexylmethyl)-3-(3-phenylpropyl)harnstoff-hydrochlorid (unpolareres und polareres Diastereoisomer)
1-(4-Dimethylamino-4-phenylcyclohexylmethyl)-3-[2-(1 H-indol-3-yl)-1-methylethyl]-harnstoff-hydrochlorid (unpolareres und polareres Diastereoisomer)
1-(4-Dimethylamino-4-phenylcyclohexylmethyl)-3-[2-(5-fluor-1 H-indol-3-yl)ethyl]harnstoff-citrat (unpolareres und polareres Diastereoisomer)
1-[3-(4-Dimethylamino-4-phenyl-cyclohexyl)-propyl]-3-[2-(1 H-indol-3-yl) ethyl]-harnstoff (unpolareres und polareres Diastereiosomer)
1-(4-Dimethylamino-4-phenylcyclohexyl)-3-(4-phenyl-propyl)thioharnstoff-citrat (unpolareres und polareres Diastereiosomer)
1-(4-Dimethylamino-4-phenylcyclohexyl)-3-[2-(1 H-indol-3-yl)methylethyl]thioharnstoff-citrat (unpolareres und polareres Diastereiosomer)
1-[3-(4-Dimethylamino-4-phenyl-cyclohexyl)-propyl]-3-[2-(1 H-indol-3-yl)-ethyl]-harnstoff (unpolareres und polareres Diastereiosomer)
1-(4-Dimethylamino-4-phenylcyclohexylmethyl)-3-[2-(1 H-indol-3-yl)ethyl]-thioharnstoff-citrat (unpolareres und polareres Diastereoisomer)
1-(4-Dimethylamino-4-phenylcyclohexylmethyl)-3-[2-(1H-indol-3-yl)-1-methylethyl]thioharnstoff-citrat (unpolareres und polareres Diastereoisomer)
2-[3-(4-Dimethylamino-4-phenylcyclohexylmethyl)thioureido]-3-(1 H-indol-3-yl)propionsäure-methylester-citrat (unpolareres und polareres Diastereoisomer)
1-[2-(4-Dimethylamino-4-phenylcyclohexyl)ethyl]-3-[2-(1 H-indol-3-yl)-ethyl]thioharnstoff-hydrochlorid (unpolareres Diastereoisomer)
1-[2-(4-Dimethylamino-4-phenylcyclohexyl)ethyl]-3-[2-(1 H-indol-3-yl)-ethyl]thioharnstoff-citrat (polareres Diastereoisomer)

**12.** Arzneimittel enthaltend mindestens ein Cyclohexyl-Harnstoff-Derivat gemäß einem der Ansprüche 1 bis 11 sowie gegebenenfalls geeignete Zusatz- und/oder Hilfsstoffe und/oder gegebenenfalls weitere Wirkstoffe.

**13.** Verwendung eines Cyclohexyl-Harnstoff-Derivats gemäß einem der Ansprüche 1 bis 11 zur Herstellung eines Arzneimittels zur Behandlung von Schmerz, insbesondere von akutem, viszeralem, neuropathischem oder chronischem Schmerz.

**14.** Verwendung eines Cyclohexyl-Harnstoff-Derivats gemäß einem der Ansprüche 1 bis 11 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als

Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

**15.** Verfahren zur Herstellung von Cyclohexyl-Harnstoff-Derivaten der Formel I umfassend die Verfahrensschritte

a1) für Verbindungen, in denen n= 0 ist, Überführung eines 4-Aminocyclohexanons durch Oximbildung und anschließende Reduktion oder durch reduktive Aminierung in das entsprechende 4-Aminocylohexylamin;
a2) für Verbindungen, in denen n = 1 ist, Umsetzung eines 4-Aminocyclohexanons mit dem Umsetzungsprodukt aus Alkoxymethyl-triphenylphosphoniumhalogenid und einer starken Base zum 4-Aminocyclohexancarbaldehyd, und anschließende reduktive Aminierung bzw. Oximbildung mit nachfolgender Reduktion zum 4-Aminocyclohexylmethylamin;
a3) für Verbindungen, in denen n = 2 ist Umsetzung eines 4-Aminocyclohexanons mit dem Umsetzungsprodukt aus Cyanomethylphosphonsäure-dialkylester und einer starken Base zum $\alpha$,$\beta$-ungesättigten Nitril, der anschließend zum 4-Aminocyclohexylethylamin reduziert wird;
a4) für Verbindungen, in denen n = 3 ist, Umsetzung eines wie unter a2) beschrieben erhaltenen 4-Aminocyclohexancarbaldehyds zur weiteren Kettenverlängerung mit dem Umsetzungsprodukt aus Cyanmethanphosphonsäuredialkylester und einer starken Base zum $\alpha$, $\beta$-ungesättigten Nitril, und anschließende Reduktion des entstandenen $\alpha$,$\beta$-ungesättigten Nitrils zum Amin;
b) Umsetzung des in Schritt a1), a2) a3) oder a4) gebildeten Amins mit einem aktivierten Kohlesäurenderivat in Gegenwart einer Base zum Carbamidsäureester;
c) Umsetzung des in Schritt b) gebildeten Carbamidsäureesters mit Aminen der Formel $R_5$-$NH_2$ zu Cyclohexyl-Harnstoff-Derivaten der Formel I mit m=0.

**16.** Verfahren zur Herstellung von Cyclohexyl-Harnstoff-Derivaten der Formel I, in denen $m \neq 0$ ist und $R^5$ ausgewählt ist aus -C(Y)-Z-$R^{12}$ oder -C(Y)-O-Z-$R^{12}$ mit Y=O oder S, **dadurch gekennzeichnet, dass** ein gemäß dem Verfahren in Anspruch 15, Schritt a1, a2), a3) oder a4) hergestelltes Amin mit einem am Carbonylterminus aktivierten Boc-Aminosäure-Derivat acyliert wird, anschließend die Boc-Schutzgruppe sauer abgespalten wird und nachfolgend die entschützte Aminogruppe mit einem aktivierten Carbonsäurederivat acyliert wird.

**17.** Verfahren zur Herstellung von Cyclohexyl-Harnstoff-Derivaten der Formel I, **dadurch gekennzeichnet, dass** ein Amin der Formel $R_5$-$NH_2$ mit einem aktivierten Kohlensäurederivat zu einem Carbamidsäureester umgesetzt wird und anschließend die Umsetzung mit einem gemäß Schritt a1, a2), a3) oder a4) von Anspruch 15 erhaltenen Amin zu einem Cyclohexyl-Harnstoff-Derivat der Formel I mit m=0 umgesetzt wird.

**18.** Verfahren zur Herstellung von Cyclohexyl-Harnstoff-Derivaten der Formel I mit $m \neq 0$, **dadurch gekennzeichnet, dass** gemäß Schritt a1, a2), a3) oder a4) von Anspruch 15 hergestellte 4-Aminocyclohexylamine gemäß dem nachfolgenden Reaktionsschema mit am Carboxyl-Terminus aktivierten Aminosäure-Derivaten acyliert werden

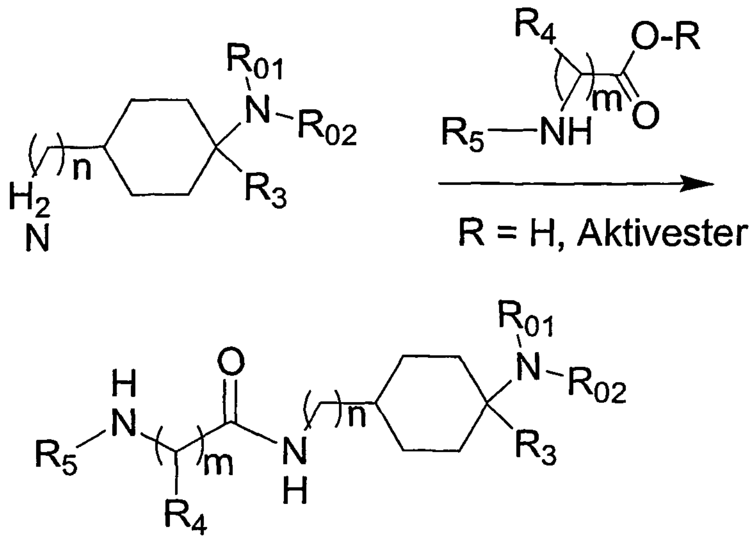


**19.** Verfahren zur Herstellung von Cyclohexyl-Harnstoff-Derivaten der Formel I, in denen X=O oder S ist und m=0 ist, **dadurch gekennzeichnet, dass** ein gemäß Schritt a1), a2), a3) oder a4) von Anspruch 15 hergestelltes Amin mit einem Isocyanat der Formel OCN-$R_5$ oder mit einem Isothiocyanat der Formel SCN-$R_5$ zum Cyclohexyl-Harnstoff-Derivat der Formel I umgesetzt wird.

## Claims

**1.** Cyclohexylurea derivatives of the general formula I,

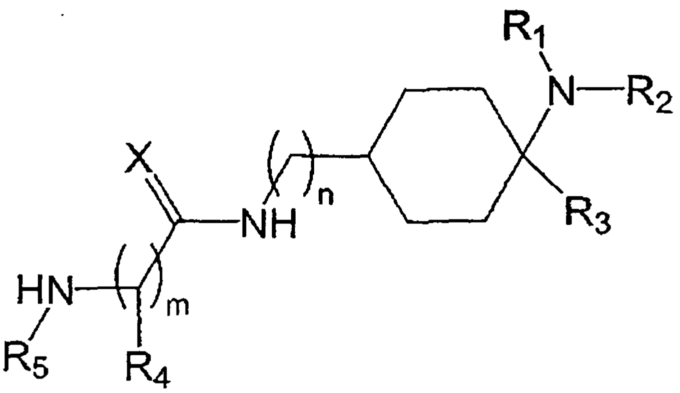

in which
n = 0-3,
m = 0-2,
X = O or S (where m = 0),
$R^1$ and $R^2$ are mutually independently selected from among H; $C_{1-8}$ alkyl or $C_{3-8}$ cycloalkyl, in each case saturated or unsaturated, branched or unbranched, mono- or polysubstituted or unsubstituted; aryl or heteroaryl, in each case mono- or polysubstituted or unsubstituted; or aryl, $C_{3-8}$ cycloalkyl or heteroaryl, in each case mono- or polysubstituted or unsubstituted, bound via $C_{1-3}$ alkylene,
or the radicals $R^1$ and $R^2$ together form a ring and mean $CH_2CH_2OCH_2CH_2$, $CH_2CH_2NR^6CH_2CH_2$ or $(CH_2)_{3-6}$, with $R^6$ selected from among H; $C_{1-8}$ alkyl or $C_{3-8}$ cycloalkyl, in each case saturated or unsaturated, branched or unbranched, mono- or polysubstituted or unsubstituted; aryl or heteroaryl, in each case mono- or polysubstituted or unsubstituted; or aryl, $C_{3-8}$ cycloalkyl or heteroaryl bound via $C_{1-3}$ alkylene, in each case mono- or polysubstituted or unsubstituted;

$R^3$ is selected from among $C_{1-8}$ alkyl or $C_{3-8}$ cycloalkyl, in each case saturated or unsaturated, branched or unbranched, mono- or polysubstituted or unsubstituted; aryl, heteroaryl, in each case unsubstituted or mono- or polysubstituted; aryl, $C_{3-8}$ cycloalkyl or heteroaryl, in each case unsubstituted or mono- or polysubstituted, bound via a saturated or unsaturated, branched or unbranched, substituted or unsubstituted $C_{1-4}$ alkyl group;
$R^4$ is selected from among H, $C_{1-8}$ alkyl, saturated or unsaturated, branched or unbranched, mono- or polysubstituted or unsubstituted; or $-(CH_2)_o-W-(CH_2)_p-H$
with W = O, $NR_7$ or S, and o = 0-3 and p = 0-4 and with $R_7$ selected from among H, $C_{1-8}$ alkyl, saturated or unsaturated, branched or unbranched, mono- or polysubstituted or unsubstituted;
$R^5$, if $m \neq 0$, is selected from among:

- $(CH_2)_qR^{12}$,
- $C(Y)-Z-R^{12}$ or
- $C(Y)-O-Z-R^{12}$,

with Y = O, $CH_2$ or S,
with Z = $C_{1-8}$ alkyl, saturated or unsaturated, branched or unbranched, mono- or polysubstituted or unsubstituted; or $(CH_2)q$
with q = 0-8
with $R^{12}$ selected from among
H; $C_{3-8}$ cycloalkyl, aryl or heteroaryl, in each case unsubstituted or mono- or polysubstituted,
or
$R^5$ if m = 0, is selected from among $C_{3-8}$ cycloalkyl, aryl or heteroaryl, in each case unsubstituted or mono- or polysubstituted; $-(CH_2)qR^{12}$,
with q = 0-8
with $R^{12}$ selected from among
H; $C_{3-8}$ cycloalkyl, aryl or heteroaryl, in each case unsubstituted or mono- or polysubstituted;
optionally in the form of the racemates thereof, the pure stereoisomers thereof, in particular enantiomers or diastereomers, or in the form of mixtures of the stereoisomers, in particular the enantiomers or diastereomers, in any desired mixing ratio;
in the form as prepared or in the form of the acids or bases thereof or in the form of the salts thereof, in particular the physiologically acceptable salts or salts of physiologically acceptable acids or cations; or in the form of the solvates thereof, in particular the hydrates.

2. Cyclohexylurea derivatives according to claim 1, **characterised in that**
$R^1$ and $R^2$ are mutually independently selected from among H; $C_{1-8}$ alkyl, saturated or unsaturated, branched or unbranched, mono- or polysubstituted or unsubstituted;
or the radicals $R^1$ and $R^2$ together form a ring and mean $CH_2CH_2OCH_2CH_2$, $CH_2CH_2NR^6CH_2CH_2$ or $(CH_2)_{3-6}$,
with $R^6$ selected from among H; $C_{1-8}$ alkyl, saturated or unsaturated, branched or unbranched, mono- or polysubstituted or unsubstituted,
preferably
$R^1$ and $R^2$ are mutually independently selected from among H; $C_{1-4}$ alkyl, saturated or unsaturated, branched or unbranched, mono- or polysubstituted or unsubstituted,
or the radicals $R^1$ and $R^2$ together form a ring and mean $(CH_2)_{4-5}$,
in particular
$R^1$ and $R^2$ are mutually independently selected from among H, methyl or ethyl.

3. Cyclohexylurea derivatives according to either claim 1 or claim 2, **characterised in that**
$R^3$ is selected from among $C_{3-8}$ cycloalkyl, aryl or heteroaryl, in each case unsubstituted or mono- or polysubstituted; aryl, $C_{3-8}$ cycloalkyl or heteroaryl, in each case unsubstituted or mono- or polysubstituted, bound via a saturated or unsaturated, unbranched, substituted or unsubstituted $C_{1-2}$ alkyl group;
preferably
$R^3$ is selected from among $C_{5-6}$ cycloalkyl, phenyl, naphthyl, anthracenyl, thiophenyl, benzothiophenyl, furyl, benzofuranyl, benzodioxolanyl, indolyl, indanyl, benzodioxanyl, pyrrolyl, pyrimidiyl or pyrazinyl, in each case unsubstituted or mono- or polysubstituted; $C_{5-6}$ cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, in each case unsubstituted or mono- or polysubstituted, bound via a saturated, unbranched $C_{1-2}$ alkyl group;
in particular
$R^3$ is selected from among phenyl, furyl, thiophenyl, cyclohexanyl, naphthyl, benzofuranyl, indolyl, indanyl, benzodioxanyl, benzodioxolanyl, pyrrolyl, pyrimidiyl, pyrazinyl or benzothiophenyl, in each case unsubstituted or mono- or polysubstituted; phenyl, furyl or thiophenyl, in each case unsubstituted or mono- or polysubstituted, bound via a saturated, unbranched $C_{1-2}$ alkyl group.

4. Cyclohexylurea derivatives according to any one of claims 1 to 3, **characterised in that** $R^4$ is selected from among H or $C_{1-8}$ alkyl, saturated or unsaturated, branched or unbranched, mono- or polysubstituted or unsubstituted, preferably H, $CH_3$ or $C_2H_5$.

5. Cyclohexylurea derivatives according to claims 1 to 4, **characterised in that**

   - $R_1$ and $R_2$ are mutually independently selected from among H, $CH_3$, $C_2H_5$ or CHO,

   in particular

   - $R_1$ and $R_2$ are mutually independently selected from among H or $CH_3$.

6. Cyclohexylurea derivatives according to any one of claims 1 to 4, **characterised in that**

   - $R_3$ is selected from among indolyl, pyridyl, thienyl, pyrrolyl, phenyl, benzyl or phenethyl, in each case unsubstituted or mono- or polysubstituted on the ring,

   preferably

   - $R_3$ is selected from among phenyl which is unsubstituted or mono-substituted on the ring; benzyl or phenethyl, indolyl, pyridyl, thienyl, pyrrolyl,

   in particular

   - $R_3$ is selected from among phenyl, benzyl, phenethyl, o-fluorophenyl, m-fluorophenyl, p-fluorophenyl, o-chlorophenyl, p-chlorophenyl, m-chlorophenyl, o-bromophenyl, m-bromophenyl, p-bromophenyl, o-iodophenyl, m-iodophenyl, p-iodophenyl, o-aminophenyl, m-aminophenyl, p-aminophenyl, o-methylphenyl, m-methylphenyl, p-methylphenyl, o-methoxyphenyl, m-methoxyphenyl, p-methoxyphenyl, o-ethylphenyl, m-ethylphenyl, p-ethylphenyl, o-ethoxyphenyl, m-ethoxyphenyl, p-ethoxyphenyl, o-hydroxyphenyl, m-hydroxyphenyl or p-hydroxyphenyl; indolyl, pyridyl, thienyl, pyrrolyl.

7. Cyclohexylurea derivatives according to any one of claims 1 to 4, **characterised in that**

   - $R_4$ is selected from among H, $CH_3$, $C_2H_5$, n-propyl, i-propyl, n-butyl, i-butyl or t-butyl.

8. Cyclohexylurea derivatives according to any one of claims 1 to 4, **characterised in that**

   - $R_5$, if $m \neq 0$, is selected from among:

     $-(CH_2)_qR^{12}$,
     $-C(Y)-Z-R^{12}$ or
     $-C(Y)-O-(Z)-R^{12}$,
     with Y = O
     with Z = $C_{1-8}$ alkyl, saturated or unsaturated, branched or unbranched, mono- or polysubstituted or unsubstituted;
     or $(CH_2)_q$
     with q = 0-8,

   in particular
   $-(CH_2)_qR^{12}$,
   $-C(Y)-Z-R^{12}$ or
   $-C(Y)-O-(Z)-R^{12}$,
   with Y = O
   with Z = $C_{1-6}$ alkyl, saturated or unsaturated, branched or unbranched, mono- or polysubstituted or

unsubstituted;
or $(CH_2)_q$
with q = 0-6.

**9.** Cyclohexylurea derivatives according to any one of claims 1 to 4, **characterised in that**

• $R_5$, if m = 0, is selected from among $-(CH_2)_qR_{12}$,
with q = 0-6.

**10.** Cyclohexylurea derivatives according to any one of claims 1 to 4, **characterised in that**
$R^{12}$ is selected from among H; cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidiyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^{12}$ is selected from among H; cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidiyl, in each case unsubstituted or mono- or polysubstituted.

**11.** Cyclohexylurea derivatives selected from among the group

1-(4-dimethylamino-4-phenylcyclohexyl)-3-(3-phenylpropyl)urea hydrochloride (more nonpolar and more polar diastereoisomer)
1-(4-dimethylamino-4-phenylcyclohexyl)-3-[2-(1 *H*-indol-3-yl)ethyl]urea hydrochloride (more nonpolar and more polar diastereoisomer)
N-[(4-dimethylamino-4-phenylcyclohexylcarbamoyl)methyl]-3-(1*H*-indol-3-yl)butyramide hydrochloride (more nonpolar and more polar diastereoisomer)
5-(1*H*-indol-3-yl)-pentanoic acid [(4-dimethylamino-4-phenylcyclohexylcarbamoyl)methyl]amide hydrochloride (more nonpolar and more polar diastereoisomer)
6-(1 *H*-indol-3-yl)hexanoic acid [(4-dimethylamino-4-phenylcyclohexylcarbamoyl)-methyl]-amide hydrochloride (more nonpolar and more polar diastereoisomer)
N-[(4-dimethylamino-4-phenylcyclohexylcarbamoyl)methyl]-3-(1*H*-indol-3-yl)propionamide hydrochloride (more nonpolar and more polar diastereoisomer)
N-(4-dimethylamino-4-phenylcyclohexyl)-2-(2-1 *H*-indol-3-ylacetylamino)-propionamide hydrochloride (more nonpolar and more polar diastereoisomer)
2-(2-1 H-indol-3-ylacetylamino)-4-methylpentanoic acid (4-dimethylamino-4-phenylcyclohexyl)amide hydrochloride (more nonpolar and more polar diastereoisomer)
1-(4-dimethylamino-4-phenylcyclohexyl)-3-[2-(1 1*H*-indol-3-yl)-1-methyl-ethyl]-urea hydrochloride (more nonpolar and more polar diastereoisomer)
1-(4-dimethylamino-4-phenylcyclohexyl)-3-[2-(5-fluoro-1 *H*-indol-3-yl)ethyl]-urea hydrochloride (more nonpolar and more polar diastereoisomer)
1-(4-dimethylamino-4-phenylcyclohexylmethyl)-3-[2-(1 H-indol-3-yl)ethyl]-urea hydrochloride (more nonpolar and more polar diastereoisomer)
1-(4-dimethylamino-4-phenylcyclohexylmethyl)-3-(3-phenylpropyl)urea hydrochloride (more nonpolar and more polar diastereoisomer)
1-(4-dimethylamino-4-phenylcyclohexylmethyl)-3-[2-(1 *H*-indol-3-yl)-1-methylethyl]-urea hydrochloride (more nonpolar and more polar diastereoisomer)
1-(4-dimethylamino-4-phenylcyclohexylmethyl)-3-[2-(5-fluoro-1*H*-indol-3-yl)ethyl]urea citrate (more nonpolar and more polar diastereoisomer)
1-[3-(4-dimethylamino-4-phenyl-cyclohexyl)-propyl]-3-[2-(1*H*-indol-3-yl)-ethyl]-urea (more nonpolar and more polar diastereoisomer)
1-(4-dirnethylamino-4-phenylcyclohexyl)-3-(4-phenylpropyl)thiourea citrate (more nonpolar and more polar diastereoisomer)
1-(4-dimethylamino-4-phenylcyclohexyl)-3-[2-(1*H*-indol-3-yl)methyl-ethyl]thiourea citrate (more nonpolar and more polar diastereoisomer)
1-[3-(4-dimethylamino-4-phenyl-cyclohexyl)-propyl]-3-[2-(1H-indol-3-yl)-ethyl]-urea (more nonpolar and more

polar diastereoisomer)
1-(4-dimethylamino-4-phenylcyclohexylmethyl)-3-[2-(1 *H*-indol-3-yl)ethyl]-thiourea citrate (more nonpolar and more polar diastereoisomer)
1-(4-dimethylamino-4-phenylcyclohexylmethyl)-3-[2-(1*H*-indol-3-yl)-1-methylethyl]thiourea citrate (more nonpolar and more polar diastereoisomer)
2-[3-(4-dimethylamino-4-phenylcyclohexylmethyl)thioureido]-3-(1*H*-indol-3-yl)propionic acid methyl ester citrate (more nonpolar and more polar diastereoisomer)
1-[2-(4-dimethylamino-4-phenylcyclohexyl)ethyl]-3-[2-(1 H-indol-3-yl)-ethyl]thiourea hydrochloride (more nonpolar diastereoisomer)
1-[2-(4-dimethylamino-4-phenylcyclohexyl)ethyl]-3-[2-(1 H-indol-3-yl)-ethyl]thiourea citrate (more polar diastereoisomer)

**12.** A pharmaceutical preparation containing at least one cyclohexylurea derivative according to any one of claims 1 to 11 and optionally suitable additives and/or auxiliary substances and/or optionally further active ingredients.

**13.** Use of a cyclohexylurea derivative according to any one of claims 1 to 11 for the production of a pharmaceutical preparation for the treatment of pain, in particular of acute, visceral, neuropathic or chronic pain.

**14.** Use of a cyclohexylurea derivative according to any one of claims 1 to 11 for the production of a pharmaceutical preparation for the treatment of anxiety, stress and stress-related syndromes, depression, epilepsy, Alzheimer's disease, senile dementia, general cognitive dysfunction, learning and memory disorders (as nootropic), withdrawal symptoms, abuse of and/or dependency on alcohol and/or drugs and/or medicines, sexual dysfunction, cardiovascular diseases, hypotension, hypertension, tinnitus, pruritus, migraine, hardness of hearing, insufficient intestinal motility, eating disorders, anorexia, obesity, locomotive disorders, diarrhoea, cachexia, urinary incontinence or as a muscle relaxant, anticonvulsive or anaesthetic or for coadministration on treatment with an opioid analgesic or with an anaesthetic, for diuresis or antinatriuresis, anxiolysis, for modulation of mobility, for modulation of neurotransmitter release and treatment of neurodegenerative diseases associated therewith, for the treatment of withdrawal symptoms and/or the reduction of the addictive potential of opioids.

**15.** A process for the production of cyclohexylurea derivatives of the formula I comprising the process steps

a1) for compounds in which n = 0, conversion of a 4-aminocyclohexanone by oxime formation and subsequent reduction or by reductive amination into the corresponding 4-aminocyclohexylamine,
a2) for compounds in which n = 1, reaction of a 4-aminocyclohexanone with the reaction product of alkoxymethyl triphenylphosphonium halide and a strong base to yield 4-aminocyclohexanecarbaldehyde, and subsequent reductive amination or oxime formation and successive reduction to yield 4-aminocyclohexylmethylamine;
a3) for compounds in which n = 2, reaction of a 4-aminocyclohexanone with the reaction product of cyanomethylphosphonic acid dialkyl ester and a strong base to yield the $\alpha,\beta$-unsaturated nitrile, which is then reduced to yield 4-aminocyclohexylethylamine;
a4) for compounds in which n = 3, reaction of a 4-aminocyclohexanecarbaldehyde obtained as described in a2) for further chain extension with the reaction product of cyanomethanephosphonic acid dialkyl ester and a strong base to yield $\alpha,\beta$-unsaturated nitrile, and subsequent reduction of the resultant $\alpha,\beta$-unsaturated nitrile to yield the amine;
b) reaction of the amine formed in step a1), a2), a3) or a4) with an activated carbonic acid derivative in the presence of a base to yield the carbamic acid ester;
c) reaction of the carbamic acid ester obtained in step b) with amines of the formula $R_5$-$NH_2$ to yield cyclohexylurea derivatives of the formula I with m = 0.

**16.** A process for the production of cyclohexylurea derivatives of the formula I in which $m \neq 0$ and $R^5$ is selected from among -C(Y)-Z-$R^{12}$ or -C(Y)-O-Z-$R^{12}$ with Y = O or S, **characterised in that** an amine produced according to the process in claim 15, step a1), a2), a3) or a4) is acylated with a Boc amino acid derivative activated on the carbonyl terminus, then the Boc protecting group is eliminated under acidic conditions and the deprotected amino group is then acylated with an activated carboxylic acid derivative.

**17.** A process for the production of cyclohexylurea derivatives of the formula I, **characterised in that** an amine of the formula $R_5$-$NH_2$ is reacted with an activated carbonic acid derivative to yield a carbamic acid ester and then the reaction with an amine obtained according to step a1, a2), a3) or a4) of claim 15 to yield a cyclohexylurea derivative of the formula I with m = 0 is performed.

**18.** A process for the production of cyclohexylurea derivatives of the formula I with m ≠ 0, **characterised in that** 4-aminocyclohexylamines obtained according to step a1, a2), a3) or a4) of claim 15 are acylated according to the following reaction scheme with amino acid derivatives activated on the carboxyl terminus

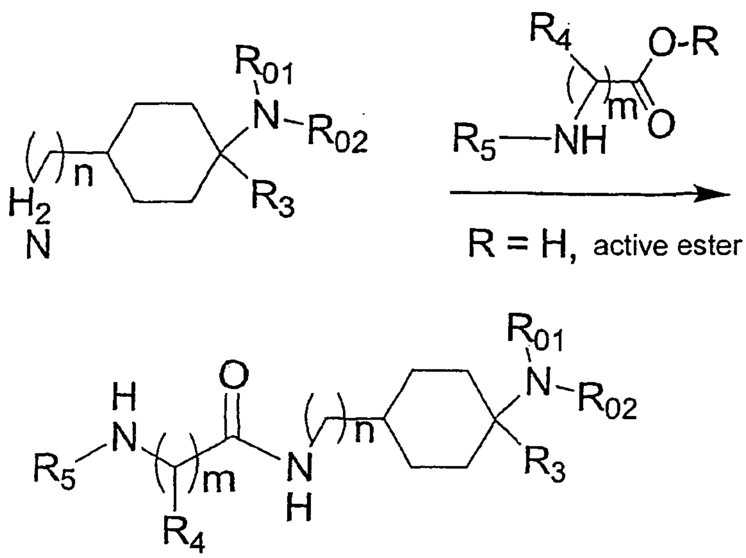


**19.** A process for the production of cyclohexylurea derivatives of the formula I, in which X = O or S and m = 0, **characterised in that** an amine produced according to step a1), a2), a3) or a4) of claim 15 is reacted with an isocyanate of the formula OCN-$R_5$ or with an isothiocyanate of the formula SCN-$R_5$ to yield the cyclohexylurea derivative of the formula I.

## Revendications

**1.** Dérivés de cyclohexylurée de formule générale I

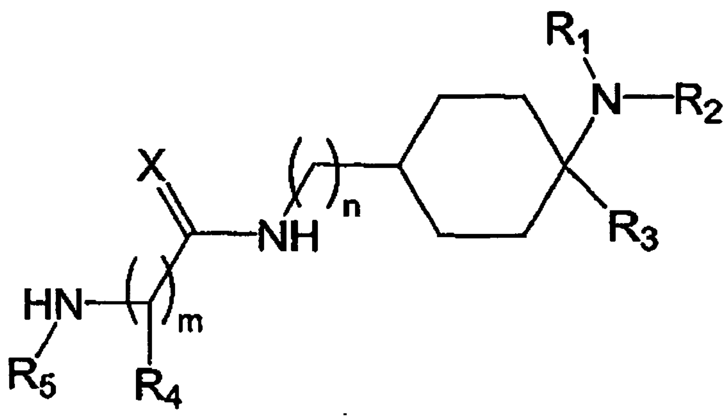

formule dans laquelle
n = 0 - 3,
m = 0 - 2,
X = O ou S (pour m = 0),
$R^1$ et $R^2$ sont choisis indépendamment l'un de l'autre entre H ; un groupe alkyle en $C_1$ à $C_8$ ou cycloalkyle en $C_3$ à $C_8$, chacun saturé ou non saturé, ramifié ou non ramifié, substitué une ou plusieurs fois ou non substitué ; aryle ou hétéroaryle, chacun substitué une ou plusieurs fois ou non substitué ; ou hétéroaryle, cycloalkyle en $C_3$ à $C_8$ ou aryle lié par l'intermédiaire d'un radical alkyle en $C_1$ à $C_3$, chacun substitué une ou plusieurs fois ou non substitué, ou bien les restes $R^1$ et $R^2$ forment ensemble un noyau et représentent $CH_2CH_2OCH_2CH_2$, $CH_2CH_2NR^6CH_2CH_2$

ou $(CH_2)_{3-6}$,
$R^6$ étant choisi entre H; un groupe alkyle en $C_1$ à $C_8$ ou cycloalkyle en $C_3$ à $C_8$, chacun saturé ou non saturé, ramifié ou non ramifié, substitué une ou plusieurs fois ou non substitué ; aryle ou hétéroaryle, chacun substitué une ou plusieurs fois ou non substitué ; ou bien hétéroaryle, cycloalkyle en $C_3$ à $C_8$ ou aryle lié par l'intermédiaire d'un radical alkyle en $C_1$ à $C_3$, chacun substitué une ou plusieurs fois ou non substitué ;
$R^3$ est choisi entre un groupe alkyle en $C_1$ à $C_8$ ou cycloalkyle en $C_3$ à $C_8$, chacun saturé ou non saturé, ramifié ou non ramifié, substitué une ou plusieurs fois ou non substitué ; aryle, hétéroaryle, chacun non substitué ou substitué une ou plusieurs fois ; hétéroaryle, cycloalkyle en $C_3$ à $C_8$ ou aryle lié par l'intermédiaire d'un groupe alkyle en $C_1$ à $C_4$ saturé ou non saturé, ramifié ou non ramifié, substitué ou non substitué, chacun non substitué ou substitué une ou plusieurs fois ;
$R^4$ est choisi entre H ; un groupe alkyle en $C_1$ à $C_8$, saturé ou non saturé, ramifié ou non ramifié, substitué une ou plusieurs fois ou non substitué ; ou un groupe $-(CH_2)_o-W-(CH_2)_p-H$
où W = O, $NR_7$ ou S, et o = 0-3 et p = 0-4, et
$R_7$ étant choisi entre H, un groupe alkyle en $C_1$ à $C_8$, saturé ou non saturé, ramifié ou non ramifié, substitué une ou plusieurs fois ou non substitué ;
$R^5$, lorsque m est différent de 0, est choisi entre un groupe :

- $(CH_2)_qR^{12}$,
-C (Y)-Z-$R^{12}$ ou
-C(Y)-O-Z-$R^{12}$,
avec Y = O, $CH_2$ ou S,
Z = alkyle en $C_1$ à $C_8$, saturé ou non saturé, ramifié ou non ramifié, substitué une ou plusieurs fois ou non substitué ;
ou $(CH_2)_q$
avec q = 0-8
$R^{12}$ étant choisi entre
H ; un groupe cycloalkyle en $C_3$ à $C_8$, aryle ou hétéroaryle, chacun non substitué ou substitué une ou plusieurs fois,

ou bien
$R^5$, lorsque m est égal à 0, est choisi entre un groupe cycloalkyle en $C_3$ à $C_8$, aryle ou hétéroaryle, chacun non substitué ou substitué une ou plusieurs fois ; un groupe $-(CH_2)_qR^{12}$,
avec q = 0-8,
$R^{12}$ étant choisi entre
H ; un groupe cycloalkyle en $C_3$ à $C_8$, aryle ou hétéroaryle, chacun non substitué ou substitué une ou plusieurs fois ;
le cas échéant sous forme de leurs racémates, de leurs stéréoisomères purs, en particulier des énantiomères ou des diastéréoisomères, ou sous forme de mélanges des stéréoisomères, en particulier des énantiomères ou diastéréoisomères, dans un rapport de mélange quelconque ;
sous la forme représentée ou sous forme de leurs acides ou de leurs bases ou sous forme de leurs sels, en particulier des sels physiologiquement acceptables, ou de sels d'acides ou de cations physiologiquement acceptables ; ou sous forme de leurs produits de solvatation, en particulier des hydrates.

**2.** Dérivés de cyclohexylurée suivant la revendication 1, **caractérisés en ce que**
$R^1$ et $R^2$ sont choisis indépendamment l'un de l'autre entre H ; un groupe alkyle en $C_1$ à $C_8$, saturé ou non saturé, ramifié ou non ramifié, substitué une ou plusieurs fois ou non substitué ;
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à
$C_8$, saturé ou non saturé, ramifié ou non ramifié,
substitué une ou plusieurs fois ou non substitué, avantageusement
$R^1$ et $R^2$ sont choisis indépendamment l'un de l'autre entre H ; un groupe alkyle en $C_1$ à $C_4$, saturé ou non saturé, ramifié ou non ramifié, substitué une ou plusieurs fois ou non substitué,
ou bien les restes $R^1$ et $R^2$ forment ensemble un noyau et représentent $(CH_2)_{4-5}$,
en particulier
$R^1$ et $R^2$ sont choisis indépendamment l'un de l'autre entre H, un reste méthyle ou éthyle.

**3.** Dérivés de cyclohexylurée suivant l'une des revendications 1 et 2, **caractérisés en ce que**
$R^3$ est choisi entre un reste cycloalkyle en $C_3$ à $C_8$, aryle ou hétéroaryle, chacun non substitué ou substitué une ou

plusieurs fois ; un reste hétéroaryle, cycloalkyle en $C_3$ à $C_8$ ou aryle lié par l'intermédiaire d'un groupe alkyle en $C_1$ ou $C_2$ saturé ou non saturé, non ramifié, substitué ou non substitué, chacun non substitué ou substitué une ou plusieurs fois ;
avantageusement
$R^3$ est choisi entre un reste cycloalkyle en $C_5$ ou $C_6$, phényle, naphtyle, anthracényle, thiophényle, benzothiophényle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ; un reste, lié par l'intermédiaire d'un groupe alkyle en $C_1$ ou $C_2$ saturé non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois,
en particulier
$R^3$ est choisi entre un reste phényle, furyle, thiophényle, cyclohexanyle, naphtyle, benzofurannyle, indolyle, indanyle, benzodioxannyle, benzodioxolannyle, pyrrolyle, pyrimidyle, pyrazinyle ou benzothiophényle, chacun non substitué ou substitué une ou plusieurs fois, un reste phényle, furyle ou thiophényle lié par l'intermédiaire d'un groupe alkyle en $C_1$ ou $C_2$ saturé, non ramifié, chacun non substitué ou substitué une ou plusieurs fois.

4. Dérivés de cyclohexylurée suivant l'une des revendications 1 à 3, **caractérisés en ce que**
$R^4$ est choisi entre H ou un reste alkyle en $C_1$ à $C_8$, saturé ou non saturé, ramifié ou non ramifié, substitué une ou plusieurs fois ou non substitué, avantageusement H, $CH_3$ ou $C_2H_5$.

5. Dérivés de cyclohexylurée suivant les revendications 1 à 4, **caractérisés en ce que**

■ $R_1$ et $R_2$ sont choisis indépendamment l'un de l'autre entre H, $CH_3$, $C_2H_5$ ou CHO,

en particulier

■ $R_1$ et $R_2$ sont choisis indépendamment l'un de l'autre entre H ou $CH_3$.

6. Dérivés de cyclohexylurée suivant l'une des revendications 1 à 4, **caractérisés en ce que**

■ $R_3$ est choisi entre un reste indolyle, pyridyle, thiényle, pyrrolyle, phényle, benzyle ou phénéthyle, chacun non substitué ou substitué une ou plusieurs fois sur le noyau,

avantageusement

■ $R_3$ est choisi entre un reste phényle, non substitué ou substitué une fois sur le noyau ; un reste benzyle ou phénéthyle, indolyle, pyridyle, thiényle, pyrrolyle,

en particulier

■ $R_3$ est choisi entre un reste phényle, benzyle, phénétyle, o-fluorophényle, m-fluorophényle, p-fluorophényle, o-chlorophényle, p-chlorophényle, m-chlorophényle, o-bromophényle, m-bromophényle, p-bromophényle, o-iodophényle, m-iodophényle, p-iodophényle, o-aminophényle, m-aminophényle, p-aminophényle, o-méthylphényle, m-méthylphényle, p-méthylphényle, o-méthoxyphényle, m-méthoxyphényle, p-méthoxyphényle, o-éthylphényle, m-éthylphényle, p-éthylphényle, o-éthoxyphényle, m-éthoxyphényle, p-éthoxyphényle, o-hydroxyphényle, m-hydroxyphényle ou p-hydroxyphényle ; indolyle, pyridyle, thiényle, pyrrolyle.

7. Dérivés de cyclohexylurée suivant l'une des revendications 1 à 4, **caractérisés en ce que**

■ $R_4$ est choisi entre H, un groupe $CH_3$, $C_2H_5$, n-propyle, isopropyle, n-butyle, isobutyle ou tertiobutyle.

8. Dérivés de cyclohexylurée suivant l'une des revendications 1 à 4, **caractérisés en ce que**

■ $R_5$, lorsque m est différent de 0, est choisi entre :

- $(CH_2)$ $qR^{12}$,
-C (Y)-Z- $R^{12}$ ou
-C(Y)-O-(Z)-$R^{12}$,

avec Y = O
avec Z = alkyle en $C_1$ à $C_8$, saturé ou non saturé, ramifié ou non ramifié, substitué une ou plusieurs fois ou non substitué ;
ou $(CH_2)_q$,
avec q = 0-8

en particulier
- $(CH_2)$ $qR^{12}$,
-C(Y)-Z-$R^{12}$ ou
-C(Y)-O-(Z)-$R^{12}$,
avec Y = O
avec Z = alkyle en $C_1$ à $C_6$, saturé ou non saturé, ramifié ou non ramifié, substitué une ou plusieurs fois ou non substitué ;
ou $(CH_2)_q$
avec q = 0-6.

**9.** Dérivés de cyclohexylurée suivant l'une des revendications 1 à 4, **caractérisés en ce que**

■ $R_5$, lorsque m est égal à 0, est choisi entre des groupes $-(CH_2)_qR^{12}$,

avec q = 0-6.

**10.** Dérivés de cyclohexylurée suivant l'une des revendications 1 à 4, **caractérisés en ce que**
$R^{12}$ est choisi entre H ; un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolanyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;
en particulier
$R^{12}$ est choisi entre H ; un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**11.** Dérivés de cyclohexylurée choisis dans le groupe :

chlorhydrate de 1-(4-diméthylamino-4-phénylcyclohexyl)-3-(3-phénylpropyl)urée (diastéréoisomère le moins polaire et le plus polaire)
chlorhydrate de 1-(4-diméthylamino-4-phénylcyclohexyl)-3-[2-(1H-indole-3-yl)éthyl]urée (diastéréoisomère le moins polaire et le plus polaire)
chlorhydrate de N-[(4-diméthylamino-4-phénylcyclohexylcarbamoyl)méthyl]-3-(1H-indole-3-yl)butyramide (diastéréoisomère le moins polaire et le plus polaire)
chlorhydrate de [(4-diméthylamino-4-phénylcyclohexylcarbamoyl)méthyl]amide d'acide 5-(1H-indole-3-yl)-pentanoïque (diastéréoisomère le moins polaire et le plus polaire)
chlorhydrate de [(4-diméthylamino-4-phénylcyclohexylcarbamoyl)méthyl]amide d'acide 6-(1H-indole-3-yl)-hexanoïque (diastéréoisomère le moins polaire et le plus polaire)
chlorhydrate de N-[(4-diméthylamino-4-phénylcyclohexylcarbamoyl)méthyl]-3-(1H-indole-3-yl)propionamide (diastéréoisomère le moins polaire et le plus polaire)
chlorhydrate de N-(4-diméthylamino-4-phénylcyclohexyl)-2-(2-1H-indole-3-ylacétylamino)propionamide (diastéréoisomère le moins polaire et le plus polaire)
chlorhydrate de (4-diméthylamino-4-phénylcyclohexyl)-amide d'acide 2-(2-1H-indole-3-ylacétylamino)-4-méthylpentanoïque (diastéréoisomère le moins polaire et le plus polaire)
chlorhydrate de 1-(4-diméthylamino-4-phénylcyclohexyl)-3-[2-(1H-indole-3-yl)-1-méthyléthyl]-urée (diastéréoisomère le moins polaire et le plus polaire)
chlorhydrate de 1-(4-diméthylamino-4-phénylcyclohexyl)-3-[2-(5-fluoro-1H-indole-3-yl)éthyl]-urée (diastéréoisomère le moins polaire et le plus polaire)

chlorhydrate de 1-(4-diméthylamino-4-phénylcyclohexylméthyl)-3-[2-(1H-indole-3-yl)éthyl]-urée (diastéréoisomère le moins polaire et le plus polaire)

chlorhydrate de 1-(4-diméthylamino-4-phénylcyclohexylméthyl)-3-(3-phénylpropyl)urée (diastéréoisomère le moins polaire et le plus polaire)

chlorhydrate de 1-(4-diméthylamino-4-phénylcyclohexylméthyl)-3-[2-(1H-indole-3-yl)-1-méthyléthyl]-urée (diastéréoisomère le moins polaire et le plus polaire)

citrate de 1-(4-diméthylamino-4-phénylcyclohexylméthyl)-3-[2-(5-fluoro-1H-indole-3-yl)éthyl]urée (diastéréoisomère le moins polaire et le plus polaire)

1-[3-(4-diméthylamino-4-phényl-cyclohexyl)-propyl]-3-[2-(1H-indole-3-yl)-éthyl]-urée (diastéréoisomère le moins polaire et le plus polaire)

citrate de 1-(4-diméthylamino-4-phénylcyclohexyl)-3-(4-phényl-propyl)thio-urée (diastéréoisomère le moins polaire et le plus polaire)

citrate de 1-(4-diméthylamino-4-phénylcyclohexyl)-3-[2-(1H-indole-3-yl)méthyléthyl]thio-urée (diastéréoisomère le moins polaire et le plus polaire)

1-[3-(4-diméthylamino-4-phényl-cyclohexyl)-propyl]-3-[2-(1H-indole-3-yl)-éthyl]-urée (diastéréoisomère le moins polaire et le plus polaire)

citrate de 1-(4-diméthylamino-4-phénylcyclohexylméthyl)-3-[2-(1H-indole-3-yl)-1-éthyl]-thio-urée (diastéréoisomère le moins polaire et le plus polaire)

citrate de 1-(4-diméthylamino-4-phénylcyclohexylméthyl)-3- [2-(1H-indole-3-yl)méthyléthyl]thio-urée (diastéréoisomère le moins polaire et le plus polaire)

citrate d'ester méthylique d'acide 2-[3-(4-diméthylamino-4-phénylcyclohexylméthyl)thio-uréido]-3-(1H-indole-3-yl)propionique (diastéréoisomère le moins polaire et le plus polaire)

chlorhydrate de 1-[2-(4-diméthylamino-4-phénylcyclohexyl)éthyl]-3-[2-(1H-indole-3-yl)-éthyl]thio-urée (diastéréoisomère le moins polaire)

citrate de 1-[2-(4-diméthylamino-4-phénylcyclohexyl)éthyl]-3-[2-(1H-indole-3-yl)-éthyl]thio-urée (diastéréoisomère le plus polaire)

**12.** Médicament contenant au moins un dérivé de cyclohexylurée suivant l'une des revendications 1 à 11 ainsi que, le cas échéant, des additifs et/ou des substances auxiliaires convenables, et/ou, le cas échéant, d'autres substances actives.

**13.** Utilisation d'un dérivé de cyclohexylurée suivant l'une des revendications 1 à 11 pour la préparation d'un médicament destiné au traitement de la douleur, en particulier d'une douleur aiguë, viscérale, neuropathique ou chronique.

**14.** Utilisation d'un dérivé de cyclohexylurée suivant l'une des revendications 1 à 11 pour la préparation d'un médicament destiné au traitement d'états d'anxiété, de stress et de syndromes liés au stress, de dépressions, de l'épilepsie, de la maladie d'Alzheimer, de la démence sénile, de dysfonctionnements cognitifs généraux, de troubles de l'apprentissage et de la mémoire (comme nootrope), de signes de privation, d'abus de l'alcool et/ou de drogues et/ou de médicaments et/ou d'alcoolodépendance, de toxicomanie et/ou de pharmacodépendance, de dysfonctionnements sexuels, de maladies cardio-vasculaires, d'hypotension, d'hypertension, d'acouphènes, de prurit, de migraine, de surdité, d'insuffisance de motilité intestinale, de trouble de l'absorption de nourriture, de l'anorexie, de l'obésité, de troubles moteurs, de diarrhée, de cachexie, d'incontinence urinaire ou comme myorelaxant, anticonvulsif ou anesthésique, ou destiné à la coadministration en cas de traitement avec un analgésique opiacé ou avec un anesthésique, à la diurèse ou à l'antinatriurèse, à l'anxiolyse, à la modulation de l'activité motrice, à la modulation de la sécrétion de neurotransmetteurs et au traitement de maladies neuro-dégénératives qui y sont associées, au traitement de signes de privation et/ou à la réduction du potentiel de dépendance envers des opiacés.

**15.** Procédé de production de dérivés de cyclohexylurée de formule (I), comprenant les étapes suivantes :

a1) pour des composés dans lesquels n est égal à 0, transformation d'une 4-aminocyclohexanone par formation d'oxime suivie d'une réduction ou par amination par voie de réduction, en la 4-aminocyclohexylamine correspondante ;

a2) pour des composés dans lesquels n est égal à 1, réaction d'une 4-aminocyclohexanone avec le produit de réaction d'un halogénure d'alkoxyméthyltriphénylphosphonium et d'une base forte pour former le 4-aminocyclohexanecarbaldéhyde suivie d'une amination par voie de réduction ou de la formation d'oxime avec réduction subséquente en 4-aminocyclohexylméthylamine ;

a3) pour des composés dans lesquels n est égal à 2, réaction d'une 4-aminocyclohexanone avec le produit de réaction d'un ester de dialkyle d'acide cyanométhyl-phosphonique et d'une base forte pour former le nitrile à

non-saturation α, β, qui est ensuite réduit en 4-aminocyclohexyléthylamine ;
a4) pour des composés dans lesquels n est égal à 3, réaction d'un 4-aminocyclohexanecarbaldéhyde obtenu comme indiqué en a2) en vue d'un allongement supplémentaire de chaîne avec le produit de réaction d'un ester dialkylique d'acide cyanométhanephosphonique et d'une base forte pour former le nitrile à non-saturation α, β, puis réduction du nitrile à non-saturation α, β produit pour former une amine ;
b) réaction de l'amine formée dans l'étape a1), a2), a3 ou a4) avec un dérivé activé d'acide carbonique en présence d'une base pour former l'ester d'acide carbamique ;
c) réaction de l'ester d'acide carbamique formé dans l'étape b) avec des amines de formule $R_5$-$NH_2$ pour obtenir les dérivés de cyclohexylurée de formule I dans laquelle m est égal à 0.

**16.** Procédé de production de dérivés de cyclohexylurée de formule I, dans lesquels m est différent de 0 et $R^5$ est choisi entre un groupe -C(Y)-Z-$R^{12}$ ou -C(Y)-O-Z-$R^{12}$ avec Y = O ou S, **caractérisé en ce qu'**une amine préparée selon le procédé de la revendication 15, étape a1), a2), a3) ou a4), est acylée avec un dérivé de Boc-aminoacide activé à l'extrémité carbonyle terminale, puis le groupe protecteur Boc est éliminé en milieu acide, après quoi le groupe amino débarrassé du groupe protecteur est acylé avec un dérivé d'acide carboxylique activé.

**17.** Procédé de production de dérivés de cyclohexylurée de formule I, **caractérisé en ce qu'**une amine de formule $R_5$-$NH_2$ est amenée à réagir avec un dérivé activé d'acide carbonique pour former un ester d'acide carbamique qui est ensuite amené à réagir avec une amine obtenue selon l'étape a1), a2), a3) ou a4) de la revendication 15 pour former un dérivé de cyclohexylurée de formule I avec m = 0.

**18.** Procédé de production de dérivés de cyclohexylurée de formule I dans lequel m est différent de 0, **caractérisé en ce que** des 4-aminocyclohexylamines préparées selon l'étape a1), a2), a3) ou a4) de la revendication 15 sont acylées conformément au schéma réactionnel suivant avec des dérivés d'amino-acides activés à l'extrémité carboxyle terminale

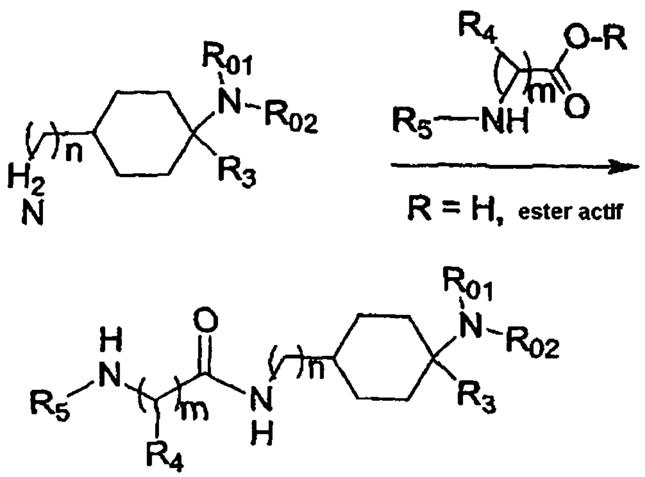


**19.** Procédé de production de dérivés de cyclohexylurée de formule I, dans lesquels X représente O ou S, et m est égal à 0, **caractérisé en ce qu'**une amine préparée selon l'étape a1), a2), a3) ou a4) de la revendication 15 est amenée à réagir avec un isocyanate de formule OCN-$R_5$ ou avec un isothiocyanate de formule SCN-$R_5$ pour former le dérivé de cyclohexylurée de formule I.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 02090317 A
- WO 02090330 A
- WO 02089783 A
- EP 0147085 A
- WO 0290317 A


### In der Beschreibung aufgeführte Nicht-Patentliteratur


- **MEUNIER et al.** *Nature,* 1995, vol. 377, 532-535
- **REINSCHEID et al.** *Science,* 1995, vol. 270, 792-794
- **MOGIL et al.** *Neuroscience,* 1996, vol. 75, 333-337
- **JENCK et al.** *Proc. Natl. Acad. Sci. USA,* 1997, vol. 94, 14854-14858
- **KING et al.** *Neurosci. Lett.,* 1997, vol. 223, 113-116
- **ABDULLA ; SMITH.** *J. Neurosci.,* 1998, vol. 18, 9685-9694
- **MANABE et al.** *Nature,* 1997, vol. 394, 577-581
- **NISHI et al.** *EMBO J.,* 1997, vol. 16, 1858-1864
- **CALO et al.** *Br.J. Pharmacol.,* 2000, vol. 129, 1261-1283
- **LEDNICER et al.** *J. Med. Chem.,* 1980, vol. 23, 424-430
- **ARDATI et al.** *Mol. Pharmacol.,* 1997, vol. 51, 816-824